# EUROPEAN PATENT SPECIFICATION

(11) **EP 1 753 862 B1**
(45) Date of publication and mention of the grant of the patent: **18.11.2015**
(21) Application number: 05718469.9
(22) Date of filing: 18.04.2005
(51) Int. Cl.: C12P 7/18, C12P 17/02, C12N 9/14, C12N 1/19

(54) **METHODS FOR OBTAINING OPTICALLY ACTIVE EPOXIDES AND VICINAL DIOLS FROM MESO-EPOXIDES**
VERFAHREN ZUR GEWINNUNG OPTISCH AKTIVER EPOXIDE UND VICINALER DIOLE AUS MESO-EPOXIDEN
PROCEDES POUR OBTENIR DES EPOXYDES OPTIQUEMENT ACTIFS ET DES DIOLS VICINAUX OPTIQUEMENT ACTIFS A PARTIR D'EPOXYDES MESO

(30) Priority: 19.04.2004 ZA 200402958
(43) Date of publication of application: 21.02.2007
(73) Proprietor: CSIR, 0184 Pretoria (ZA)
(72) Inventor: BOTES, Adriana, Leonora, 0002 Pretoria (ZA); LOTTER, Jeanette, 1644 Modderfontein (ZA); LABUSCHAGNE, Michel, 0002 Pretoria (ZA)
(74) Representative: D Young & Co LLP
(86) International application number: PCT/IB2005/001022
(87) International publication number: WO 2005/100578

(56) References cited:
- WEIJERS CAREL A G M ET AL: "Epoxide hydrolases from yeast and other sources: versatile tools in biocatalysis" JOURNAL OF MOLECULAR CATALYSIS. B, ENZYMATIC, ELSEVIER, AMSTERDAM,, NL, vol. 6, no. 3, 11 March 1998 (1998-03-11), pages 199-214, XP000946716 ISSN: 1381-1177
- WEIJERS C A G M: "Enantioselective hydrolysis of aryl, alicyclic and aliphatic epoxides by Rhodotorula glutinis" TETRAHEDRON ASYMMETRY 1997 UNITED KINGDOM, vol. 8, no. 4, 1997, pages 639-647, XP002348537 ISSN: 0957-4166
- CHANG D ET AL: "Highly enantioselective hydrolysis of alicyclic meso-epoxides with a bacterial epoxide hydrolase from Sphingomonas sp. HXN-200: Simple syntheses of alicyclic vicinal trans-diols" CHEMICAL COMMUNICATIONS 21 APR 2003 UNITED KINGDOM, vol. 9, no. 8, 21 April 2003 (2003-04-21), pages 960-961, XP002348538 ISSN: 1359-7345
- BELLUCCI G ET AL: "ENANTIOSELECTIVITY OF THE ENZYMATIC HYDROLYSIS OF CYCLOHEXENE OXIDE AND RACEMIC-1 METHYLCYCLOHEXENE OXIDE A COMPARISON BETWEEN MICROSOMAL AND CYTOSOLIC EPOXIDE HYDROLASES" JOURNAL OF THE CHEMICAL SOCIETY PERKIN TRANSACTIONS I, no. 12, 1989, pages 2369-2374, XP008053581 ISSN: 0300-922X
- WISTUBA D ET AL: "ENANTIO AND REGIOSELECTIVITY IN THE EPOXIDE HYDROLASE-CATALYZED RING OPENING OF ALIPHATIC OXIRANES PART II DIALKYL AND TRIALKYL-SUBSTITUTED OXIRANES" CHIRALITY, vol. 4, no. 3, 1992, pages 185-192, XP008053537 ISSN: 0899-0042
- BELLUCCI GIUSEPPE ET AL: "Kinetics and stereochemistry of the microsomal epoxide hydrolase-catalyzed hydrolysis of cis-stilbene oxides" CHIRALITY, vol. 6, no. 7, 1994, pages 577-582, XP008053538 ISSN: 0899-0042
- BELLUCCI GIUSEPPE ET AL: "Enantioconvergent transformation of racemic cis-dialkyl substituted epoxides to (R,R) threo diols by microsomal epoxide hydrolase catalysed hydrolysis" TETRAHEDRON LETTERS, vol. 37, no. 50, 1996, pages 9089-9092, XP002348539 ISSN: 0040-4039
- VISSER H ET AL: "Cloning and characterization of an epoxide hydrolase-encoding gene from Rhodotorula glutinis" APPLIED MICROBIOLOGY AND BIOTECHNOLOGY, vol. 53, no. 4, April 2000 (2000-04), pages 415-419, XP002348540 ISSN: 0175-7598
- MADZAK C ET AL: "Heterologous Protein Expression and Secretion in the Non-conventional Yeast Yarrowia lipolytica: A Review", JOURNAL OF BIOTECHNOLOGY, ELSEVIER SCIENCE PUBLISHERS, AMSTERDAM, NL, vol. 109, no. 1-2, 1 April 2004 (2004-04-01), pages 63-81, XP002995176, ISSN: 0168-1656, DOI: 10.1016/J.JBIOTEC.2003.10.027
- LEE E Y ET AL: "Production of (S)-styrene oxide by recombinant Pichia pastoris containing epoxide hydrolase from Rhodotorula glutinis", ENZYME AND MICROBIAL TECHNOLOGY, STONEHAM, MA, US, vol. 35, no. 6-7, 1 December 2004 (2004-12-01), pages 624-631, XP004619962, ISSN: 0141-0229, DOI: 10.1016/J.ENZMICTEC.2004.08.016

## Description

### TECHNICAL FIELD

This invention relates to biocatalytic reactions, and more particularly to the use of enantiomer selective hydrolases to obtain optically active epoxides and vicinal diols.

### BACKGROUND

Optically active epoxides and vicinal diols are versatile fine chemical intermediates for use in the production of pharmaceuticals, agrochemicals, ferro-electric liquid crystals and flavours and fragrances. Epoxides are highly reactive electrophiles because of the strain inherent in the three-membered ring and the electronegativity of the oxygen. Epoxides react readily with various O-, N-, S-, and C-nucleophiles, acids, bases, reducing and oxidizing agents, allowing the production to bifunctional molecules. Vicinal diols, employed as their highly reactive cyclic sulfites and sulfates, act like epoxide-like synthons with a broad range of nucleophiles. The possibility of double nucleophilic displacement reactions with amidines and azide, allow access to dihydroimidazole derivatives, aziridines, diamines and diazides.

Desymmetrisation of *meso*-epoxides by enantioselective addition of nucleophiles gives access to bifuncional molecules in 100 % theoretical yield. For example, optically active diols prepared from *meso* epoxides which possess a C₂-axis of symmetry such as *trans*-2,3-butanediol, *trans*-cyclopentane-1,2-diol, *trans*-cyclohexane-1,2-diol and hydrobenzoin have versatile applications as chiral intermediates, chiral auxiliaries or chiral ligands. In addtion, for example, enantiomerically pure *trans* cyclohexane-1,2-diol can be employed as chiral auxiliary in conjugate addition reactions (e.g. α,β-unsaturated esters), asymmetric alkylation via chiral acetals, auxiliary for enantioselective rearrangement and cyclopropanation of allyl ethers, and preparation of chiral phosphines and crown ethers.

Epoxide hydrolases (EC 3.3.2.3) are hydrolytic enzymes that convert epoxides to vicinal diols by ring-opening of the epoxide with water. Epoxide hydrolases are present in mammals, plants, insects and microorganisms.

Weijers et al., Journal of Molecular Catalysis B: Enzymatic 6 (1999) 199-124, reviews epoxide hydrolase activity in yeasts.

### SUMMARY

The invention is based in part on the surprising discovery by the inventors that certain microorganisms express epoxide hydrolases with high enantioselectivity. These microorganisms and the associated enantioselective *meso*-epoxide hydrolase (YEMH) polypeptides of the invention hydrolyse *meso*-epoxides in favour of a particular optically active vicinal diol enantiomer product. In this respect, the genomes of the microorganims encode a polypeptide having highly enantioselective epoxide hydrolase activity.

Even though *meso*-epoxides have two chiral centers in the epoxide ring, the molecule has a plane of symmetry and the respective isomers can be superimposed on each other, and therefore these type of molecules are not optically active. Vicinal diols produced by hydrolysis of *meso*-epoxides can, with respect to their corresponding two chiral centers, be R,R; S,S; R,S; or S,R diols. The first two vicinal diols (R,R and S,S) are trans diols and are asymmetric molecules that cannot be superimposed on each other, and are thus optically active. On the other hand, the second two vicinal diols (R,S and S,R), are *cis*-diols and because they (like the *meso*-epoxides) have a plane of symmetry, can be superimposed on each other. For this reason, the latter vicinal diols (R,S and S,R), which are actually the same compound, are referred to as *meso*-diols.

Even though, as explained above, *meso*-epoxides are not optically active, and thus do not exist as optical enantiomers, reactions and enzymes (e.g., epoxide hydrolases) that effect the selective production of one optically active enantiomer of a vicinal diol over the other, are referred to herein as "enantioselective". Because the relevant reactions convert symmetrical *meso*-epoxides into asymmetrical vicinal diols, they are sometimes referred to herein as "desymmetrisations" or "desymmetrisation reactions."

More specifically, the invention provides a process for obtaining an optically active vicinal diol, which process includes the steps of: providing a *meso*-epoxide (MEO); creating a reaction mixture by mixing with the *meso*-epoxide a polypeptide, or a functional fragment thereof, having enantioselective *meso*-epoxide hydrolase activity, the polypeptide being a polypeptide encoded by a gene of a yeast cell or a gene derived from a yeast cell incubating the reaction mixture; and recovering from the reaction mixture an enantiopure, or a substantially enantiopure, vicinal diol.

Another aspect of the invention is a process for obtaining an optically active vicinal diol, which process includes the steps of: providing a *meso*-epoxide: creating a reaction mixture by mixing with the *meso*-epoxide a Yarrowia lipolytica cell comprising an exogenous nucleic acid encoding, and capable of expressing a heterologous polypeptide having enantio selective epoxide hydrolase activity;
incubating the reaction mixture; and recovering from the reaction mixture an enantiopure, or a substantially enantiopure, vicinal diol.

The following embodiments apply to both of the above processes. The cell can be a yeast cell.

The cell can be a recombinant cell, the polypeptide being encoded by a nucleic acid sequence with which the cell is transformed. The nucleic acid sequence can be an exogenous nucleic acid sequence or a heterologous nucleic acid sequence. A homologous nucleic acid sequence is taught herein. The polypeptide can be a full-length yeast epoxide hydrolase or a functional fragment of a full length yeast epoxide hydrolase.

Moreover both processes can be carried out at a pH from 5 to 10. They can be carried out at a temperature of 0°C to 70 °C. In the processes, the concentration of the styrene epoxide can be at least equal to the solubility of the styrene epoxide in water.

In both processes, the *meso*-epoxide can be a compound of the general formula (I), (H), (V), (VI) or (VII) and the vicinal diol produced hy the process is a compound of the general formula (III), (IV), (VIII), (IX), or (X), 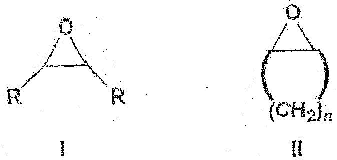 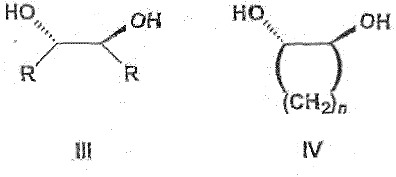 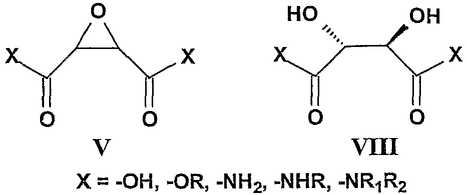 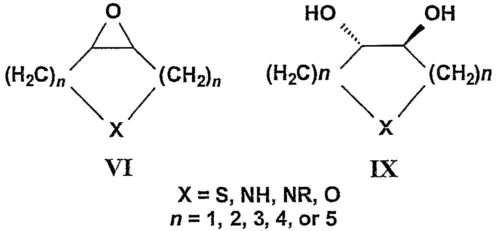 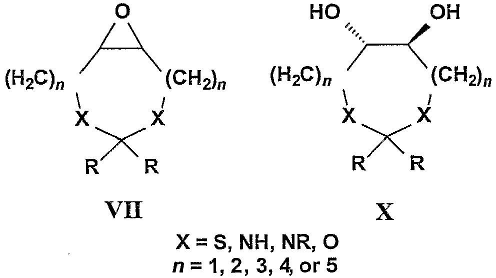 wherein,
R is selected from the group consisting of a variably substituted straight-chain or branched alkyl group, a variably substituted straight-chain or branched alkenyl group, a variably substituted straight-chain or branched alkynyl group, a variably substituted cycloalkyl group as well as cycloalkenyl groups, a variably substituted aryl group, a variably substituted aryl alkyl group, a variably substituted heterocyclic group, a variably substituted straight-chain or branched alkoxy group, a variably substituted straight-chain or branched alkenyloxy group, a variably substituted aryloxy group, a variably substituted aryl alkyloxy group, a variably substituted alkylthio group, a variably substituted alkoxycarbonyl group, a variably substituted straight chain or branched alkylamino or alkenyl amino group, a variably substituted arylamino or arylalkylamino group, a variably substituted carbamoyl group, a variably substituted acyl group, and a functional group.

The processes can include adding to the reaction mixture water and at least one water-immiscible solvent, including, for example, toluene, 1,1,2-trichlorotrifluoroethane, methyl *tert*-butyl ether, methyl isobutyl ketone, dibutyl-*o*-phtalate, aliphatic alcohols containing 6 to 9 carbon atoms, aliphatic hydrocarbons containing 6 to 16 carbon atoms, or tributyl phosphate. Alternatively, or in addition, the processes can include adding to the reaction mixture water and at least one water-miscible organic solvent, for example, acetone, methanol, ethanol, propanol, isopropanol, acetonitrile, dimethylsulfoxide, *N*,*N-*dimethylformamide, or *N*-methylpyrrolidine. In addition, or alternatively, one or more surfactants, one or more cyclodextrins, or one or more phase-transfer catalysts can be added to the reaction mixtures. Both processes can include stopping the reaction when one enantiomer of the vicinal diol is in excess compared to the other enantiomer of the vicinal diol. Furthermore, the processes can include recovering continuously during the reaction the optically active vicinal diol produced by the reaction directly from the reaction mixture.

It is taught herein that the yeast cell can be of one of the following exemplary genera: *Arxula*, *Brettanomyces*, *Bullera*, *Bulleromyces*, *Candida*, *Cryptococcus*, *Debaryomyces*, *Dekkera*, *Exophiala*, *Geotrichum*, *Hormonema, Issatchenkia*, *Kluyveromyces, Lipomyces*, *Mastigomyces*, *Myxozyma, Pichia*, *Rhodosporidium*, *Rhodotorula*, *Sporidiobo lus*, *Sporobolomyces*, *Trichosporon, Wingea,* or *Yarrowia*

Disclosed herein are yeast cells of one of the following exemplary species: *Arxula adeninivorans*, *Arxula terrestris, Brettanomyces bruxellensis*, *Brettanomyces naardenensis*, *Brettanomyces anomalus*, *Brettanomyces species* (e.g.NCYC 3151), *Bullera dendrophila*, *Bulleromyces albus*, *Candida albicans*, *Candida fabianii*, *Candida glabrata*, *Candida haemulonii*, *Candida intermedia*, *Candida magnoliaeCandida parapsilosis*, *Candida rugosa*, *Candida tenuis, Candida tropicalis*, *Candida famata*, *Candida kruisei*, *Candida sp. (new) rel to C. sorbophila, Cryptococcus albidus*, *Cryptococcus amylolentus*, *Cryptococcus bhutanensis, Cryptococcus curvatus, Cryptococcus gastricus, Cryptococcus humicola*, *Cryptococcus hungaricus*, *Cyptococcus laurentii, Cryprococcus luteolus, Cryptococcus macerans, Cryptococcus padzolicus, Cryptococcus terreus, Cryptococcus macerans, Debaryomyces hansenii, Dekkera anomala*, *Exophiala dermatitidis, Geotrichum species* (e.g. UOFS Y-0111), *Hormonema species* (e.g. NCYC 3171), *Issatchenkia occidentalis, Kluyveromyces marxianus, Lipomyces species* (e.g.UOFS Y-2159), *Lipomyces tetrasporus, Mastigomyces philipporii*, *Myxozyma melibiosi, Pichia anomala, Pichia finlandica, Pichia guillermondii, Pichia haplophila, Rhodosporidium lusitaniae, Rhodosporidium paludigenum, Rhodosporidium sphaerocarpum, Rhodosporidium toruloides, Rhodosporidium paludigenum, Rhodotorula araucariae, Rhodotorula glutinis, Rhodotorula minuta*, *Rhodotorula minuta var. minuta*, *Rhodotorula mucilaginosa, Rhodotorula philyla*, *Rhodotorula rubra*, *Rhodotorula species* (e.g. UOFS Y-2042], *Rhodotorula species* (e.g. UOFS Y-0448), *Rhodotorula species* (e.g. NCYC 3193), *Rhodotorula species* (e.g. UOFS Y-0139), *Rhodotorula secies* (e.g. UOFS Y-0560), *Rhodotorula aurantiaca, Rhodotorula species* (e.g. NCYC 3224), *Rhodotorula sp.* "*mucilaginosa*", *Sporidiobolus salmonicolor*, *Sporobolomyces holsaticus, Sporobolomyces roseus, Sporobolomyces tsugae, Trichosporon beigelii, Trichosporon cutaneum var. cutaneum, Trichosporon delbrueckii, Trichosporon jirovecii*, *Trichosporon mucoides*, *Trichosporon ovoides, Trichosporon pullulans, Trichosporon species* (e.g. UOFS Y-0861), *Trichosporon species* (e.g. UOFS Y-1615), *Trichosporon species* (e.g. UOFS Y-0451), *Trichosporon species* (e.g. NCYC 3212), *Trichosporon species* (e.g. UOFS Y-0449), *Trichosporon species* (e.g. NCYC 3211), *Trichosporon species* (e.g. UOFS Y-2113), *Trichosporon species* (e.g. NCYC 3210), *Trichosporon moniliiforme, Trichosporon montevideense, Wingea robertsiae, or Yarrowia lipolytica.*

Taught herein are also yeast cells of any of the other genera, species, or strains disclosed herein.

Disclosed herein is a method for producing a polypeptide, which process includes the steps of: providing a cell comprising a nucleic acid encoding and capable of expressing a polypeptide that has enantioselective *meso*-epoxide hydrolase activity; culturing the cell; and recovering the polypeptide from the culture. Recovering the polypeptide from the culture includes, for example, recovering it from the medium in which the cell was cultured or recovering it from the *cell per se.* The cell can be a yeast cell. The polypeptide can be encoded by an endogenous gene of the cell or the cell can be a recombinant cell, the polypeptide being encoded by a nucleic acid sequence with which the cell is transformed. The nucleic acid sequence can be an exogenous nucleic acid sequence, a heterologous nucleic acid sequence, or a homologous nucleic acid sequence. The polypeptide can be a full-length yeast epoxide hydrolase or a functional fragment of a full-length yeast epoxide hydrolase. The cell can be of any of the yeast genera, species, or strains disclosed herein or any recombinant cell disclosed herein.

A crude or pure enzyme preparation which includes an The isolated polypeptide having enantioselective *meso*-epoxide hydrolase activity is taught herein. polypeptide can be one encoded by any of the yeast genera, species, or strains disclosed herein or one encoded by a recombinant cell.

Disclosed herein is a substantially pure culture of cells, a substantial number of which comprise a nucleic acid encoding, and are capable of expressing, a polypeptide having enantioselective *meso*-epoxide hydrolase activity. The cells can be recombinant cells or cells of any of the yeast genera, species, or strains disclosed herein.

Also disclosed herein is an isolated cell, the cell comprising a nucleic acid encoding a polypeptide having enantioselective *meso*-epoxide hydrolase activity, the cell being capable of expressing the polypeptide. The cell can be any of those disclosed herein.

An isolated DNA that includes: (a) a nucleic acid sequence that encodes a polypeptide that has enantioselective *meso*-epoxide hydrolase activity and that hybridizes under highly stringent conditions to the complement of a sequence that can be SEQ ID NO: 10,11, 12, 13, 14, 15, 16, 17, or 18; or (b) the complement of the nucleic acid sequence is taught herein. The nucleic acid sequence can encode a polypeptide that includes an amino acid sequence that can be SEQ ID NO: 1, 2, 3, 4, 5, 6, 7, 8, or 9. The nucleic acid sequence can be, for example, one of those with SEQ ID NO: 10, 11, 12, 13, 14, 15, 16, 17, or 18.

Also disclosed is an isolated DNA that includes: (a) a nucleic acid sequence that is at least 55% identical to a sequence that can be SEQ ID NO: 10, 11, 12,13, 14, 15, 16,17, or 18; or (b) the complement of the nucleic acid sequence, the nucleic acid sequence encoding a polypeptide that has enantioselective *meso*-epoxide hydrolase activity.

An isolated DNA that includes: (a) a nucleic acid sequence that encodes a polypeptide consisting of an amino acid sequence that is at least 55% identical to a sequence that can be SEQ ID NO: 1, 2, 3, 4, 5, 6, 7, 8, or 9; or (b) the complement of the nucleic acid sequence, the polypeptide having enantioselective *meso*-epoxide hydrolase activity is disclosed herein.

Also included are vectors (e.g., those in which the coding sequence is operably linked to a transcriptional regulatory element) containing any of the above DNAs and cells (e.g., eukaryotic or prokaryotic cells) containing such vectors. Also taught is an isolated polypeptide encoded by any of the above DNAs. The polypeptide can include an amino acid sequence that is at least 55% identical to SEQ ID NO: 1, 2, 3, 4, 5, 6, 7, 8, or 9, the polypeptide having enantioselective styrene epoxide hydrolase activity. The polypeptide can also include:
(a) a sequence that can be SEQ ID NO: 1, 2, 3, 4, 5, 6, 7, 8, or 9, or a functional fragment of the sequence; or (b) the sequence of (a), but with no more than five conservative substitutions, the polypeptide having enantioselective *meso*-epoxide hydrolase activity.

There is also disclosed an isolated antibody (e.g., a polyclonal or a monoclonal antibody) that binds to any of the above-described polypeptides.

The term "exogenous" as used herein with reference to nucleic acid and a particular host cell refers to any nucleic acid that does not occur in (and cannot be obtained from) that particular cell as found in nature. Thus, a non-naturally-occurring nucleic acid is considered to be exogenous to a host cell once introduced into the host cell. It is important to note that non-naturally-occurring nucleic acids can contain nucleic acid subsequences or fragments of nucleic acid sequences that are found in nature provided the nucleic acid as a whole does not exist in nature. For example, a nucleic acid molecule containing a genomic DNA sequence within an expression vector is non-naturally-occurring nucleic acid, and thus exogenous to a host cell once introduced into the host cell, since that nucleic acid molecule as a whole (genomic DNA plus vector DNA) does not exist in nature. Thus, any vector, autonomously replicating plasmid, or virus (e.g., retrovirus, adenovirus, or herpes virus) that as a whole does not exist in nature is considered to be non-naturally-occurring nucleic acid. It follows that genomic DNA fragments produced by PCR or restriction endonuclease treatment as well as cDNAs are considered to be non-naturally-occurring nucleic acid since they exist as separate molecules not found in nature. It also follows that any nucleic acid containing a promoter sequence and polypeptide-encoding sequence (e.g., cDNA or genomic DNA) in an arrangement not found in nature is non-naturally-occurring nucleic acid. Nucleic acid that is naturally-occurring can be exogenous to a particular cell. For example, an entire chromosome isolated from a cell of yeast x is an exogenous nucleic acid with respect to a cell of yeast y once that chromosome is introduced into a cell of yeast y.

It will be clear from the above that "exogenous" nucleic acids can be "homologous" or "heterologous" nucleic acids. As used herein, "homologous" nucleic acids are those that are derived from a cell of the same species as the host cell and "heterologous" nucleic acids are those that are derived from a species other than that of the host cell.

In contrast, the term "endogenous" as used herein with reference to nucleic acids or genes and a particular cell refers to any nucleic acid or gene that does occur in (and can be obtained from) that particular cell as found in nature.

The MEO used by the methods of the invention can be a compound of the general formula (I) or (II) or (V) or (VI) or (VII) and the vicinal diol produced by the process can be a compound of the general formula (III), or (IV), or (VIII) or (IX) or (X), 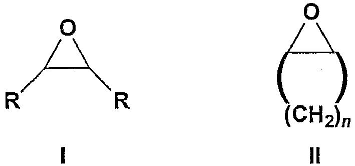 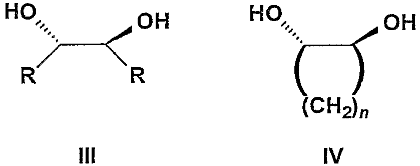 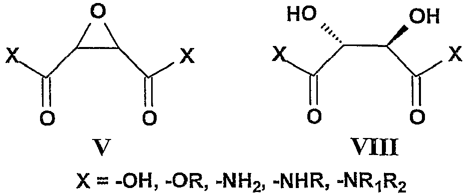 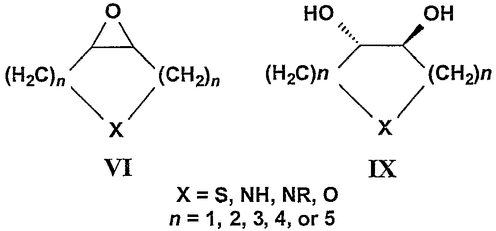 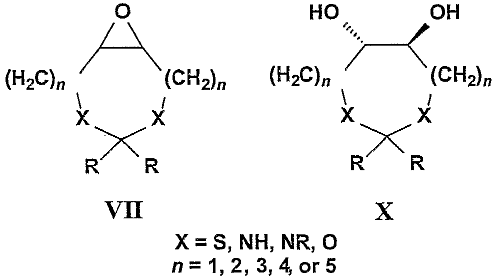 wherein:
R can be selected from the group consisting of a variably substituted straight-chain or branched alkyl group, a variably substituted straight-chain or branched alkenyl group, a variably substituted straight-chain or branched alkynyl group, a variably substituted cycloalkyl group as well as cycloalkenyl groups, a variably substituted aryl group, a variably substituted aryl alkyl group, a variably substituted heterocyclic group, a variably substituted straight-chain or branched alkoxy group, a variably substituted straight-chain or branched alkenyloxy group, a variably substituted aryloxy group, a variably substituted aryl alkyloxy group, a variably substituted alkylthio group, a variably substituted alkoxycarbonyl group, a variably substituted straight chain or branched alkylamino or alkenyl amino group, a variably substituted arylamino or arylalkylamino group, a variably substituted carbamoyl group, a variably substituted acyl group, and a functional group.
R can be present as a carbocycle or a heterocycle having 4 to 7 carbon atoms.

In an alternative embodiment, in compounds presented by general formulae (VI), (VII), (IX), and (X 'ten'), X can be in a 5-10 membered heterocyclic group containing nitrogen, oxygen, or sulfur atoms.

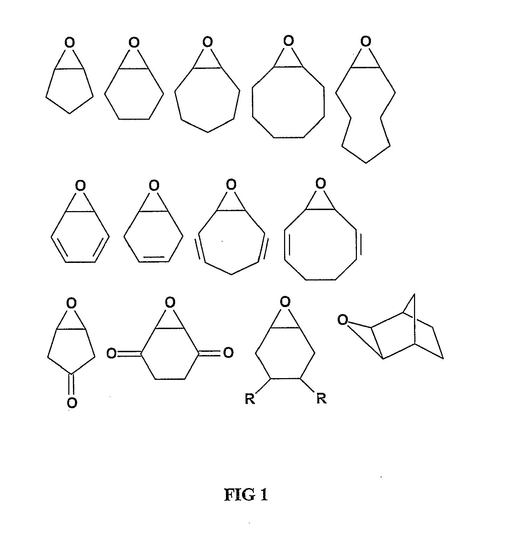

The cyclic meso epoxides presented by the general formula II can contain a saturated or unsaturated (single or more than one unsaturated bond) cycloalkyl group that can be symmetrically substituted as further exemplified in Fig 1.

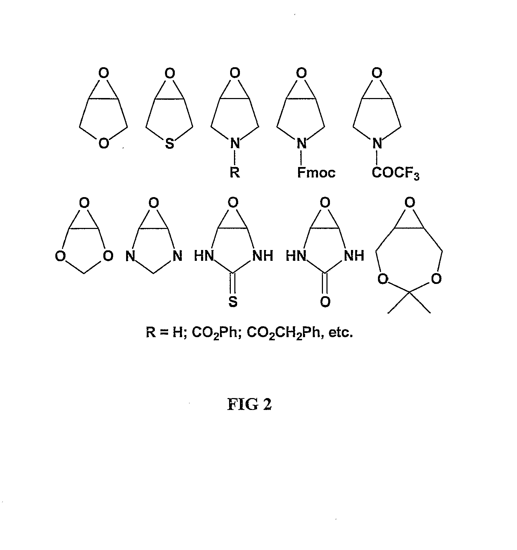

The heterocyclic meso-epoxides presented by general formula VI containing one or more heteroatoms such as O, N or S in the cycloalkyl ring, as well as substitutents on the ring, are further exemplified in Fig 2..

**The alkyl group** can be a straight chain or branched alkyl group with 1 to 12 carbon atoms. The alkyl group can be selected from the group consisting of: methyl-; ethyl-; propyl-; isopropyl-; butyl-; isobutyl-; s-butyl-; t-butyl-; pent-1-yl-; pent-2-yl-; pent-3-yl-; 2-methylbut-1-yl-; 3-methylbut-1-yl-; 2-methylbut-2-yl-; 3-methylbut-2-yl-; hex-1-yl-; hex-2-yl-; hex-3-yl-; 1-methylpent-1-yl-; 2-methylpent-1-yl-; 3-methylpent-1-yl-; 2-methylpent-2-yl-; 3-methylpent-2-yl-; 4-methylpent-2-yl-; 2-methylpent-3-yl-; 3-methylpent-3-yl-; 2-ethylbut-1-yl-; hept-1-yl-; hept-2-yl-; hept-3-yl-; hept-4-yl-; 1-methylhex-1-yl-; 2-methylhex-1-yl-; 3-methylhex-1-yl-; 4-methylhex-1-yl-; 5-methylhex-1-yl-; 2-methylhex-2-yl-; 3-methylhex-2-yl-; 4-methylhex-2-yl-; 5-methylhex-2-yl-; 2-methylhex-3-yl-; 3-methylhex-3-yl-; 4-methylhex-3-yl-; 5-methylhex-3-yl-; 2-methylhex-4-yl-; 1,1-dimethylpent-1-yl-; 1,2-dimethylpent-1-yl-; 1,3-dimethylpent-1-yl-; 1,4-dimethylpent-1-yl-; 2,2-dimethylpent-1-yl-; 2,3-dimethylpent-1-yl-; 2,4-dimethylpent-1-yl-; 2,5-dimethylpent-1-yl-; 3,3-dimethylpent-1-yl-; 3,4-dimethylpent-1-yl-; 3,5-dimethylpent-1-yl-; 4,4-dimethylpent-1-yl-; 4,5-dimethylpent-1-yl-; 5,5-dimethylpent-1-yl-; 2,2-dimethylpent-2-yl-; 2,3-dimethylpent-2-yl-; 2,4-dimethylpent-2-yl-; 3,3-dimethylpent-2-yl-; 3,4-dimethylpent-2-yl-; 2,2-dimethylpent-3-yl-; 2,3-dimethylpent-3-yl-; 2,4-dimethylpent-3-yl-; 2,2-dimethylpent-4-yl-; 2-ethylpent-1-yl-; 3-ethylpent-1-yl-; 1,1,2-trimethylbut-1-yl-; 1,2,2-trimethylbut-1-yl-; 1,2,3-trimethylbut-1-yl-; 2,2,3-trimethylbut-1-yl-; 2,3,3-trimethylbut-1-yl-; 2,3,3-but-2-yl-; 2-isopropylbut-1-yl-; 2-isopropylbut-2-yl-; oct-1-yl-; oct-2-yl-; oct-3-yl-; oct-4-yl-; 2-methylhept-1-yl-; 3-methylhept-1-yl-; 4-methylhept-1-yl-; 5-methylhept-1-yl-; 6-methylhept-1-yl-; 2-methylhept-2-yl-; 3-methylhept-2-yl-; 4-methylhept-2-yl-; 5-methylhept-2-yl-; 6-methylhept-2-yl-; 2-methylhept-3-yl-; 3-methylhept-3-yl-; 4-methylhept-3-yl-; 5-methylhept-3-yl-; 6-methylhept-3-yl-; 2-methylhept-4-yl-; 3-methylhept-4-yl-; 4-methylhept-4-yl-; 2,2-dimethylhex-1-yl-; 2,3-dimethylhex-1-yl-; 2,4-dimethylhex-1-yl-; 2,5-dimethylhex-1-yl-; 3,3-dimethylhex-1-yl-; 3,4-dimethylhex-1-yl-; 3,5-dimethylhex-1-yl-; 4,4-dimethylhex-1-yl-; 4,5-dimethylhex-1-yl-; 5,5-dimethylhex-1-yl-; 2,3-dimethylhex-2-yl-; 2,4-dimethylhex-2-yl-; 2,5-dimethylhex-2-yl-; 3,3-dimethylhex-2-yl-; 3,4-dimethylhex-2-yl-; 3,5-dimethylhex-2-yl-; 4,4-dimethylhex-2-yl-; 4,5-dimethylhex-2-yl-; 5,5-dimethylhex-2-yl-; 2,2-dimethylhex-3-yl-; 2,3-dimethylhex-3-yl-; 2,4-dimethylhex-3-yl-; 2,5-dimethylhex-3-yl-; 3,3-dimethylhex-3-yl-; 3,4-dimethylhex-3-yl-; 3,5-dimethylhex-3-yl-; 4,4-dimethylhex-3-yl-; 4,5-dimethylhex-3-yl-; 5,5-dimethylhex-3-yl-; 2,2,3-trimethylpent-1-yl-; 2,2,4-trimethylpent-1-yl-; 2,3,3-trimethylpent-1-yl-; 2,3,4-trimethylpent-1-yl-; 3,3,4-trimethylpent-1-yl-; 3,4,4-trimethylpent-1-yl-; 2,4,4-trimethylpent-1-yl-; 2,3,3-trimethylpent-2-yl-; 2,3,4-trimethylpent-2-yl-; 3,3,4-trimethylpent-2-yl-; 3,4,4-trimethylpent-2-yl-; 2,4,4-trimethylpent-2-yl-; 2,2,3-trimethylpent-3-yl-; 2-methyl-3-ethylpen-1-yl-; 3-ethyl-3-methylpent-1-yl-; 3-ethyl-4-methylpent-1-yl-; (3-methylhex-3-yl)methyl-; (4-methylhex-3-yl)methyl-; (5-methylhex-3-yl)methyl-; (2-methylhex-2-yl)methyl-; 2-methyl-3-ethylpent-2-yl-; 3-ethyl-3-methylpent-2-yl-; 3-ethyl-4-methylpent-2-yl-; 2-methyl-2-ethylpent-3-yl-; 2-methyl-3-ethylpent-3-yl-; 2,2,3,3-tetramethylbut-1-yl-; 2-ethyl-3,3-dimethylbut-2-yl; 2-isopropyl-3-methylbut-2-yl-; (3-ethylpent-3-yl)methyl-; (2,3-dimethylpent-3-yl)methyl-; (2,4-dimethylpent-3-yl)methyl-; non-1-yl-; non-2-yl-; non-3-yl-; non-4-yl-; non-5-yl-; 2-methyloct-1-yl; 3-methyloct-1-yl-; 4-methyloct-1-yl-; 5-methyloct-1-yl-; 6-methyloct-1-yl-; 7-methyloct-1-yl-; 2-methyloct-2-yl; 3-methyloct-2-yl-; 4-methyloct-2-yl-; 5-methyloct-2-yl-; 6-methyloct-2-yl-; 7-methyloct-2-yl-; 2-methyloct-3-yl; 3-methyloct-3-yl-; 4-methyloct-3-yl-; 5-methyloct-3-yl-; 6-methyloct-3-yl-; 7-methyloct-3-yl-; 2-methyloct-4-yl; 3-methyloct-4-yl-; 4-methyloct-4-yl-; 5-methyloct-4-yl-; 6-methyloct-4-yl-; 7-methyloct-4-yl-; 2,2-dimethylhept-1-yl-; 2,3-dimethylhept-1-yl-; 2,4-dimethylhept-1-yl-; 2,5-dimethylhept-1-yl-; 2,6-dimethylhept-1-yl-; 3,3-dimethylhept-1-yl-; 3,4-dimethylhept-1-yl-; 3,5-dimethylhept-1-yl-; 3,6-dimethylhept-1-yl-; 4,4-dimethylhept-1-yl-; 4,5-dimethylhept-1-yl-; 4,6-dimethylhept-1-yl-; 5,5-dimethylhept-1-yl-; 5,6-dimethylhept-1-yl-; 6,6-dimethylhept-1-yl-; 2,3-dimethylhept-2-yl-; 2,4-dimethylhept-2-yl-; 2,5-dimethylhept-2-yl-; 2,6-dimethylhept-2-yl-; 3,3-dimethylhept-2-yl-; 3,4-dimethylhept-2-yl-; 3,5-dimethylhept-2-yl-; 3,6-dimethylhept-2-yl-; 4,4-dimethylhept-2-yl-; 4,5-dimethylhept-2-yl-; 4,6-dimethylhept-2-yl-; 5,5-dimethylhept-2-yl-; 5,6-dimethylhept-2-yl-; 6,6-dimethylhept-2-yl-; 2,2-dimethylhept-3-yl-; 2,3-dimethylhept-3-yl-; 2,4-dimethylhept-3-yl-; 2,5-dimethylhept-3-yl-; 2,6-dimethylhept-3-yl-; 3,4-dimethylhept-3-yl-; 3,5-dimethylhept-3-yl-; 3,6-dimethylhept-3-yl-; 4,4-dimethylhept-3-yl-; 4,5-dimethylhept-3-yl-; 4,6-dimethylhept-3-yl-; 5,5-dimethylhept-3-yl-; 5,6-dimethylhept-3-yl-; 6,6-dimethylhept-3-yl-; 3-ethylhept-1-yl-; 3-ethylhept-1-yl-; 4-ethylhept-1-yl-; 3-ethylhept-2-yl-; 4-ethylhept-2-yl-; 5-ethylhept-2-yl-; 3-ethylhept-3-yl-; 4-ethylhept-3-yl-; 5-ethylhept-3-yl-; 3-ethylhept-4-yl-; 4-ethylhept-4-yl-; 2,2,3-trimethylhex-1-yl-; 2,2,4-trimethylhex-1-yl-; 2,2,5-trimethylhex-1-yl-; 2,3,3-trimethylhex-1-yl-; 2,3,4-trimethylhex-1-yl-; 2,3,5-trimethylhex-1-yl-; 2,4,4-trimethylhex-1-yl-; 2,4,5-trimethylhex-1-yl-; 2,5,5-trimethylhex-1-yl-; 3,3,4-trimethylhex-1-yl-; 3,3,5-trimethylhex-1-yl-; 4,4,5-trimethylhex-1-yl-; 4,5,5-trimethylhex-1-yl-; 2,3,3-trimethylhex-2-yl-; 2,3,4-trimethylhex-2-yl-; 2,3,5-trimethylhex-2-yl-; 2,4,4-trimethylhex-2-yl-; 2,4,5-trimethylhex-2-yl-; 2,5,5-trimethylhex-2-yl-; 3,3,4-trimethylhex-2-yl-; 3,3,5-trimethylhex-2-yl-; 3,4,4-trimethylhex-2-yl-; 3,4,5-trimethylhex-2-yl-; 3,5,5-trimethylhex-2-yl-; 4,4,5-trimethylhex-2-yl-; 4,5,5-trimethylhex-2-yl-; 2,2,3-trimethylhex-3-yl-; 2,2,4-trimethylhex-3-yl-; 2,2,5-trimethylhex-3-yl-; 2,3,4-trimethylhex-3-yl-; 2,3,5-trimethylhex-3-yl-; 2,4,4-trimethylhex-3-yl-; 2,4,5-trimethylhex-3-yl-; 2,5,5-trimethylhex-3-yl-; 4,4,5-trimethylhex-3-yl-; 4,5,5-trimethylhex-3-yl-; (2-methylhex-3-yl)methyl-; 3-ethyl-2-methylhex-1-yl-; 3-ethyl-3-methylhex-1-yl-; 3-ethyl-4-methylhex-1-yl-; 3-ethyl-5-methylhex-1-yl-; 4-ethyl-2-methylhex-1-yl-; 4-ethyl-3-methylhex-1-yl-; 4-ethyl-4-methylhex-1-yl-; 4-ethyl-5-methylhex-1-yl-; (2-methylhex-1-yl)methyl-; (3-methylhex-1-yl)methyl-; (4-methylhex-1-yl)methyl-; (5-methylhex-1-yl)methyl-; (6-methylhex-1-yl)methyl-; 3-isopropylhex-1-yl-; 4-ethyl-5-methylhex-1-yl-; 3-ethyl-3-methylhex-2-yl-; 3-ethyl-4-methylhex-2-yl-; 3-ethyl-5-methylhex-2-yl-; 4-ethyl-2-methylhex-2-yl-; 4-ethyl-3-methylhex-2-yl-; 4-ethyl-4-methylhex-2-yl-; 4-ethyl-5-methylhex-2-yl-; 3-isopropylhex-2-yl-; 4-ethyl-5-methylhex-2-yl-; 3-ethyl-2-methylhex-3-yl-; 3-ethyl-4-methylhex-3-yl-; 3-ethyl-5-methylhex-3-yl-; 4-ethyl-2-methylhex-3-yl-; 4-ethyl-3-methylhex-3-yl-; 4-ethyl-4-methylhex-3-yl-; 4-ethyl-5-methylhex-3-yl-; 4-isopropylhex-1-yl-; 2,2,3,3-tetramethylpent-1-yl-; 2,2,3,4-tetramethylpent-1-yl-; 2,2,4,4-tetramethylpent-1-yl-; 2,3,3,4-tetramethylpent-1-yl-; 2,3,4,4-tetramethylpent-1-yl-; 2,3,4,4-tetramethylpent-1-yl-; 3,3,4,4-tetramethylpent-1-yl-; 2,3,3,4-tetramethylpent-2-yl-; 2,3,4,4-tetramethylpent-2-yl-; 2,3,4,4-tetramethylpent-2-yl-; 3,3,4,4-tetramethylpent-2-yl-; 2,2,3,4-tetramethylpent-3-yl-; 2,2,4,4-tetramethylpent-3-yl-; 2,3,4,4-tetramethylpent-3-yl-; 2,3,4,4-tetramethylpent-3-yl-; (3-ethylhex-3-yl)methyl-; (4-ethylhex-3-yl)methyl-; (5-methylhept-3-yl)methyl-; 2,4-dimethyl-3-ethylpent-1-yl-; 3,4-dimethyl-3-ethylpent-1-yl-; 4,4-dimethyl-3-ethylpent-1-yl-; 2-ethyl-2-methylhex-1-yl-; 3-ethyl-2-methylhex-1-yl-; 4-ethyl-2-methylhex-1-yl-; 2-ethyl-3-methylhex-1-yl-; 2-ethyl-4-methylhex-1-yl-; 3-ethyl-3-methylhex-1-yl-; 3-ethyl-4-methylhex-1-yl-; 3-ethyl-5-methylhex-1-yl-; 4-ethyl-3-methylhex-1-yl-; 4-ethyl-4-methylhex-1-yl-; 4-ethyl-5-methylhex-1-yl-; and the like from dec-1-yl-; dec-2-yl-; dec-3-yl-; dec-4-yl-; dec-5-yl-; dec-6-yl-; undec-1-yl-; undec-2-yl-; undec-3-yl-; undec-4-yl-; undec-5-yl-; undec-6-yl-; undec-7-yl-; dodec-1-yl; dodec-2-yl; dodec-3-yl; dodec-4-yl; dodec-5-yl; dodec-6-yl groups. Preferably, the alkyl group is a straight chain or branched alkyl group with 1 to 8 carbon atoms.

**The alkenyl group** can be a straight chain or branched alkenyl group having 2-12 carbon atoms. The alkenyl group can be selected from the group consisting of: vinyl-; allyl-; α-methallyl-; β-methallyl-; 1-propenyl-; isopropenyl-; 1-butenyl-; 2-butenyl-; 3-butenyl; 1-buten-2-yl-; 1-buten-3-yl-; 1-methyl-1-propenyl-; 2-methyl-1-propenyl-; 1-pentenyl-; 2-pentenyl-; 3-pentenyl-; 4-pentenyl-; 1-penten-2-yl-; 1-penten-3-yl-; 2-methyl-1-butenyl-; 1-hexenyl-; 2-hexenyl-; 3-hexenyl-; 4-hexenyl-; 5-hexenyl-; 1-heptenyl-; 2-heptenyl-; 3-heptenyl-; 4-heptenyl-; 5-heptenyl-; 6-heptenyl-; 1-octenyl-; 2-octenyl-; 3-octenyl-; 4-octenyl-; 5-octenyl-; 6-octenyl-; 7-octenyl-; 1-nonenyl-; 2-nonenyl-; 3-nonenyl-; 4-nonenyl-; 5-nonenyl-; 6-nonenyl-; 7-nonenyl-; 8-nonenyl-; 1-decenyl-; 2-decenyl-; 3-decenyl-; 4-decenyl-; 5-decenyl-; 6-decenyl-; 7-decenyl-; 8-decenyl-; 9-decenyl-; 1-undecenyl; 2-undecenyl; 3-undecenyl; 4-undecenyl; 5-undecenyl; 6-undecenyl; 7-undecenyl; 8-undecenyl; 9-undecenyl; 10-undecenyl; 1-dodecenyl; 2-dodecenyl; 3-dodecenyl; 4-dodecenyl; 5-dodecenyl; 6-dodecenyl; 7-dodecenyl; 8-dodecenyl; 9-dodecenyl; 10-dodecenyl; 11-dodecenyl groups; and related branched isomers. Preferably, the group is a straight chain or branched alkenyl group with 2 to 8 carbon atoms.

**The alkynyl group** can a straight chain or branched alkynyl group having 2-12 carbon atoms. The alkynyl group can be selected from the group consisting of: ethynyl-; 1-propynyl-; 2-propynyl-; 1-butynyl-; 2-butynyl-; 3-butynyl-; 1-pentynyl-; 2-pentynyl-; 3-pentynyl-; 4-pentynyl-; 1-hexynyl-; 2-hexynyl-; 3-hexynyl-; 4-hexynyl-; 5-hexynyl-; 1-heptynyl-; 2-heptynyl-; 3-heptynyl-; 4-heptynyl-; 5-heptynyl-; 6-hepynyl-; 1-octynyl-; 2-octynyl-; 3-octynyl-; 4-octynyl-; 5-octynyl-; 6-octynyl-; 7-octynyl-; 1-nonynyl-; 2-nonynyl-; 3-nonynyl-; 4-nonynyl-; 5-nonynyl-; 6-nonynyl-; 7-nonynyl-; 8-nonynyl-; 1-decynyl-; 2-decynyl-; 3-decynyl-; 4-decynyl-; 5-decynyl-; 6-decynyl-; 7-decynyl-; 8-decynyl-; 9-decynyl-; 1-undecynyl-; 2-undecynyl-; 3-undecynyl-; 4-undecynyl-; 5-undecynyl-; 6-undecynyl-; 7-undecynyl-; 8-undecynyl-; 9-undecynyl-; 10-undecynyl-; 1-dodecynyl-; 2-dodecynyl-; 3-dodecynyl-; 4-dodecynyl-; 5-dodecynyl-; 6-dodecynyl-; 7-dodecynyl-; 8-dodecynyl-; 9-dodecynyl-; 10-dodecynyl-; 11-dodecynyl- groups; and related branched isomers. Preferably, the alkynyl group is a straight chain or branched alkenyl group with 2 to 8 carbon atoms.

**The cycloalkyl group** can be cycloalkyl groups with 3 to 10 carbon atoms. The cycloalkyl group can be selected from the group consisting of: cyclopropyl-; cyclobutyl-; cyclopentyl-; cyclohexyl-; cycloheptyl-; and cyclooctyl- groups. These groups can be variably substituted at any position(s) around the ring. Preferably, the cycloalkyl group is a cycloalkyl group with 5 to 8 carbon atoms.

**The cycloalkenyl group** can be cycloalkenyl groups with 3 to 10 carbon atoms. The cycloalkenyl group can be selected from the group consisting of: cyclobutenyl-; cyclopentenyl-; cyclohexenyl-; cycloheptenyl-; and cyclooctenyl- groups. This group can variably be substituted at any position(s) around the ring. Preferably, the cycloalkenyl group is a cycloalkenyl group with 5 to 7 carbon atoms.

**The aryl group** can be selected from the group consisting of phenyl; biphenyl; naphtyl; anthracenyl groups; and the like. Preferably; the aryl group is a phenyl group.

**The aryl alkyl group** can be a group with 7 to 18 carbons. The aryl alkyl group can be selected from the group consisting of: benzyl-; 1-methylbenzyl-; 2-phenylethyl-; 3-phenylpropyl-; 4-phenylbutyl-; 5-phenylpentyl-; 6-phenylhexyl-; 1-naphtylmethyl; and 2-(1-naphtyl)-ethyl groups; and the like. Preferably, the aryl alkyl group is an aryl alkyl group with 7 to 12 carbon atoms.

**The heterocyclic group** represented by R in structure of general formula I, or in alternative embodiments of X in the compounds of general structure (VI), (VII), (IX), and (X), can include 5- to 10-membered heterocyclic groups containing, as heteroatom X, nitrogen, oxygen, or sulfur. The heterocyclic ring can be fused with a cyclic or aromatic ring having 3 to 7 carbon atoms such as: benzene; cyclopropyl; cyclobutane; cyclopentane; and cyclohexane ring systems. Preferably the heterocyclic ring has 5 or 6 carbon atoms. The heterocyclic ring can be selected from the group consisting of: furyl-; dihydrofuranyl-; tetrahydrofuranyl-; dioxolanyl-; oxazolyl-; dihydrooxazolyl-; oxazolidinyl-; isoxazolyl-; dihydroisoxazolyl-; isoxazolidinyl-; oxathiolanyl-; thienyl-; tetrahydrothienyl-; dithiolanyl-; thiazolyl-; dihydrothiazolyl-; thiazolidinyl-; isothiazolyl-; dihydroisothiazolyl-; isothiazolidinyl-; pyrrolyl-; dihydropyrrolyl-; pyrrolidinyl-; pyrazolyl-; dihydropyrazolyl-; pyrazolidinyl-; imidazolyl-; dihydroimidazolyl-; imidazolidinyl-; triazolyl-; dihydrotriazolyl-; triazolidinyl-; tetrazolyl-; dihydrotetrazolyl- ; tetrazolidinyl-; pyridyl-; dihydropyridyl-; piperidinyl-; morpholinyl-; dioxanyl-; oxathianyl-; trioxanyl-; thiomorpholinyl-; pyridazinyl-; dihydropyridazinyl-; tetrahydropyridazinyl-; hexahydropyridazinyl-; pyrimidinyl-; dihydropyrimadinyl-; tetrahydropyrimadinyl-; hexahydropyrimadinyl-; pyrazinyl-; piperazinyl-; pyranyl-; dihydropyranyl-; tetrahydropyranyl-; thiopyranyl-; dihydrothiopyranyl-; tetrahydrothiopyranyl-; dithianyl-; purinyl-; pyrimidinyl-; pyrrolizinyl-; pyrrolizidinyl; indolyl-; dihydroindolyl-; isoindolyl-; indolizinyl-; indolizidinyl-; quinolyl-; dihydroquinolyl-; tetrahydroquinolyl-; isoquinolyl-; dihydroquinolyl-; tetrahydroquinolyl-; quinolizinyl-; quinolizidinyl-; phenanthrolinyl-; chromenyl-; chromanyl-; isochromenyl-; isochromanyl-; benzofuranyl-; carbazolyl- groups; and tetrahydrofurane; 1,3-dioxolane; tetrahydrotiophene; pyrrolidine; dioxolane; 2-imidazolidinethione; 2-imidazolidene; dioxane, piperazine; and dithione (see Fig. 2). In compounds of general formula (I) and (III), the heterocyclic ring can befused with a cyclic or aromatic ring having 3 to 7 carbon atoms such as:benzene; cyclopropyl; cyclobutane; cyclopentane; and cyclohexane ring systems.

**The alkoxy group** can be a straight chain or branched alkoxy group having 2-12 carbon atoms such as: methoxy; ethoxy; propyloxy; isopropyloxy; butyloxy; isobutyloxy; tert-butyloxy; pentyloxy; hexyloxy; heptyloxy; or octyloxy.

**The alkenyloxy group** can be a straight chain or branched alkenyloxy group having 2-12 carbon atoms. The alkenyloxy group can be selected from the group consisting of: ethynyloxy-; 1-propynyloxy-; 2-propynyloxy-; 1-butynyloxy-; 2-butynyloxy-; 3-butynyloxy-; 1-pentynyloxy-; 2-pentynyloxy-; 3-pentynyloxy-; 4-pentynyloxy-; 1-hexynyloxy-; 2-hexynyloxy-; 3-hexynyloxy-; 4-hexynyloxy-; 5-hexynyloxy-; 1-heptynyloxy-; 2-heptynyloxy-; 3-heptynyloxy-; 4-heptynyloxy-; 5-heptynyloxy-; 6-heptynyloxy-; 1-octynyloxy-; 2-octynyloxy-; 3-octynyloxy-; 4-octynyloxy-; 5-octynyloxy-; 6-octynyloxy-; 7-octynyloxy-; 1-nonynyloxy-; 2-nonynyloxy-; 3-nonynyl-oxy-; 4-nonynyloxy-; 5-nonynyloxy-; 6-nonynyloxy-; 7-nonynyloxy-; 8-nonynyloxy-; 1-decynyloxy-; 2-decynyloxy-; 3-decynyloxy-; 4-decynyloxy-; 5-decynyloxy-; 6-decynyloxy-; 7-decynyloxy-; 8-decynyloxy-; 9-decynyloxy-; 1-undecynyloxy-; 2-undecynyloxy-; 3-undecynyloxy-; 4-undecynyloxy-; 5-undecynyloxy-; 6-undecynyloxy-; 7-undecynyloxy-; 8-undecynyloxy-; 9-undecynyloxy-; 10-undecynyloxy-; 1-dodecynyloxy-; 2-dodecynyloxy-; 3-dodecynyloxy-; 4-dodecynyloxy-; 5-dodecynyloxy-; 6-dodecynyloxy-; 7-dodecynyloxy-; 8-dodecynyloxy-; 9-dodecynyloxy-; 10-dodecynyloxy-; 11-dodecynyloxy- groups; and related branched isomers. Preferably, the alkenyloxy group is a straight chain or branched alkenyloxy groups with 2 to 8 carbon atoms.

**The aryloxy group** can be an aryloxy group, such as a phenoxy or naphtyloxy group (e.g.: phenoxy; 2-methylphenoxy; 3-methylphenoxy; 4-methylphenoxy; 2-allylphenoxy; 2-chlorophenoxy; 3-chlorophenoxy; 4-chlorophenoxy; 4-methoxyphenoxy; 2-allyloxyphenoxy; naphtyloxy; and the like). The group can optionally be substituted with an alkyl or alkenyl or alkoxy group having 1 to 4 carbon atoms or halogens.

**The aryl alkyloxy group** can be benzyloxy or 2-phenylethyloxy.

**The alkylamino group** can be a straight chain or branched alkylamino group having 2-12 carbon atoms such as: methylamino; ethylamino; propylamino; isopropylamino; butylamino; isobutylamino; tert-butylamino; pentylamino; hexylamino; heptylamino; or octylamino.

**The alkenyl amino group** can be a straight chain or branched alkenylamino group having 2-12 carbon atoms. The alkenyl amino group can be selected from the group consisting of: ethynylamino-; 1-propynylamino-; 2-propynylamino-; 1-butynylamino-; 2-butynylamino-; 3-butynylamino-; 1-pentynylamino-; 2-pentynylamino- ; 3-pentynylamino-; 4-pentynylamino-; 1-hexynylamino-; 2-hexynylamino-; 3-hexynylamino-; 4-hexynylamino-; 5-hexynylamino-; 1-heptynylamino-; 2-heptynylamino-; 3-heptynylamino-; 4-heptynylamino-; 5-heptynylamino-; 6-heptynylamino-; 1-octynylamino-; 2-octynylamino-; 3-octynylamino-; 4-octynylamino-; 5-octynylamino-; 6-octynylamino-; 7-octynylamino-; 1-nonynylamino-; 2-nonynylamino- ; 3-nonynyl-amino; 4-nonynylamino-; 5-nonynylamino-; 6-nonynylamino-; 7-nonynylamino-; 8-nonynylamino-; 1-decynylamino-; 2-decynylamino-; 3-decynylamino-; 4-decynylamino-; 5-decynylamino-; 6-decynylamino-; 7-decynylamino-; 8-decynylamino-; 9-decynylamino-; 1-undecynylamino-; 2-undecynylamino-; 3-undecynylamino-; 4-undecynylamino-; 5-undecynylamino-; 6-undecynylamino-; 7-undecynylamino-; 8-undecynylamino-; 9-undecynylamino-; 10-undecynylamino-; 1-dodecynylamino-; 2-dodecynylamino-; 3-dodecynylamino-; 4-dodecynylamino-; 5-dodecynylamino-; 6-dodecynylamino-; 7-dodecynylamino-; 8-dodecynylamino-; 9-dodecynylamino-; 10-dodecynylamino-; and 11-dodecynylamino- groups; and related branched isomers. Preferably, the alkenyl amino group is a straight chain or branched alkenylamino group with 2 to 8 carbon atoms.

**The arylamino group** can be an arylamino group such as a phenylamino or naphtylamino group, optionally substituted with an alkyl or alkenyl or alkoxy group having 1 to 4 carbon atoms, or halogens. The arylamino group can be selected from the group consisting of: phenylamino; 2-methylphenylamino; 3-methylphenylamino; 4-methylphenylamino; 2-allylphenylamino; 2-chlorophenylamino; 3-chlorophenylamini; 4-chlorophenylamino; 4-methoxyphenylamino; 2-allyloxyphenylamino; naphtylamino; and the like.

**The arylalkylamino group** can be benzylamino or 2-phenylethylamino.

**The alkylthio group** can be an alkylthio group having 1 to 8 carbon atoms. The alkylthio group can be selected from the group consisting of: methylthio; ethylthio; propylthio; butylthio; isobutylthio; and pentylthio.

**The alkenylthio group** can be a straight chain or branched alkenylthio group having 1 to 8 carbon atoms. The alkenylthio group can be selected from the group consisting of: ethynylthio-; 1-propynylthio-; 2-propynylthio-; 1-butynylthio-; 2-butynylthio-; 3-butynylthio-; 1-pentynylathio-; 2-pentynylthio-; 3-pentynylthio-; 4-pentynylthio-; 1-hexynylthio-; 2-hexynylthio-; 3-hexynylthio-; 4-hexynylthio-; 5-hexynylthio-; and the like.

**The arylrthio group** can be an alkenylthio group having 1 to 8 carbon atoms such as a phenylthio or naphtylthio group, which can optionally be substituted with an alkyl or alkenyl or alkoxy group having 1 to 4 carbon atoms; and also halogens, e.g.: phenylthio; 2-methylphenylthio; 3-methylphenylthio; 4- methylphenylthio; 2-allylphenylthio; 2-chlorophenylthio; 3-chlorophenylamini; 4-chlorophenylthio; 4-methoxyphenylthio; 2-allyloxyphenylthio; naphtylthio; and the like.

**The arylalkylthio group** can be an alkenylthio group having 1 to 8 carbon atoms such as a benzylthio- group or a 2-phenylethylthio-group.

**The alkoxycarbonyl group** can be: methoxycarbonyl; ethoxycarbonyl; or the like.

**The substituted or unsubstituted carbamoyl group** can be: carbamoyl; methylcarbamoyl; dimethylcarbamoyl; diethylcarbamoyl; or the like.

**The acyl group** can be an acyl group with 1 to 8 carbon atoms such as: formyl; acetyl; propionyl; or benzoyl groups; or the like.

The alkyl, alkenyl, alkynyl, cycloalkyl, aryl, aryl alkyl, heterocyclic, alkoxy, alkenyloxy, aryloxy, aryl alkyloxy, alkylamino, alkenylamino, arylamino, arylalkylamino, alkylthio, alkenylthio, arylthio, arylalkylthio, alkoxycarbonyl, substituted and unsubstituted carbamoyl and acyl groups mentioned above can optionally be substituted. Examples of substituents include: halogens (F; Cl; Br; I); hydroxyl groups; mercapto groups; carboxylates; nitro groups; cyano groups; substituted or unsubstituted amino groups (including amino, methylamino, dimethylamino, ethylamino, diethylamino, and various protected amines such as tert-butoxycarbonyl- and arylsulfonamido groups); alkoxy groups (having 1 to 8 carbon atoms such as methoxy, ethoxy, propyloxy, isopropyloxy, butyloxy, isobutyloxy, tert-butyloxy, pentyloxy, hexyloxy, heptyloxy or octyloxy); alkenyloxy groups (having 2 to 8 carbon atoms such as a vinyloxy; allyloxy; 3-butenyloxy or 5-hexenyloxy); aryloxy groups (such as a phenoxy or naphtyloxy group which can be optionally substituted with an alkyl or alkenyl or alkoxy group having 1 to 4 carbon atoms; and also halogens, e.g., phenoxy, 2-methylphenoxy, 3-methylphenoxy, 4-methylphenoxy, 2-allylphenoxy, 2-chlorophenoxy, 3-chlorophenoxy, 4-chlorophenoxy, 4-methoxyphenoxy, 2-allyloxyphenoxy, naphtyloxy, and the like); aryl alkyloxy groups (e.g., benzyloxy and 2-phenylethyloxy); alkylthio groups (having 1 to 8 carbon atoms such as methylthio, ethylthio, propylthio, butylthio, isobutylthio, pentylthio); alkoxycarbonyl groups (e.g. methoxycarbonyl, ethoxycarbonyl, and the like); substituted or unsubstituted carbamoyl group (e.g. carbamoyl, methylcarbamoyl, dimethylcarbamoyl, diethylcarbamoyl and the like); acyl groups (with 1 to 8 carbon atoms such as formyl, acetyl, propionyl, or benzoyl groups); and others.

The above-mentioned cycloalkyl, cycloalkenyl, aryl, aryl alkyl, heterocyclic, alkoxy, alkenyloxy, aryloxy, aryl alkyloxy, alkylthio, and alkoxycarbonyl groups can also be substituted with alkyl groups having 1 to 10 carbon atoms, alkenyl groups with 2 to 10 carbon atoms, or haloalkyl groups with 1 to 10 carbon atoms in addition to the substituents specified above.

The number of substituents can be one or more than one. The substituents can be the same or different.

The R group can be a functional group. The functional group is selected from the group consisting of: halo, pseudohalo, hydroxyl, variably substituted mercapto, variably substituted sulfinyl, variably substituted sulfonyl, carboxylates, variably substituted amino, variably substituted amido, variably substituted ureido, variably substituted carbamoyl, and variably substituted urethano. Pseudohalo is nitro, cyano, azido, cyanato, isocyanato, or isothiocyanato.

The R group can also be present as a saturated or unsaturated optionally substituted carbocycle or a heterocycle having 5 to 12 carbon atoms, such as: cyclopentane; cyclohexane; cyclohexene; cyclohexadiene; cycloheptane; cyclooctane; cyclononane; or cyclodecane.

As used herein, a "variably substituted" group is a group that is unsubstituted or is substituted with one or more, in another embodiment one to five, in another embodiment one, two or three, substituents.

"*n*" in general formulae (II) and (IV) can be 2-8, i.e., 2, 3,4 5, 6, 7 or 8.

"Polypeptide" and "protein" are used interchangeably and mean any peptide-linked chain of amino acids, regardless of length or post-translational modification. The invention also features YEMH polypeptides with conservative substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The term "isolated" polypeptide or peptide fragment, as used herein, refers to a polypeptide or a peptide fragment which either has no naturally-occurring counterpart or has been separated or purified from components which naturally accompany it, e.g., microorganism cellular components such as yeast cell cellular components. Typically, the polypeptide or peptide fragment is considered "isolated" when it is at least 70%, by dry weight, free from the proteins and other naturally-occurring organic molecules with which it is naturally associated. Preferably, a preparation of a polypeptide (or peptide fragment thereof) of the invention is at least 80%, more preferably at least 90%, and most preferably at least 99%, by dry weight, the polypeptide (or the peptide fragment thereof), respectively, of the invention. Thus, for example, a preparation of polypeptide z is at least 80%, more preferably at least 90%, and most preferably at least 99%, by dry weight, polypeptide z. Since a polypeptide that is chemically synthesized is, by its nature, separated from the components that naturally accompany it, the synthetic polypeptide is "isolated."

An isolated polypeptide (or peptide fragment) of the invention can be obtained, for example, by: extraction from a natural source (e.g., from yeast cells); expression of a recombinant nucleic acid encoding the polypeptide; or chemical synthesis. A polypeptide that is produced in a cellular system different from the source from which it naturally originates is "isolated," because it will necessarily be free of components which naturally accompany it. The degree of isolation or purity can be measured by any appropriate method, e.g., column chromatography, polyacrylamide gel electrophoresis, or HPLC analysis.

An "isolated DNA" is either (1) a DNA that contains sequence not identical to that of any naturally occurring sequence, or (2), in the context of a DNA with a naturally-occurring sequence (e.g., a cDNA or genomic DNA), a DNA free of at least one of the genes that flank the gene containing the DNA of interest in the genome of the organism in which the gene containing the DNA of interest naturally occurs. The term therefore includes a recombinant DNA incorporated into a vector, into an autonomously replicating plasmid or virus, or into the genomic DNA of a prokaryote or eukaryote. The term also includes a separate molecule such as: a cDNA (e.g., SEQ ID NOs: 10, 11, 12, 13, 14, 15, 16, 17, or 18) where the corresponding genomic DNA can have introns and therefore can have a different sequence; a genomic fragment that lacks at least one of the flanking genes; a fragment of cDNA or genomic DNA produced by polymerase chain reaction (PCR) and that lacks at least one of the flanking genes; a restriction fragment that lacks at least one of the flanking genes; a DNA encoding a non-naturally occurring protein such as a fusion protein, mutein, or fragment of a given protein; and a nucleic acid which is a degenerate variant of a cDNA or a naturally occurring nucleic acid. In addition, it includes a recombinant nucleotide sequence that is part of a hybrid gene, i.e., a gene encoding a non-naturally occurring fusion protein. Also included is a recombinant DNA that includes a portion of SEQ ID NOs: 10-18. It will be apparent from the foregoing that isolated DNA does not mean a DNA present among hundreds to millions of other DNA molecules within, for example, cDNA or genomic DNA libraries or genomic DNA restriction digests in, for example, a restriction digest reaction mixture or an electrophoretic gel slice.

As used herein, a "functional fragment" of a YEMH polypeptide is a fragment of the polypeptide that is shorter than the full-length polypeptide and has at least 20% (e.g., at least: 30%; 40%; 50%; 60%; 70%; 80%; 90%; 95%; 98%; 99%; 100%, or more) of the ability of the full-length polypeptide to enantioselectively hydrolyse a MEO of interest. Fragments of interest can be made by either recombinant, synthetic, or proteolytic digestive methods and tested for their ability to enantioselectively hydrolyse a MEO.

As used herein, "operably linked" means incorporated into a genetic construct so that an expression control sequence effectively controls expression of a coding sequence of interest.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

The materials, methods, and examples disclosed herein are illustrative only and not intended to be limiting.

Other features and advantages of the invention, e.g., vicinal diols substantially enriched for one optical enantiomer, will be apparent from the following description, from the drawings and from the claims.

### DESCRIPTION OF DRAWINGS

Fig. 1 is a depiction of the chemical structures of exemplary MEO represented by general formula (II) that are useful for the invention.
Fig. 2 is a depiction of the chemical structures of exemplary MEO represented by general formula (VI) and (VII) that have heteroatoms in the cyclic ring and are useful for the invention.
Fig. 3 is a schematic representation of desymmetrisation of five exemplary MEO (cis-2,3- epoxybutane, cyclohexene oxide, cyclopentene oxied, cis-epoxysuccinic acid and 3,4-epoxytetrahydrofuran) by epoxide hydrolases to the corresponding R,R-diols.
Fig. 4 is a restriction map of the pYLHmA (pINA1291) expression vector. The positions of the hp4d promoter and LIP2 terminator and of unique restriction sites available for the insertion of coding sequences are indicated.
Fig. 5 is a restriction map of the pYLTsA (pINA3313) expression vector. The positions of the TEF promoter and LIP2 terminator and of unique restriction sites available for the insertion of coding sequences are indicated.

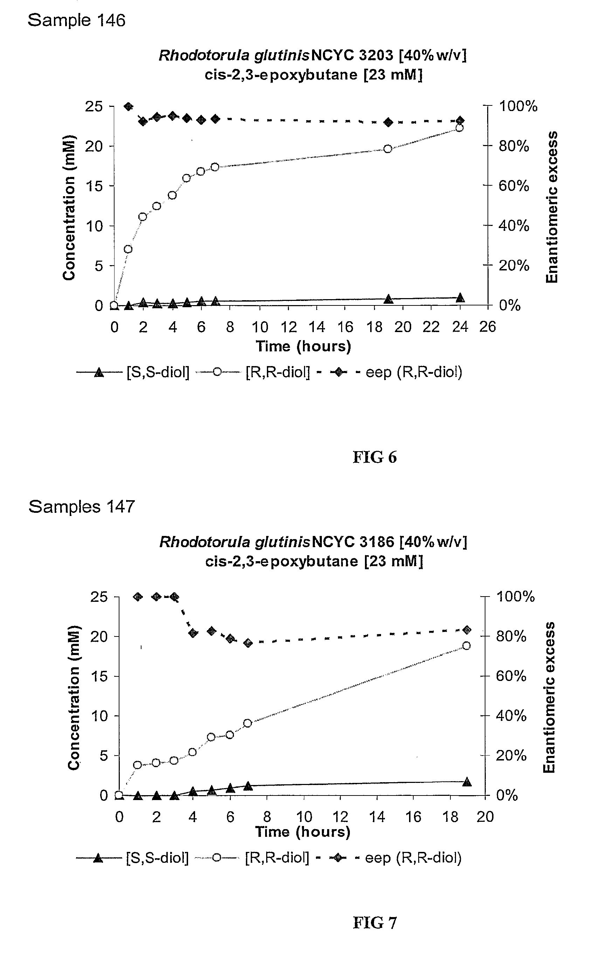

Figs. 6-8 (Samples 146-148) are line graphs showing the desymmetrisation of cis-2,3- epoxybutane by the indicated wild-type yeast strains. The left y-axis (and lines with shaded circle and triangle data points) in each graph show the changes in concentrations of the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis. The biocatalyst loadings are indicated in brackets beside the strain names in each figure. The percentage biocatalyst loading refers to a percentage wet weight of yeast cells or an equivalent wet weight of lysed/broken yeast cells in the aqueous fraction of the reaction matrix. In all these instances the value of the percentage wet weight of biocatalyst is approximately five-fold the value of the equivalent dry weight of biocatalyst. The biocatalyst loading are indicated in a similar fashion in all the biotransformation reaction profile graphs shown in the figures described below.

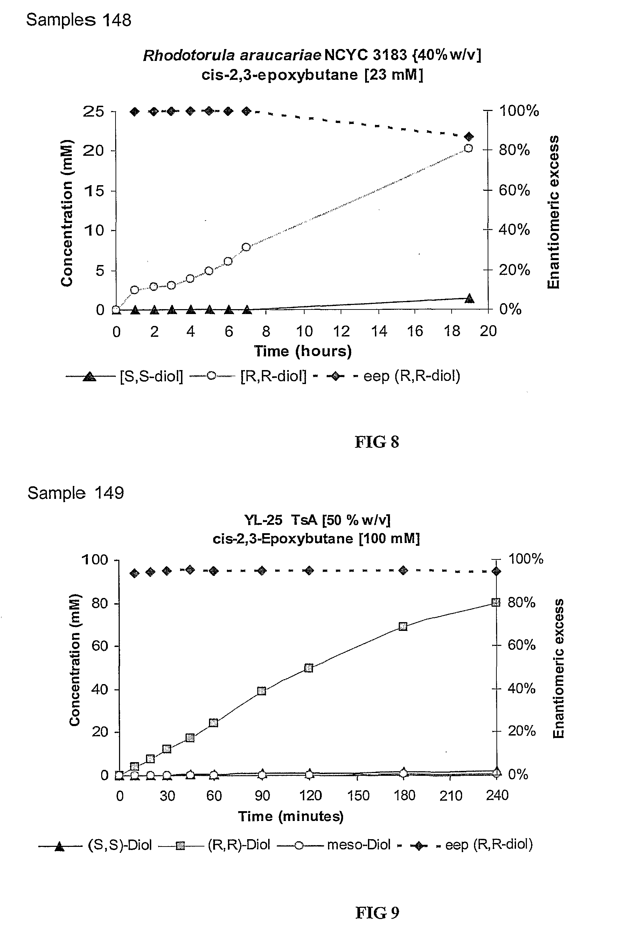

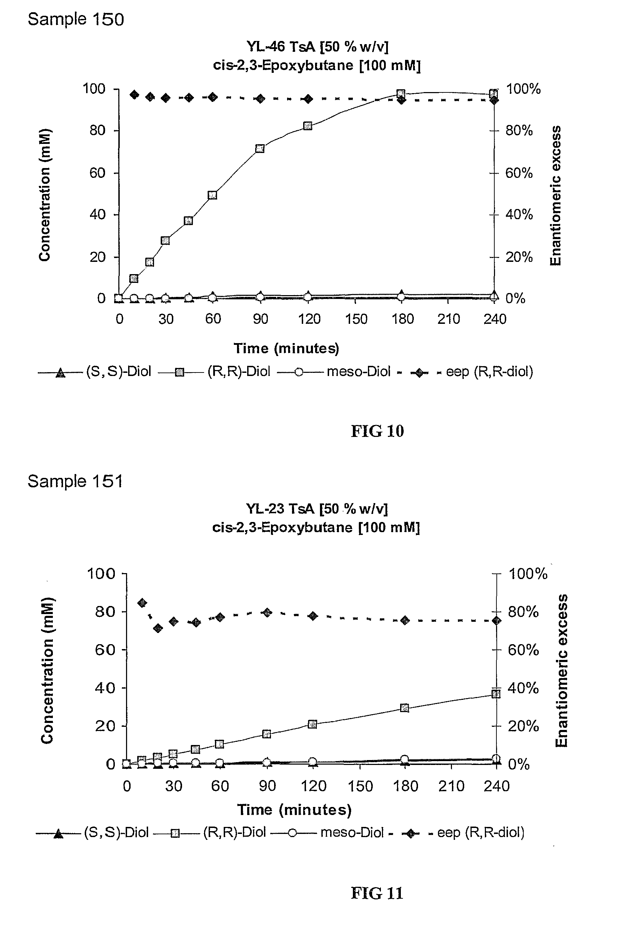

Figs. 9-12 (Samples 149-152) are line graphs showing the desymmetrisation of cis-2,3- epoxybutane by yeast host strains transformed with vectors expressing YEMH polypeptides of selected wild-type yeast strains. The left y-axis (and lines with shaded circle, square, and triangle data points) in each graph show the changes in concentrations of the *meso*-vicinal diol and optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.
Figs. 13-15 (Samples 153-155) are line graphs showing the desymmetrisation of cyclohexene oxide by the indicated wild-type yeast strains. The left y-axis (and lines with shaded square, circle, and triangle data points) in each graph show the changes in concentrations of the cyclohexene oxide and the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.

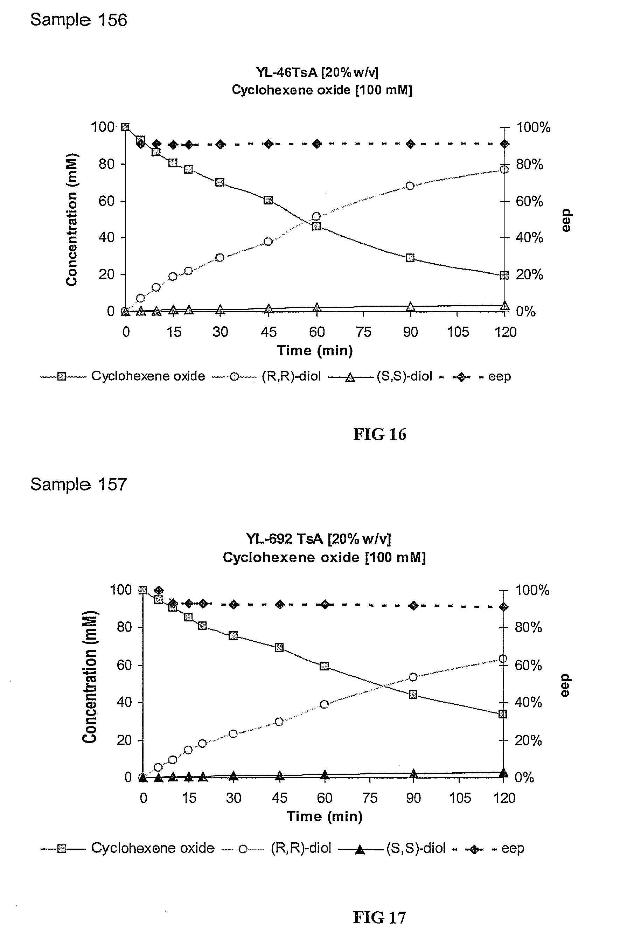

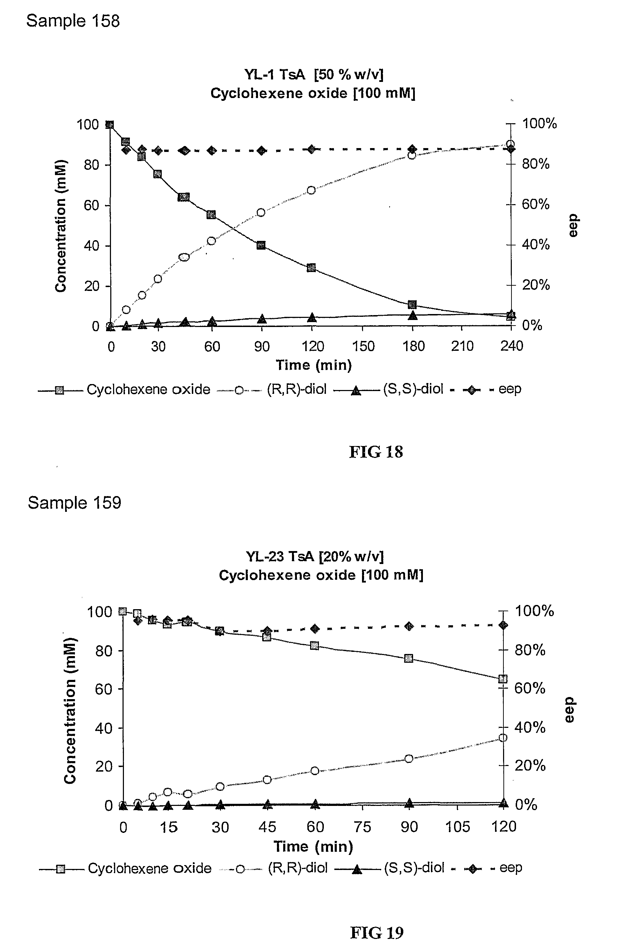

Figs. 16-20 (Samples 156-160) are line graphs showing the desymmetrisation of cyclohexene oxide by yeast host strains transformed with vectors expressing YEMH polypeptides of selected wild-type yeast strains. The left y-axis (and lines with shaded square, circle, and triangle data points) in each graph show the changes in concentrations of the cyclohexene oxide and the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.

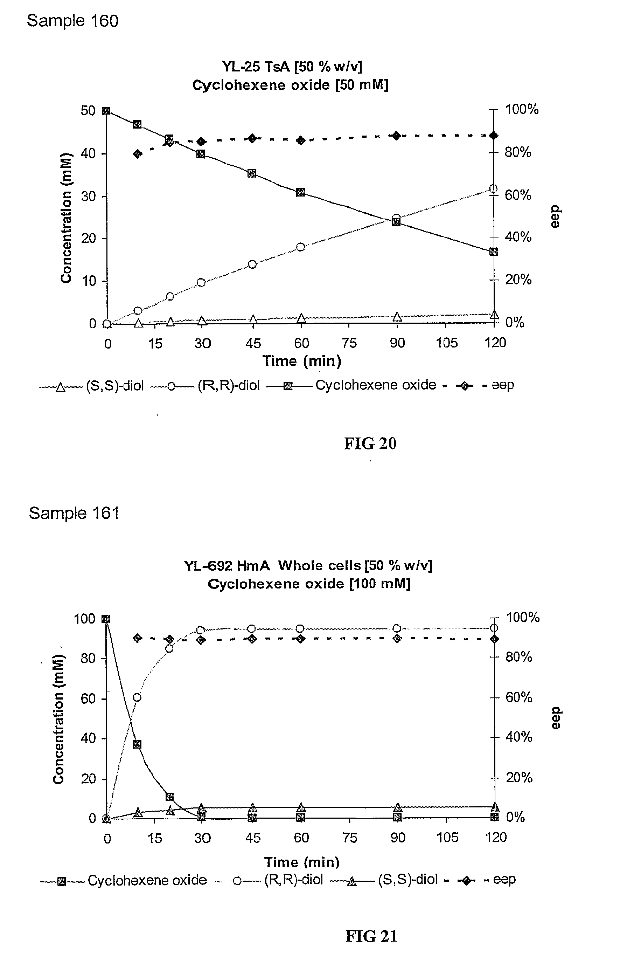

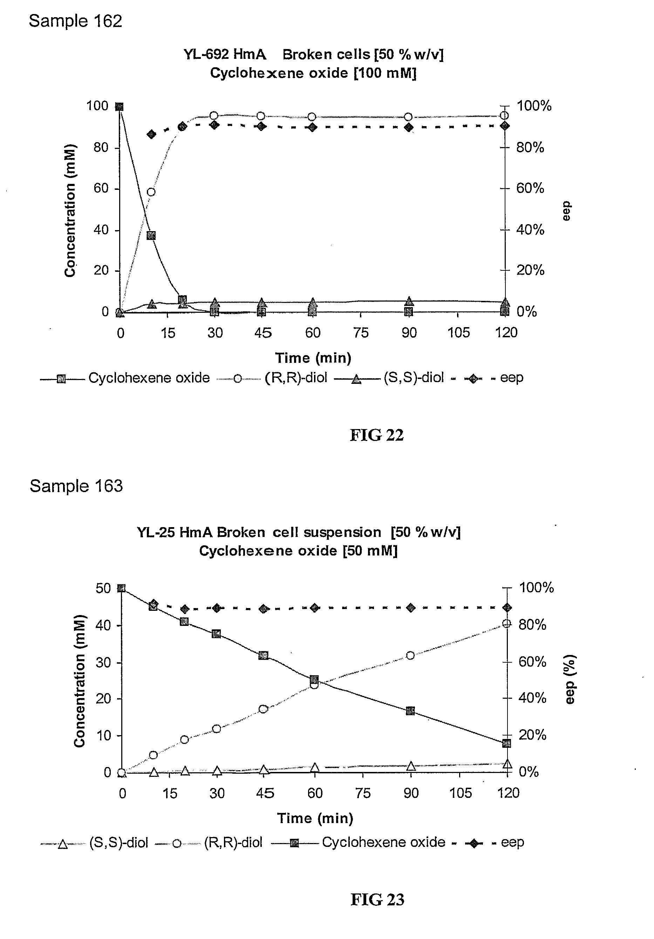

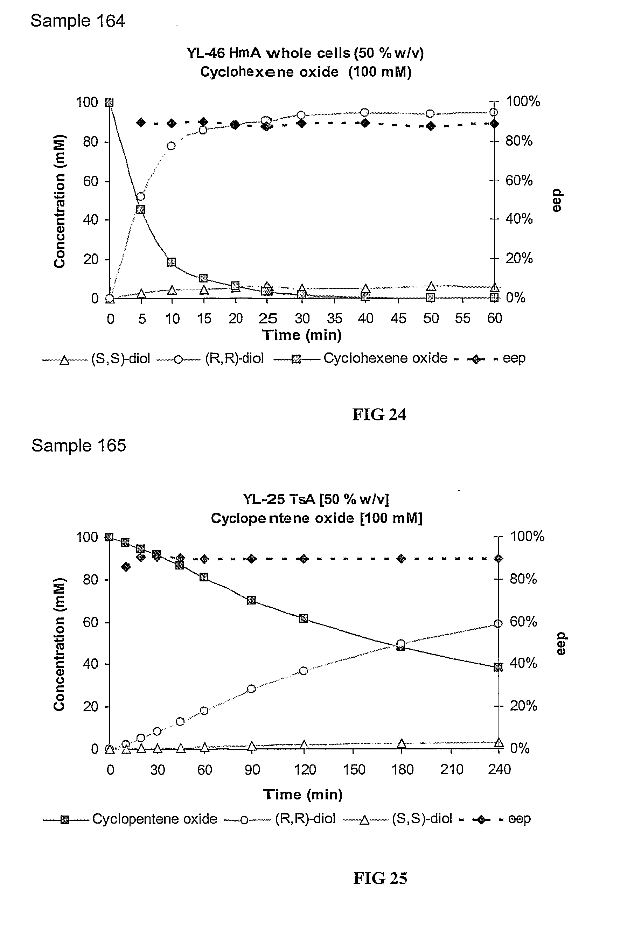

Figs. 21-24 (Samples 161-164) are line graphs showing the desymmetrisation of cyclohexene oxide by whole (Figs 21 and 24) and lysed/broken (Figs. 22 and 23) yeast host cells transformed with vectors expressing three different YEMH polypeptides of selected wild-type yeast strains. The left y-axis (and lines with shaded square, circle, and triangle data points) in each graph show the changes in concentrations of the cyclohexene oxide and the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.

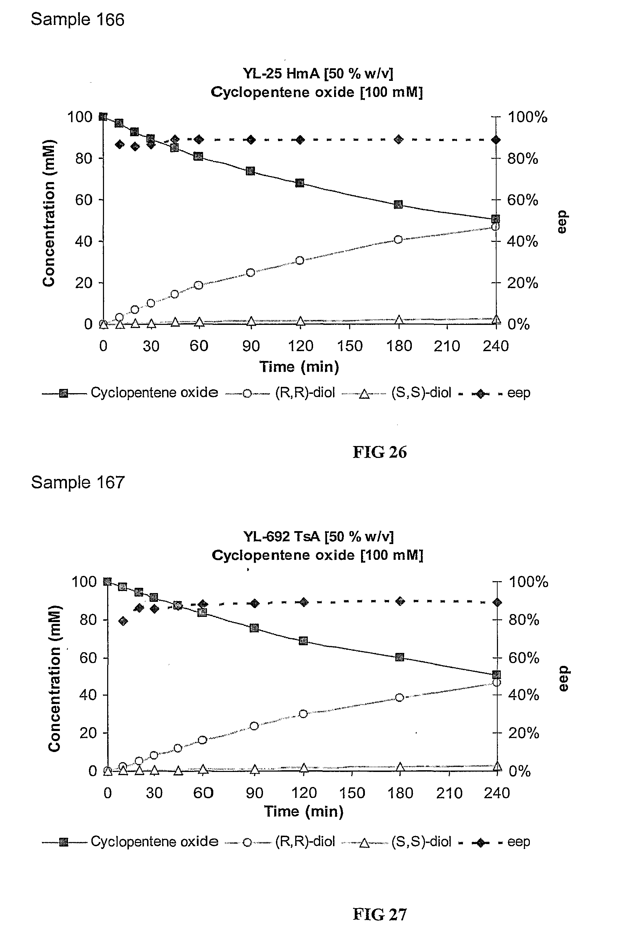

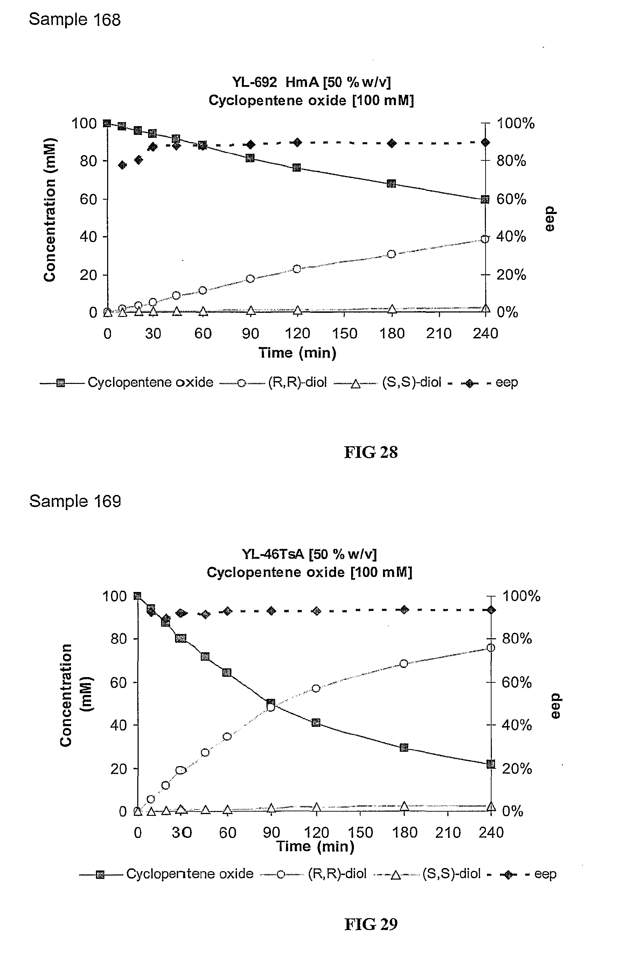

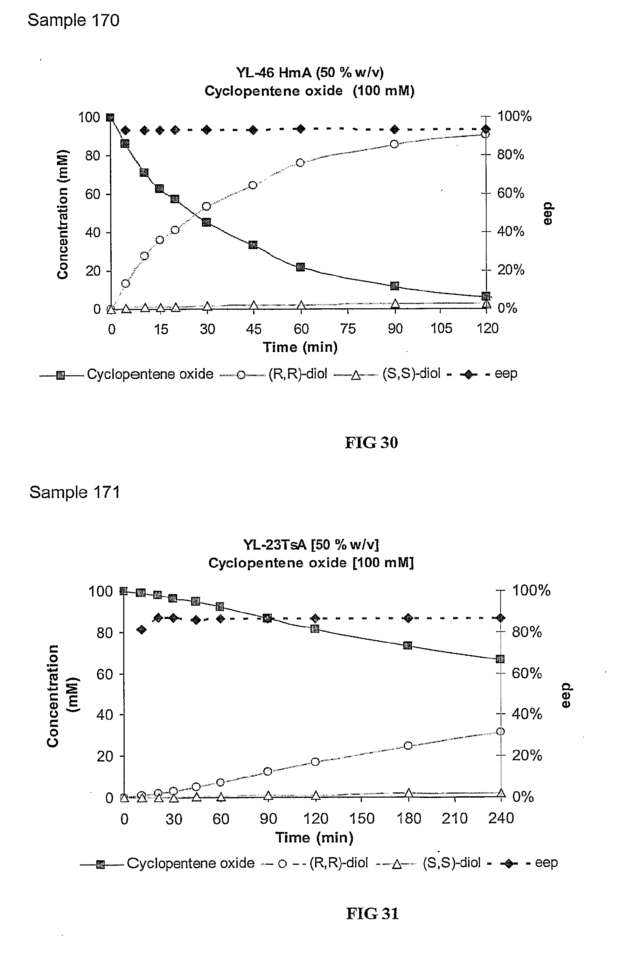

Figs. 25-31 (Samples 165-171) are line graphs showing the desymmetrisation of cyclopentene oxide by yeast host strains transformed with vectors expressing YEMH polypeptides of selected wild-type yeast strains. The left y-axis (and lines with shaded square, circle, and triangle data points) in each graph show the changes in concentrations of the cyclopentene oxide and the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.

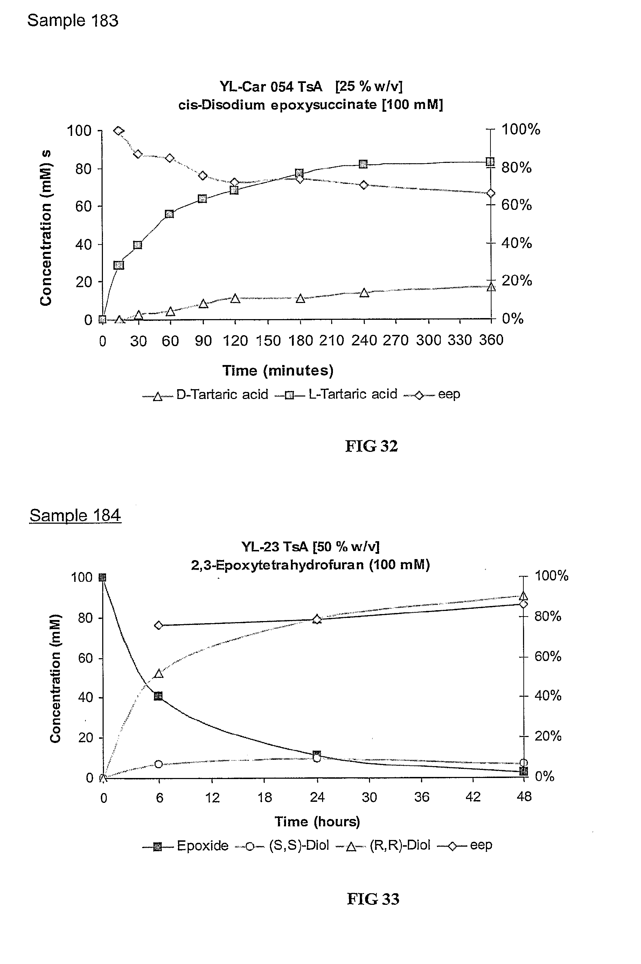

Figs. 32 (Samples 183) is a line graph showing the desymmetrisation of cis-epoxysuccinic acid disodium salt by a yeast host strain transformed with a vector expressing YEMH polypeptides of a selected wild-type yeast strains. The left y-axis (and lines with shaded square, and triangle data points) in each graph show the changes in concentrations of the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.
Figs. 33 (Samples 184) is a line graph showing the desymmetrisation of 3,4-epoxytetrahydrofuran by a yeast host strain transformed with a vector expressing YEMH polypeptides of a selected wild-type yeast strains. The left y-axis (and lines with shaded square, circle, and triangle data points) in each graph show the changes in concentrations of the cyclopentene oxide and the optically active vicinal diol products at the time points indicated on the x-axis and the right y-axis (and lines with shaded diamond-shaped data points) in each graph show the enantiomeric excesses ("ee") of the formed optically active vicinal diol at the time points indicated on the x-axis.

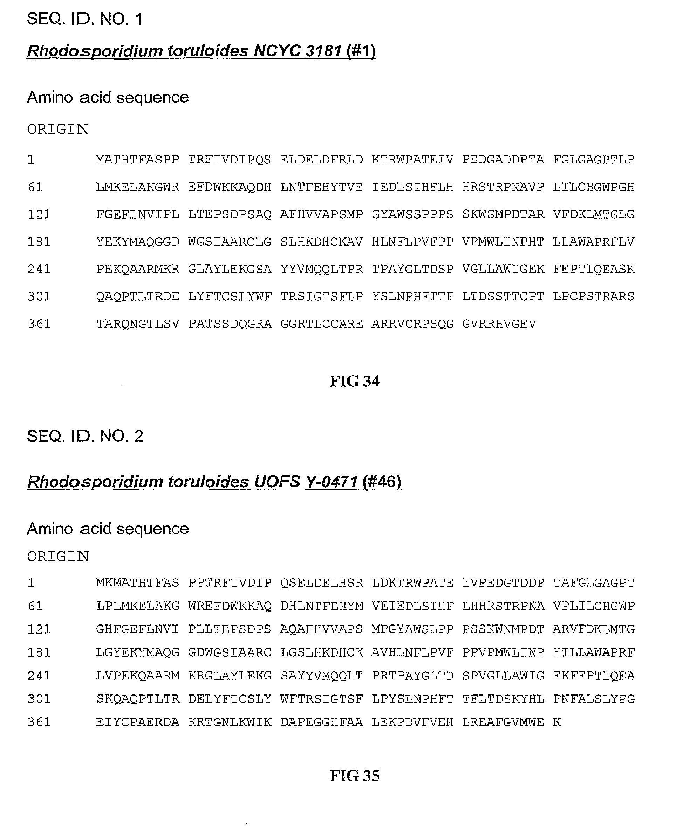

Fig. 34 is a depiction of the amino acid sequence (SEQ ID NO:1) of a YEMH polypeptide encoded by cDNA derived from a *Rhodosporidium toruloides* strain (assigned accession no. NCYC 3181).
Fig. 35 is a depiction of the amino acid sequence (SEQ ID NO:2) of a YEMH polypeptide encoded by cDNA derived from a *Rhodosporidium toruloides* strain (assigned identification no. UOFS Y-0471).

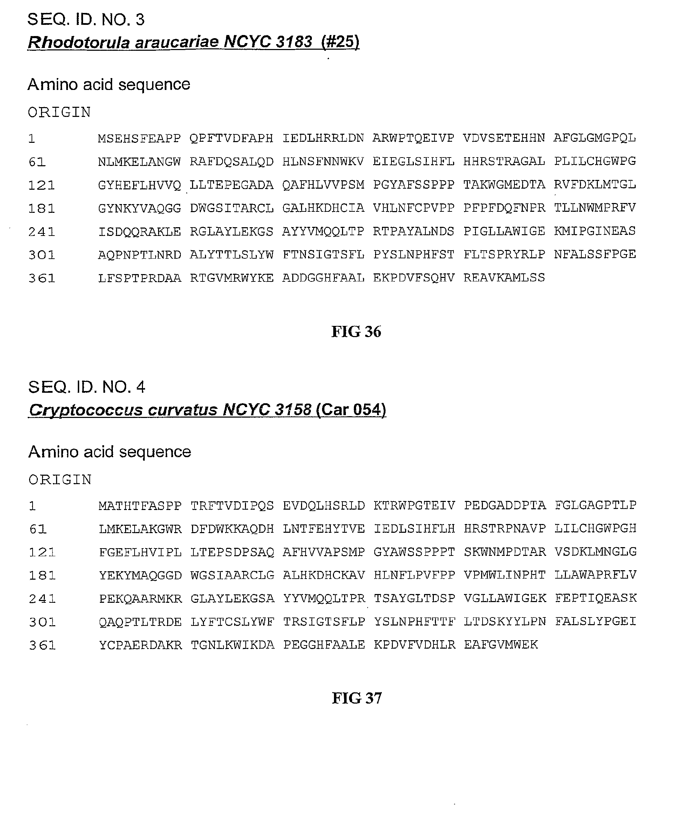

Fig. 36 is a depiction of the amino acid sequence (SEQ ID NO:3) of a YEMH polypeptide encoded by cDNA derived from a *Rhodotorula araucariae* strain (assigned accession no. NCYC 3183).
Fig. 37 is a depiction of the amino acid sequence (SEQ ID NO:4) of a YEMH polypeptide encoded by cDNA derived from a *Cryptococcus curvatus* strain (assigned accession no. NCYC 3158).

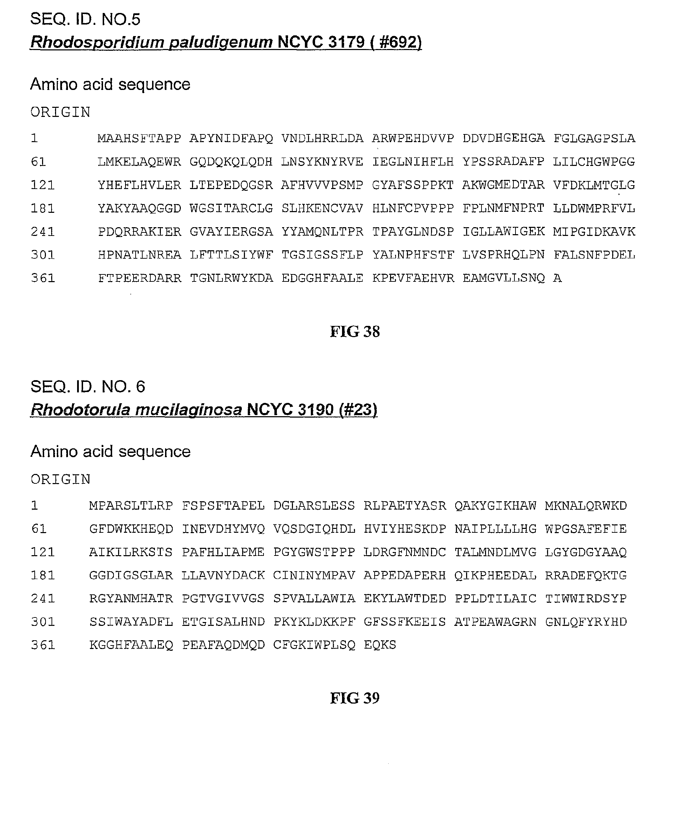

Fig. 38 is a depiction of the amino acid sequence (SEQ ID NO:5) of a YEMH polypeptide encoded by cDNA derived from a *Rhodosporidium paludigenum* strain (assigned accession no. NCYC 3179).
Fig. 39 is a depiction of the amino acid sequence (SEQ ID NO:6) of a YEMH polypeptide encoded by a cDNA derived from a *Rhodotorula mucilaginosa* strain (assigned accession no. NCYC 3190).

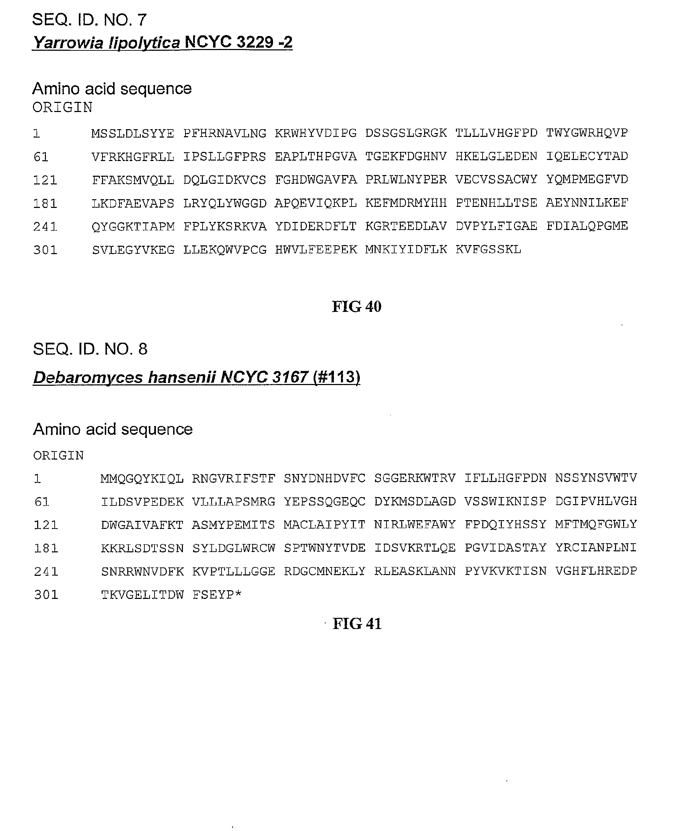

Fig. 40 is a depiction of the amino acid sequence (SEQ ID NO:7) of a YEMH polypeptide encoded by cDNA derived from a *Yarrowia lipolytica* strain (assigned accession no. NCYC 3229-2 (Polh-2)).
Fig. 41 is a depiction of the amino acid sequence (SEQ ID NO:8) of a YEMH polypeptide encoded by cDNA derived from a *Debaromyces hansenii_*strain (assigned accession no. NCYC 3167).

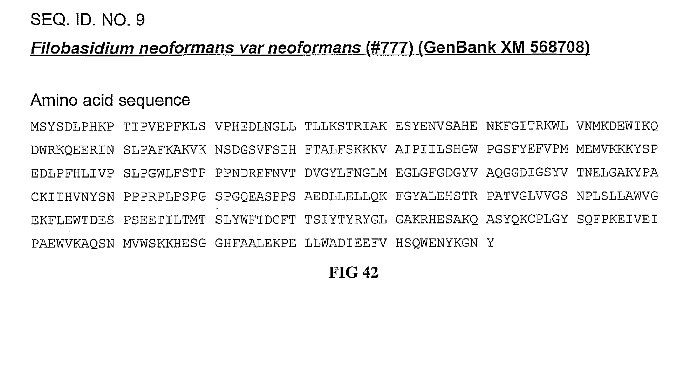

Fig. 42 is a depiction of the amino acid sequence (SEQ ID NO:9) of a YEMH polypeptide encoded by cDNA derived from a *Filobasidium neoformans var neoformans* strain (assigned GenBank accession no. NM_568708 (#777)).

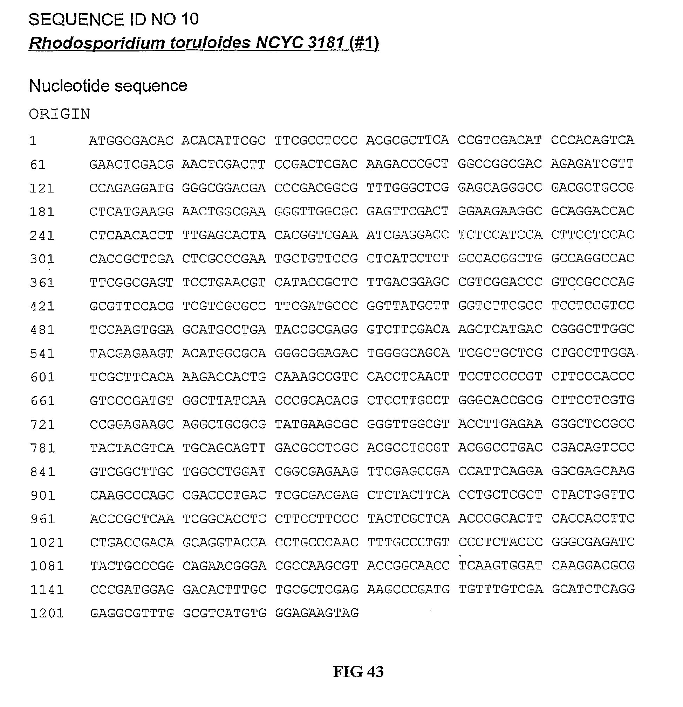

Fig. 43 is a depiction of the nucleotide sequence (SEQ ID NO: 10) of a YEMH polypeptide-encoding cDNA derived from a *Rhodosporidium toruloides* strain (assigned accession no. NCYC 3181).

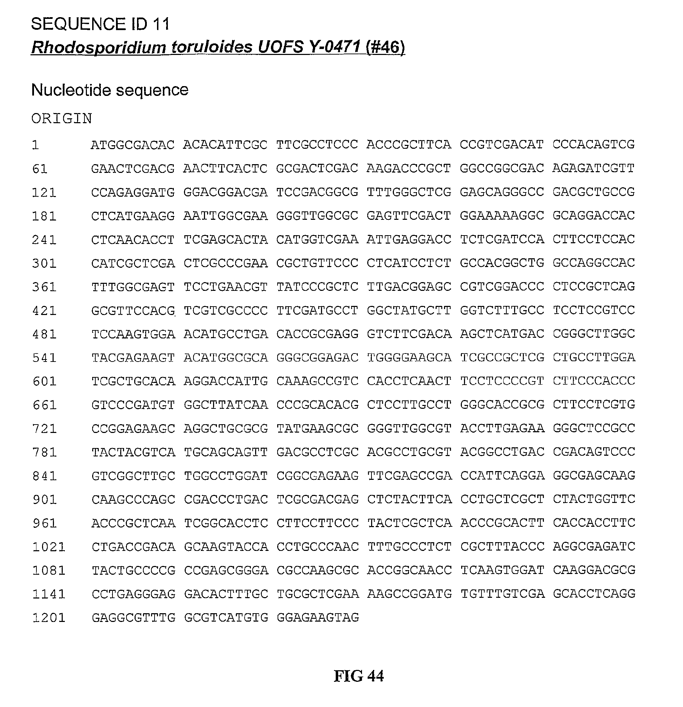

Fig. 44 is a depiction of the nucleotide sequence (SEQ ID NO: 11) of a YEMH polypeptide-encoding cDNA derived from a *Rhodosporidium toruloides* strain (assigned identification no. UOFS Y-0471).

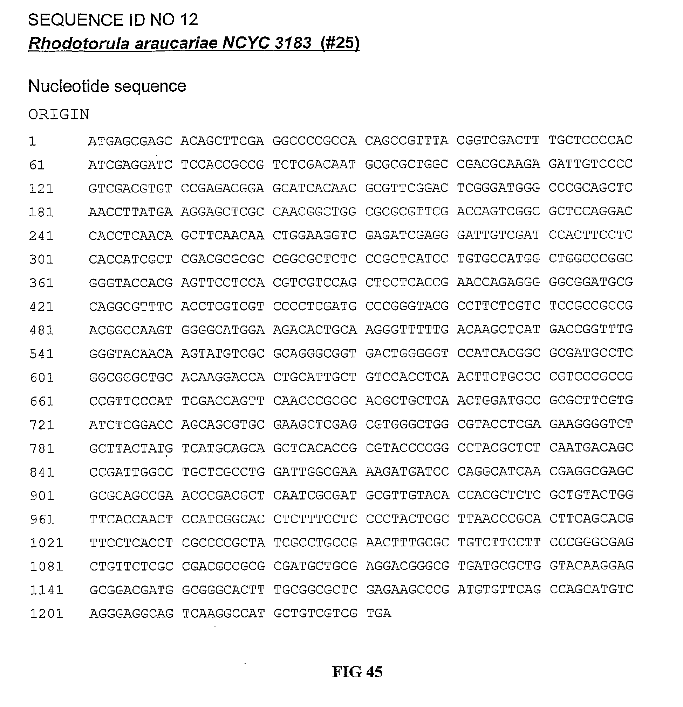

Fig. 45 is a depiction of the nucleotide sequence (SEQ ID NO: 12) of a YEMH polypeptide-encoding cDNA derived from a *Rhodotorula araucariae* strain (assigned accession no. NCYC 3183).

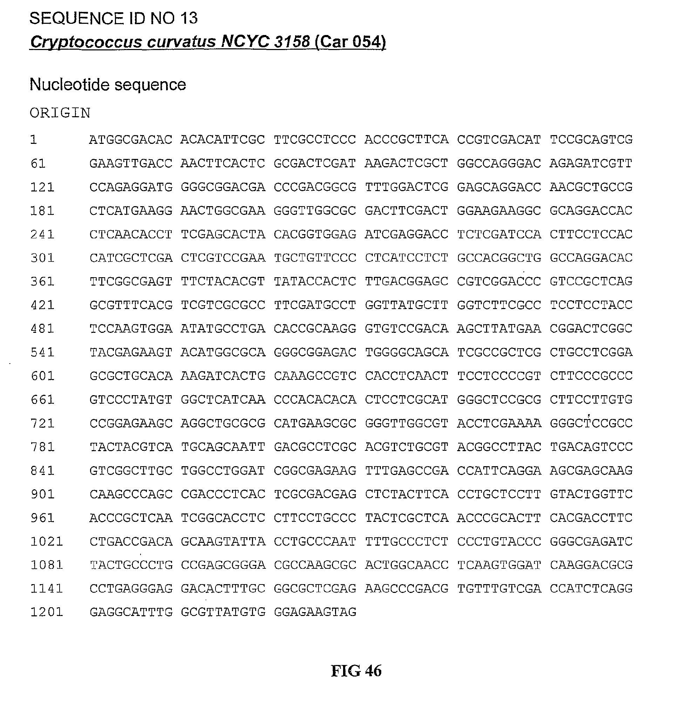

Fig. 46 is a depiction of the nucleotide sequence (SEQ ID NO: 13) of a YEMH polypeptide-encoding by cDNA derived from a. *Cryptococcus curvatus* strain (assigned accession no. NCYC 3158).

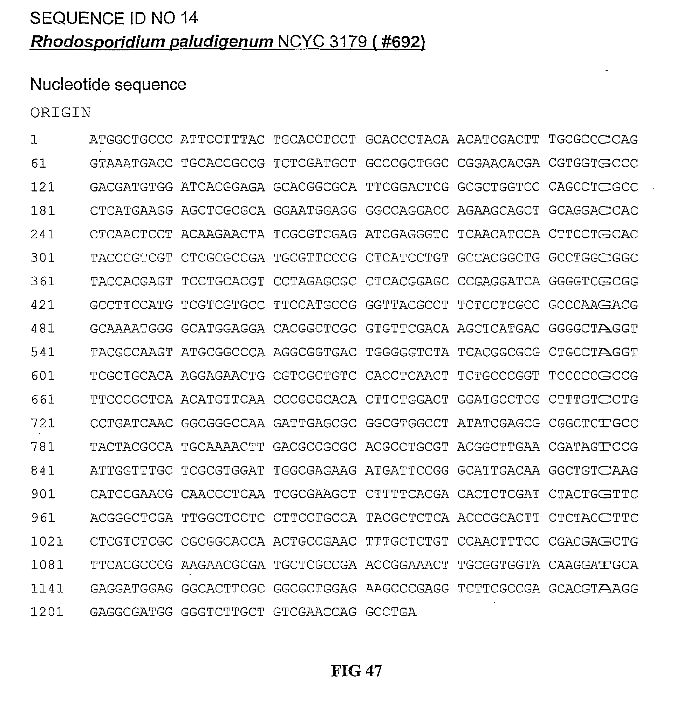

Fig. 47 is a depiction of the nucleotide sequence (SEQ ID NO: 14) of a YEMH polypeptide-encoding cDNA derived from a *Rhodosporidium paludigenum* strain (assigned accession no. NCYC 3179).

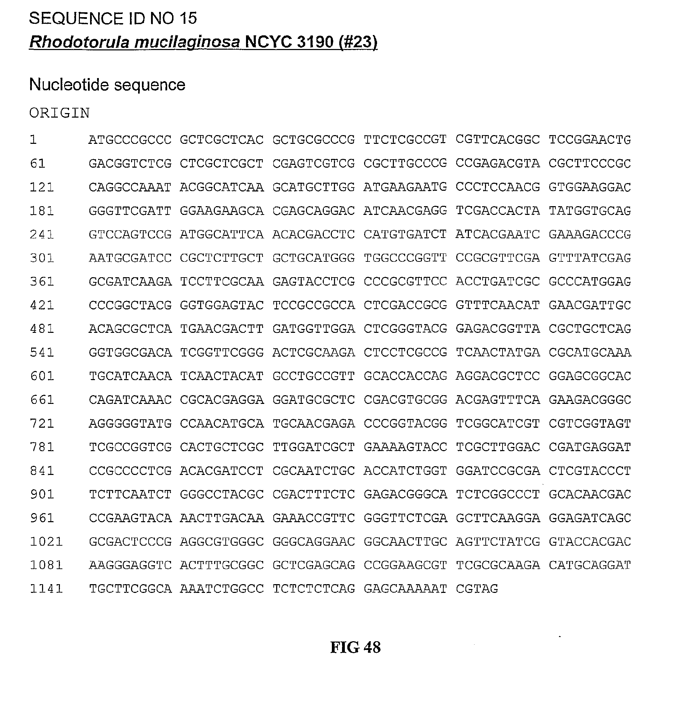

Fig. 48 is a depiction of the nucleotide sequence (SEQ ID NO: 15) of a YEMH polypeptide-encoding cDNA derived from a *Rhodotorula mucilaginosa* strain (assigned accession no. NCYC 3190).

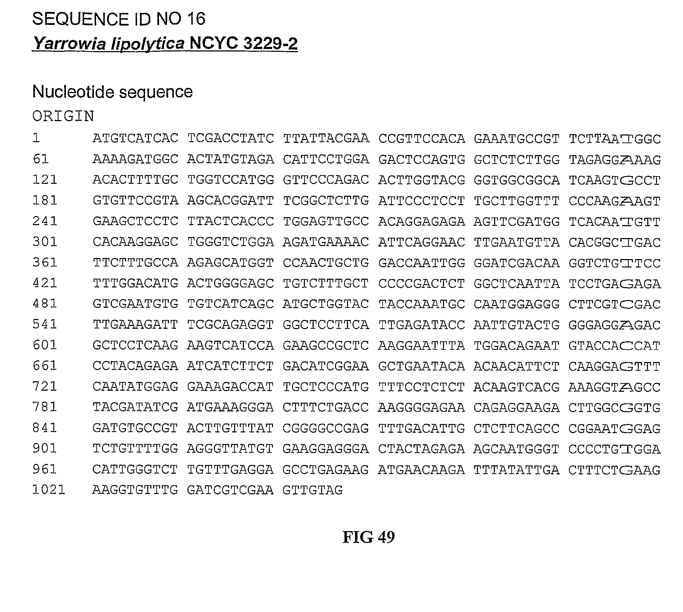

Fig. 49 is a depiction of the nucleotide sequence (SEQ ID NO:16) of a YEMH polypeptide-encoding cDNA derived from a *Yarrowia lipolytica* strain (assigned accession no. NCYC 3229-2 (Polh-2)).

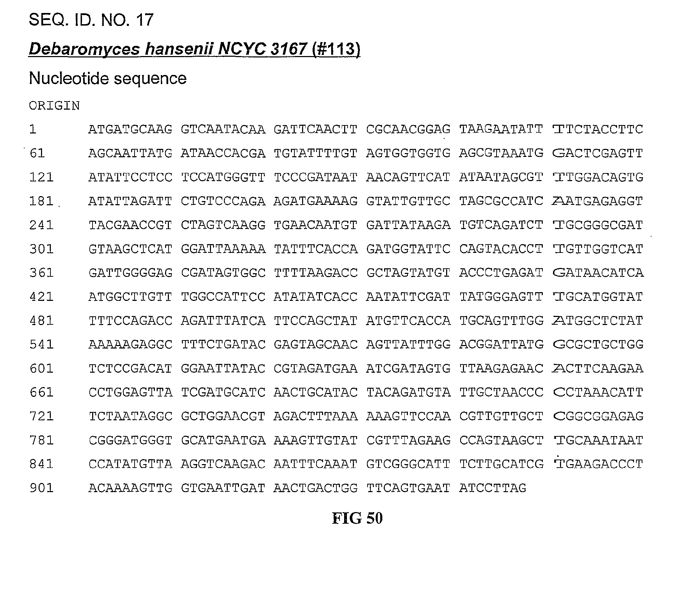

Fig. 50 is a depiction of the nucleotide sequence (SEQ ID NO: 17) of a YEMH polypeptide-encoding cDNA derived from a *Debaromyces hansenii* strain (assigned accession no. NCYC 3167).

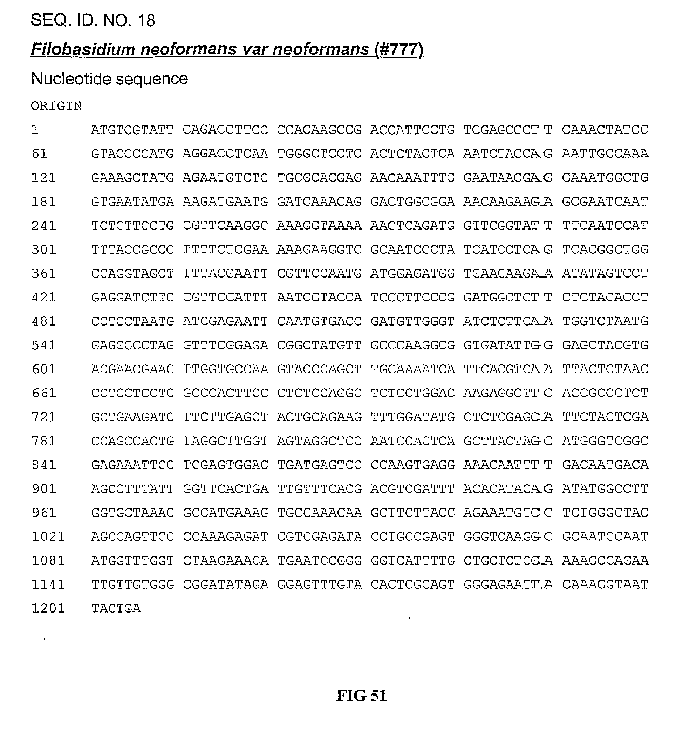

Fig. 51 is a depiction of the nucleotide sequence (SEQ ID NO: 18) of a YEMH polypeptide-encoding cDNA derived from a *Filobasidium neoformans var neoformans* strain (assigned GenBank accession no. XM_568708 (#777)).

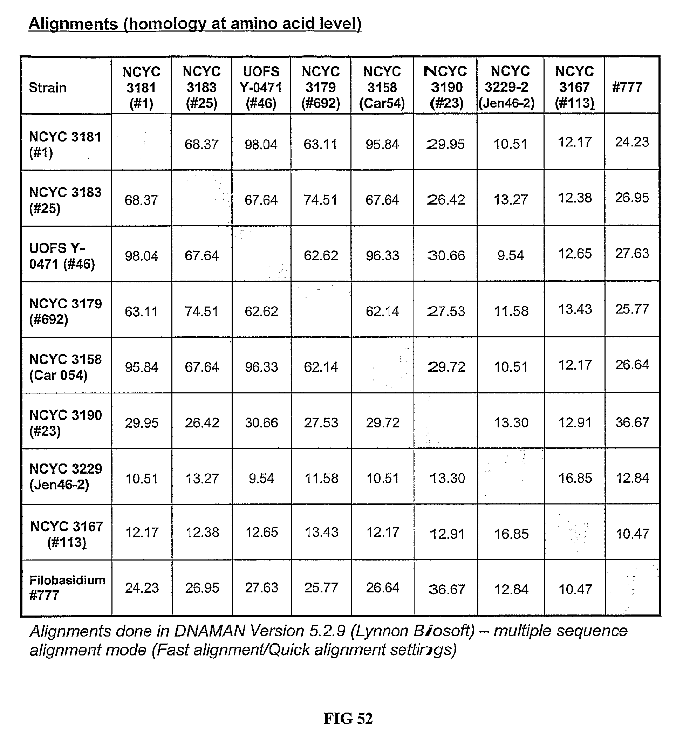

Fig. 52 is a table showing the homology at the amino acid level of the YESH polypeptides with SEQ ID NOs: 1-9.

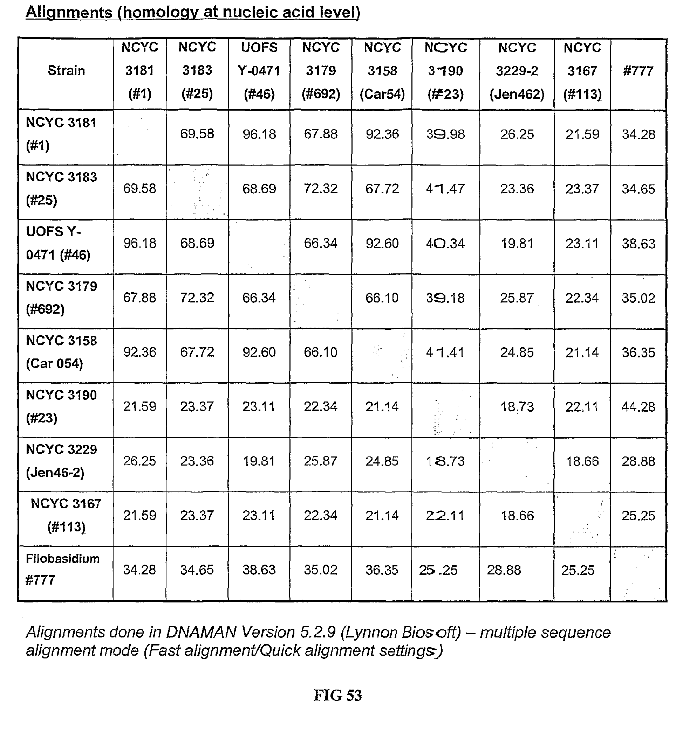

Fig. 53 is a table showing the homology at the nucleotide level of YEMH-encoding cDNA molecules with SEQ ID NOs: 10-18.
Fig. 54 is a depiction of the amino acid sequences of eight enantioselective epoxide hydrolases aligned for maximum homology. Also shown are consensus amino acids.
The sequences labelled #1, #46, #25, Car054, #692, #23, and Jen 46-2 and #777 correspond to SEQ ID NOs: 1, 2, 3, 4, 5, 6, 7, and 9. The consensus catalytic triad is composed of a nucleophile, an acid and a base, the positions of which are indicated by N, A and B, respectively. "HGXP" represents the region of the oxy-anion hole of the enzymes. "sxNxss" represents the genetic motif found in αβ/-hydrolase fold enzymes. (P = homology; P = identity of 75-100%; P = identity of 50-75%;. = gap).

### DETAILED DESCRIPTION

Various aspects of the invention are described below.

### Nucleic Acid Molecules

The YEMH nucleic acid molecules of the invention can be cDNA, genomic DNA, synthetic DNA, or RNA, and can be double-stranded or single-stranded (i.e., either a sense or an antisense strand). Segments of these molecules are also considered within the scope of the invention, and can be produced by, for example, the polymerase chain reaction (PCR) or generated by treatment with one or more restriction endonucleases, A ribonucleic acid (RNA) molecule can be produced by *in vitro* transcription. Preferably, the nucleic acid molecules encode polypeptides that, regardless of length, are soluble under normal physiological conditions.

The nucleic acid molecules of the invention can contain naturally occurring sequences, or sequences that differ from those that occur naturally, but, due to the degeneracy of the genetic code, encode the same polypeptide.

In addition, these nucleic acid molecules are not limited to coding sequences, e.g., they can include some or all of the non-coding sequences that lie upstream or downstream from a coding sequence.

The nucleic acid molecules of the invention can be synthesized (for example, by phosphoramidite-based synthesis) or obtained from a biological cell, such as the cell of a eukaryote (e.g., a mammal such as human or a mouse or a yeast such as any of the genera, species, and strains of yeast disclosed herein) or a prokarote (e.g., a bacterium such as *Escherichia coli*). The nucleic acids can be those of a yeast such as any of the genera, species, and strains of yeast disclosed herein. Combinations or modifications of the nucleotides within these types of nucleic acids are also encompassed.

In addition, the isolated nucleic acid molecules of the invention encompass segments that are not found as such in the natural state. Thus, taught herein are nucleic acid molecules (for example, isolated nucleic acid molecules encoding the polypeptides of SEQ ID NOs: 1- 9) incorporated into a vector (for example, a plasmid or viral vector) or into the genome of a heterologous cell (or the genome of a homologous cell, at a position other than the natural chromosomal location). Recombinant nucleic acid molecules and uses therefor are discussed further below.

Techniques associated with detection or regulation of genes are well known to skilled artisans. Such techniques can be used, for example, to test for expression of a YEMH gene in a test cell (e.g., a yeast cell) of interest.

A YEMH family gene or protein can be identified based on its similarity to the relevant YEMH gene or protein, respectively. For example, the identification can be based on sequence identity. Taught herein are isolated nucleic acid molecules which are, or are at least 50% (e.g., at least: 55%; 60%; 65%; 75%; 85%; 95%; 99%; or 99%) identical to: (a) a nucleic acid molecule that encodes the polypeptide of SEQ ID NOs: 1-9; (b) the nucleotide sequence of SEQ ID NOs: 10-18; (c) a nucleic acid molecule which includes a segment of at least 15 (e.g., at least: 20; 25; 30; 35; 40; 50; 50; 80; 100; 125; 150; 175; 200; 250; 300; 350; 400; 500; 600; 700; 800; 900; 1,000; 1,030; 1,035; 1,040; 1,042; 1,043; 1,050; 1,055; 1,058; 1,060; 1,061; 1,100; 1,150; 1,160; 1,170; 1,175; 1,178; 1,180; 1,181; 1,190; 1,190; 1,195; 1,198; 1,200; 1,201; 1,220; 1,224; 1,225; 1,226; 1,227; 1,228 1,230; 1,231; or 1,232) nucleotides of SEQ ID NOs; 10-18; (d) a nucleic acid molecule encoding any of the polypeptides or fragments thereof disclosed below; and (e) the complement of any of the above nucleic acid molecules. The complements of the above molecules can be full-length complements or segment complements containing a segment of at least 15 (e.g., at least: 20; 25; 30; 35; 40; 50; 60; 80; 100; 125; 150; 175; 200; 250; 300; 350; 400; 500; 600; 700; 800; 900; 1,000; 1,100; 1,200; 1,220; 1,225; 1,228; 1,230; 1,231; or 1,232) consecutive nucleotides complementary to any of the above nucleic acid molecules. Identity can be over the full-length of SEQ ID NOs: 10-18, or over one or more contiguous or non-contiguous segments.

The determination of percent identity between two sequences is accomplished using the mathematical algorithm of Karlin and Altschul, Proc. Natl. Acad. Sci. USA 90, 5873-5877, 1993. Such an algorithm is incorporated into the BLASTN AND BLASTP programs of Altschul et al. (1990) J. Mol. Biol. 215, 403-410. BLAST nucleotide searches are performed with the BLASTN program, score = 100, wordlength = 12, to obtain nucleotide sequences homologous to YEMH-encoding nucleic acids. BLAST protein searches are performed with the BLASTP program, score = 50, wordlength = 3, to obtain amino acid sequences homologous to the YEMH polypeptide. To obtain gapped alignments for comparative purposes, Gapped BLAST is utilized as described in Altschul et al. (1997) Nucleic Acids Res. 25, 3389-3402. When utilizing BLAST and Gapped BLAST programs, the default parameters of the respective programs (e.g., XBLAST and NBLAST) are used.

Hybridization can also be used as a measure of homology between two nucleic acid sequences. A YEMH-encoding nucleic acid sequence, or a portion thereof, can be used as a hybridization probe according to standard hybridization techniques. The hybridization of a YEMH probe to DNA or RNA from a test source (e.g., a mammalian cell) is an indication of the presence of YEMH DNA or RNA in the test source. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1% SDS at 50°C. Highly stringent conditions are defined as equivalent to hybridization in 6X sodium chloride/sodium citrate (SSC) at 45°C, followed by a wash in 0.2 X SSC, 0.1% SDS at 65°C.

Also disclosed herein are (a) vectors (see below) that contain any of the foregoing YEMH coding sequences (including coding sequence segments) and/or their complements (that is, "antisense" sequences); (b) expression vectors that contain any of the foregoing YEMH coding sequences (including coding sequence segments) operably linked to one or more transcriptional and/or translational regulatory elements (TRE; examples of which are given below) necessary to direct expression of the coding sequences; (c) expression vectors encoding, in addition to a YEMH polypeptide (or a fragment thereof), a sequence unrelated to YEMH, such as a reporter, a marker, or a signal peptide fused to YEMH; and (d) genetically engineered host cells (see below) that contain any of the foregoing expression vectors and thereby express the nucleic acid molecules of the invention.

Recombinant nucleic acid molecules can contain a sequence encoding a YEMH polypeptide or YEMH polypeptide having anheterologous signal sequence. The full length YEMH polypeptide, or a fragment thereof, can be fused to such heterologous signal sequences or to additional polypeptides, as described below. Similarly, the nucleic acid molecules can encode a YEMH polypeptide that includes an exogenous polypeptide that facilitates secretion.

The TRE referred to above and further described below include but are not limited to inducible and non-inducible promoters, enhancers, operators and other elements that are known to those skilled in the art and that drive or otherwise regulate gene expression. Such regulatory elements include but are not limited to the cytomegalovirus hCMV immediate early gene, the early or late promoters of SV40 adenovirus, the *lac* system, the *trp* system, the *TAC* system, the *TRC* system, the major operator and promoter regions of phage A, the control regions of fd coat protein, the promoter for 3-phosphoglycerate kinase, the promoters of acid phosphatase, and the promoters of the yeast a-mating factors. Other useful TRE are listed in the examples below.

Similarly, the nucleic acid can form part of a hybrid gene encoding additional polypeptide sequences, for example, a sequence that functions as a marker or reporter. Examples of marker and reporter genes include β-lactamase, chloramphenicol acetyltransferase (CAT), adenosine deaminase (ADA), aminoglycoside phosphotransferase (neo^{r}, G418^{r}), dihydrofolate reductase (DHFR), hygromycin-B-phosphotransferase (HPH), thymidine kinase (TK), lacZ (encoding β-galactosidase), xanthine guanine phosphoribosyltransferase (XGPRT), and green, blue, or yellow fluorescent protein. As with many of the standard procedures associated with the practice of the invention, skilled artisans will be aware of additional useful reagents, for example, additional sequences that can serve the function of a marker or reporter. Generally, the hybrid polypeptide will include a first portion and a second portion; the first portion being a YEMH polypeptide (or any of YEMH fragments described below) and the second portion being, for example, the reporter described above or an Ig heavy chain constant region or part of an Ig heavy chain constant region, e.g., the CH2 and CH3 domains of IgG2a heavy chain. Other hybrids could include an antigenic tag or a poly-His tag to facilitate purification.

Expression systems are taught herein that include, but are not limited to, microorganisms such as yeasts (e.g, any of the genera, species or strains listed herein) or bacteria (for example, *E. coli* and *B. subtilis*) transformed with recombinant bacteriophage DNA, plasmid DNA, or cosmid DNA expression vectors containing the nucleic acid molecules of the invention; yeast (for example, *Saccharomycesi, Yarrowia*, *Pichia*, and other genera, species, and strains listed herein) transformed with recombinant yeast expression vectors containing the nucleic acid molecule of the invention; insect cell systems infected with recombinant, virus expression vectors (for example, baculovirus) containing the nucleic acid molecule of the invention; plant cell systems infected with recombinant virus expression vectors (for example, cauliflower mosaic virus (CaMV) or tobacco mosaic virus (TMV)) or transformer with recombinant plasmid expression vectors (for example, Ti plasmid) containing a YEMH nucleotide sequence; or mammalian cell systems (for example, COS, CHO, BHK, 293, VERO, HeLa, MDCK, WI38, and NIH 3T3 cells) harboring recombinant expression constructs containing promoters derived from the genome of mammalian cells (for example, the metallothionein promoter) or from mammalian viruses (for example, the adenovirus late promoter and the vaccinia virus 7.5K promoter). Also useful as host cells are primary or secondary cells obtained directly from a mammal and transfected with a plasmid vector or infected with a viral vector.

Taught herein are wild-type and recombinant cells including, but not limited to, yeast cells (e.g., any of those disclosed herein) containing any of the above YEMH genes, nucleic acid molecules, and genetic constructs. The cells are preferably isolated cells. As used herein, the term "isolated" as applied to a microorganism (e.g., a yeast cell) refers to a microorganism which either has no naturally-occurring counterpart (e.g., a recombinant microorganism such as a recombinant yeast) or has been extracted and/or purified from an environment in which it naturally occurs. Thus, an "isolated microorganism" does not include one residing in an environment in which it naturally occurs, for example, in the air, outer space, the ground, oceans, lakes, rivers, and streams and the like, ground at the bottom of oceans, lakes, rivers, and streams and the like, snow, ice on top of the ground or in/on oceans lakes, rivers, and streams and the like, man-made structures (e.g., buildings), or in natural hosts (e.g., plant, animal or microbial hosts) of the microorganism, unless the microorganism (or a progenitor of the microorganism) was previously extracted and/or purified from an environment in which it naturally occurs and subsequently returned to such an environment or any other environment in which it can survive. An example of an isolated microorganism is one in a substantially pure culture of the microorganism.

There is also disclosed a substantially pure culture of a microorganism (e.g., a microbial cell such as a yeast cell). As used herein, a "substantially pure culture" of a microorganism is a culture of that microorganism in which less than about 40% (i.e., less than about: 35%; 30%; 25%; 20%; 15%; 10%; 5%; 2%; 1%; 0.5%; 0.25%; 0.1%; 0.01%; 0.001%; 0.0001%; or even less) of the total number of viable microbial (e.g., bacterial, fungal (including yeast), mycoplasmal, or protozoan) cells in the culture are viable microbial cells other than the specified microorganism. The term "about" in this context means that the relevant percentage can be 15% percent of the specified percentage above or below the specified percentage. Thus, for example, about 20% can be 17% to 23%. Such a culture of microorganisms includes the microorganisms and a growth, storage, or transport medium. Media can be liquid, semi-solid (e.g., gelatinous media), or frozen. The culture includes the cells growimg in the liquid or in/on the semi-solid medium or being stored or transported in a storage or transport medium, including a frozen storage or transport medium. The cultures are in a culture vessel or storage vessel or substrate (e.g., a culture dish, flask, or tube or a storage vial or tube).

The microbial cells can be stored, for example, as frozen cell suspensions, e.g. in buffer containing a cryoprotectant such as glycerol or sucrose, as lyophilized cells. Alternatively, they can be stored, for example, as dried cell preparations obtained, e.g., by fluidised bed drying or spray drying, or any other suitable drying method. Similarly the enzyme preparations can be frozen, lyophilised, or immobilized and stored under appropriate conditions to retain activity.

### Polypeptides and Polypeptides Fragments

The YEMH polypeptides include all the YEMH and fragments of YEMH disclosed herein. They can be, for exampLe, the polypeptides with SEQ ID NOs:1-9 and functional fragments of these polypeptides. The polypeptides embraced by the invention also include fusion proteins that contain either full-length or a functional fragment of it fused to unrelated amino acid sequence. The unrelated sequences can be additional functional domains or signal peptides.

Disclosed herein are isolated polypeptides which are, or are at least 50% (e.g., at least: 55%; 60%; 65%; 75%; 85%; 95%; 98%; or 99%) identical to the polypeptides with amino acid SEQ ID NOs: 1-9. The identity can be over the full-length of the latter polypeptides or over one or more contiguous or non-contiguous segments.

Fragments of YEMH are segments of the full-length YEMH that are shorter than full-length YEMH. Fragments of YEMH can contain 5-410 (e.g., 10,20,30,40, 50, 60, 70, 80, 90, 100, 120, 150, 250, 300, 340, 342, 344, 346, 347, 349, 350, 351, 352, 353, 380, 390, 391, 392, 393, 393, 395, 396, 397, 400, 405, 406, 407, 408, or 409) amino acids of SEQ ID NOs:1-9. Fragments of YEMH can be functional fragments or antigenic fragments.

The polypeptides can be any of those described above but with not more 50 (e.g., not more than 50, 45, 40, 35, 30, 25, 20, 17,14, 12, 10, nine, eight, seven, six, five, four, three, two, or one) conservative substitution(s). Such substitutions can be made by, for example, site-directed mutagenesis or random mutagenesis of appropriate YEMH coding sequences.

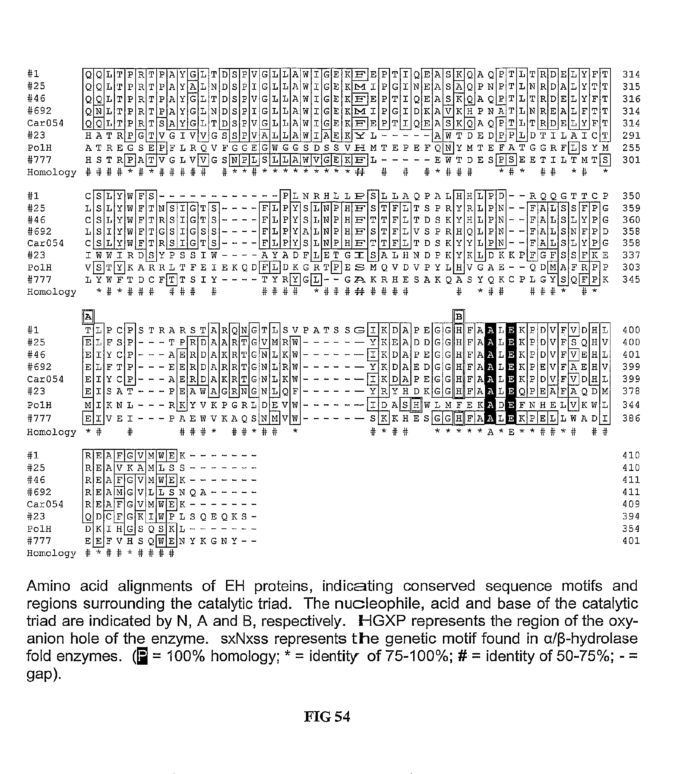

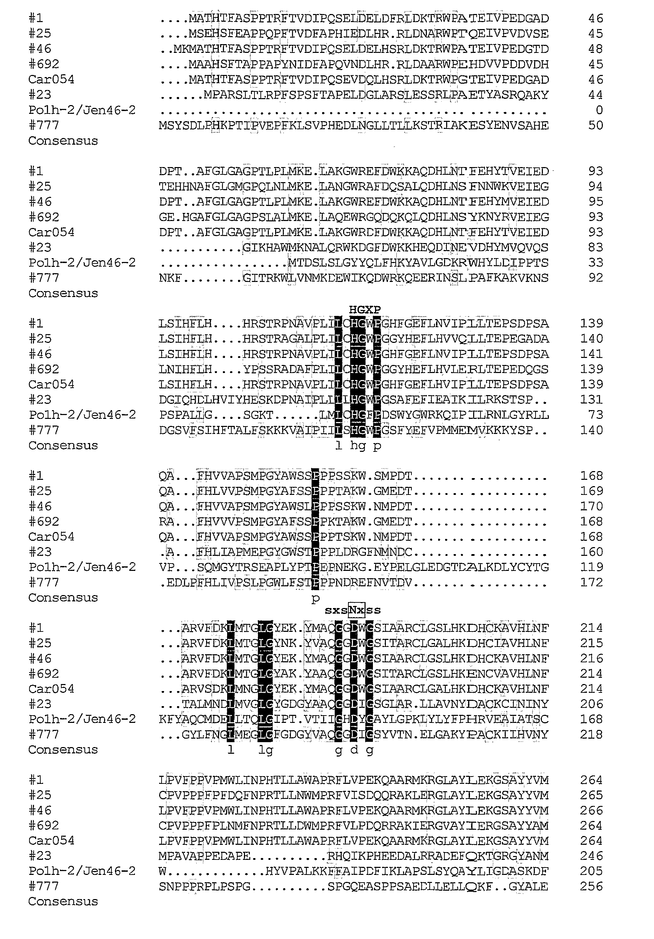

"Functional fragments" of a YEMH polypeptide (and, optionally, any of the above-described YEMH polypeptide variants) have at least 20% (e.g., 30%, 40%, 50%, 60%, 70%, 80%, 90%, 95%, 98%, 99%, 100%, or more) of the ability of the full-length, wild-type YEMH polypeptide to enantioselectively hydrolyse a MEO of interest. One of skill in the art will be able to predict YEMH functional fragments using his or her own knowledge and information provided herein, e.g., the amino acid alignments in Fig. 54 showing highly conserved domains and residues required for epoxide hydrolase activity.

Fragments of interest can be made by either recombinant, synthetic, or proteolytic digestive methods and tested for their ability to enantioselectively hydrolyse a MEO.

Antigenic fragments of the polypeptides of the invention are fragments that can bind to an antibody. Methods of testing whether a fragment of interest can bind to an antibody are known in the art.

The polypeptides can be purified from natural sources (e.g., wild-type or recombinant yeast cells such as any of those described herein). Smaller peptides (e.g., those less than about 100 amino acids in length) can also be conveniently synthesized by standard chemical means. In addition, both polypeptides and peptides can be produced by standard *in vitro* recombinant DNA techniques and *in vivo* transgenesis, using nucleotide sequences encoding the appropriate polypeptides or peptides. Methods well-known to those skilled in the art can be used to construct expression vectors containing relevant coding sequences and appropriate transcriptional/translational control signals. *See,* for example, the techniques described in Sambrook et al., Molecular Cloning: A Laboratory Manual (2nd Ed.) [Cold Spring Harbor Laboratory, N.Y., 1989], and Ausubel et al., Current Protocols in Molecular Biology [Green Publishing Associates and Wiley Interscience, N.Y., 1989].

Polypeptides and fragments of the invention also include those described above, but modified by the addition, at the amino- and/or carboxyl-terminal ends, of a blocking agent to facilitate survival of the relevant polypeptide. This can be useful in those situations in which the peptide termini tend to be degraded by proteases. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill.

Alternatively, blocking agents such as pyroglutamic acid or other molecules known in the art can be attached to the amino and/or carboxyl terminal residues, or the amino group at the amino terminus or carboxyl group at the carboxyl terminus can be replaced with a different moiety. Likewise, the peptides can b e covalently or noncovalently coupled to pharmaceutically acceptable "carrier" proteins prior to administration.

Also of interest are peptidomimetic compounds that are designed based upon the amino acid sequences of the functional peptide fragments. Peptidomimetic compounds are synthetic compounds having a three-dimensional confirmation (i.e., a "peptide motif") that is substantially the same as the three-dimensional conformation of a selected peptide. The peptide motif provides the peptidomimetic compound with the ability to enantioselectively hydrolyse a MEO of interest in a manner qualitatively identical to that of the YEMH functional fragment from which the peptidomimetic was derived. Peptidomimetic compounds can have additional characteristics that enhance their therapeutic utility, such as increased cell permeability and prolonged biological half-life.

The peptidomimetics typically have a backbone that is partially or completely non-peptide, but with side groups that are identical to the side groups of the amino acid residues that occur in the peptide on which the peptidomimetic is based. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics.

The invention also provides compositions and preparations containing one or more (e.g., two, three, four, five, six, seven, eight, nine, ten, 12, 15, 20, 25, or more) of the above-described polypeptides, polypeptide variants, and polypeptide fragments. The composition or preparation can be, for example a crude cell (e.g. a yeast cell) extract or culture supernatant, a crude enzyme preparation, a highly purified enzyme preparation. The compositions and preparations can also contain one or more of a variety of carriers or stabilizers known in the art. Carriers and stabilizers are known in the art and include, for example: buffers, such as phosphate, citrate, and other non-organic acids; antioxidants such as ascorbic acid; low molecular weight (less than 10 residues) polypeptides; proteins such as serum albumin, gelatin or immunoglobulins; hydrophilic polymers such polyvinylpyrrolidone; amino acids such as glycine, glutamine, asparagine, arginine, or lysine; monosaccharides, disaccharides, and other carbohydrates including glucose, mannose, or dextrans; chelating agents such as ethylenediaminetetraacetic acid (EDTA); sugar alcohols such as mannitol, or sorbitol; salt-forming counterions such as sodium; and/or nonionic surfactants such as Tween and Pluronics.

### Methods of Producing Optically Active Vicinal Diols

The invention provides methods for obtaining enantiopure, or substantially enantiopure, optically active vicinal diols. Enantiopure optically active vicinal diol preparations are preparations containing one enantiomer of the vicinal diol and none of the other enantiomer of the vicinal diol. "Substantially enantiopure" optically active vicinal diol preparations are preparations containing at least 55% (e.g., at least: 60%; 70%; 80%; 85%; 90%; 95%; 97%; 98%; 99%; 99.5%; 99.8%; or 99.9%), relative to the total amount of both vicinal diol enantiomers, of the particular enantiomer of the vicinal diol.

The method involves exposing a MEO to a YEMH polypeptide (e.g., isolated or in a microbial cell), which selectively catalyzes the conversion of the MEO to one optically active vicinal diol enantiomer (R,R or S,S). In this way the desired optically active vicinal diol is produced. YEMH polypeptides useful for the invention (i.e., those with enantioselective activity) will catalyze the conversion of a MEO to one optically active vicinal diol enantiomer with less than 80% (e.g., less than: 70%, 60%, 50%, 40%, 30%; 20%; 10%; 5%; 2.5%; 1%; 0.5%; 0.01 %), or even none, of the efficiency that it catalyzes the conversion of the MEO to the other optically active enantiomer of the vicinal diol. Useful concentrations of the MEO and conditions of incubation will vary from one YEMH polypeptide to another and from one MEO to another. Given the teachings of the working examples contained herein, one skilled in the art will know how to select working conditions for the production of a desired optically active enantiomer of a desired vicinal diol.

The method can be implemented by, for example, incubating (e.g., culturing) the MEO with a wild-type yeast cell or a recombinant cell (yeast or any other host species listed herein) containing a nucleic acid sequence (e.g. a gene, or a recombinant nucleic acid sequence) encoding a YEMH polypeptide, a crude extract from such cells, a semipurified preparation of aYEMH polypeptide, or an isolated YEMH polypeptide. The strain of the yeast cell may be selected from the following genera: *Arxula*, *Brettanomyces*, *Bullera*, *Bulleromyces*, *Candida*, *Cyptococcus, Debaryomyces, Dekhera, Exophiala*, *Geotrichm*, *Hormonema*, *Issatchenkia*, *Kluyveromyces*, *Lipomyces*, *Mastigomyces*, *Myxozyma*, *Pichia*, *Rhodosporidium*, *Rhodotorula*, *Sporidiobolus, Sporobolomyces*, *Trichosporon, Wingea*, or *Yarrowia*.

For the MEO cis-2,3-epoxybutane, yeast strains can be selected from, for example, the following genera: *Brettanomyces*, *Candida*, *Cryptococcus*, *Debaryomces*, *Dekkera*, *Geotricum*, *Issatchenkia, Liponayces*, *Myxozyma*, *Pichia*, *Rhodosporidium*, *Rhodotorula*, or *Trichosporon*.

For the MEO cyclohexene-oxide, yeast strains can be selected from, for example, the following genera: *Bullera*, *Cryptococcus*, *Rhodosponidim*, *Rhodotorula*, *Sporobolomyces*, or *Trichosporon.*

For the MEO cis-epoxysuccinate, the yeast strain can be selected from, for example, the following genera: *Cryptococcus*, *Pichia*, *Rhodosporidium*, *Rhodotorula*, or *Trichosporon.*

In other words, the polypeptide or functional fragment thereof having enantioselective epoxide hydrolase activity can be derived from any yeast strain able to produce a polypeptide which converts or hydrolyses the epoxide shown by the general formula (I), (II), (V), (VI) or (VII) to optically vicinal diols.

Yeast strains innately capable of producing a polypeptide which catalyses the hydrolysis of MEO (e.g., the hydrolysis of cis-2,3-epoxybutane to optically active 2,3 - butanediol) can be selected from the following yeast species:

| No. | **Genus** | **Species** |
|---|---|---|
| 1 | *Brettanomyces* | *B. anomalus* |
| 2 | | *B. bruxellensis* |
| 3 | | *B. species(e.g.,* NCYC 3151) |
| 4 | *Candida* | *C. fabianii* |
| 5 | | *C. kruisii* |
| 6 | *Cryptococcus* | *C. gastricus* |
| 7 | | *C. hungaricus* |
| 8 | | *C. podzolicus* |
| 9 | *Debaryomces* | *D. hansenii* |
| 10 | | *D. napalensis* |
| 11 | *Dekkera* | *D. anomala* |
| 12 | *Geotricum* | *G. species* (e.g., UOFS Y-0111) |

| | | |
|---|---|---|
| 13 | *Issatchenkia* | *I*. *orientalis* |
| 14 | *Lipomyces* | *L. species* (e.g., UOFS Y-2159) |
| 15 | *Myxozyma* | *M. melibiosi* |
| 16 | *Pichia* | *P. finlandica* |
| 17 | | *P. haplophila* |
| 18 | *Rhodosporidium* | *R. lusitaniae* |
| 19 | | *R. sphaerocarpum* |
| 20 | | *R. toruloides* |
| 21 | *Rhodotorula* | *R. araucariae* |
| 22 | | *R. aurantiaca* |
| 23 | | *R. glutinis* |
| 24 | | *R. minuta var. minuta* |
| 25 | | *R. species* (e.g., NCYC 3224) |
| 26 | | *R. sp. minuta* / *mucilaginosa* |
| 27 | *Trichosporon* | *T. delbrueckii* |
| 28 | | *T. moniliiforme* |
| 29 | | *T. pullulans* |
| 30 | | *T. species*(e.g., NCYC 3210) |
| 31 | *Wingea* | *W. robertsiae* |

Yeast strains innately capable of producing a polypeptide which catalyses the hydrolysis of MEO (e.g., the hydrolysis of cyclohexene oxide or cyclopentene oxide to optically active cyclohexane diol or cyclopentane diol) can be selected from the following yeast species:

| | **Genus** | **Species** |
|---|---|---|
| 1 | *Bullera* | *B. dendrophila* |
| 2 | *Cryptococcus* | *C. laurentii* |
| 3 | | *C. podzolicus* |
| 4 | | *C. terreus* |
| 5 | *Rhodosporidium* | *R. paludigenum* |
| 6 | | *R. toruloides* |
| 7 | *Rhodotorula* | *R. araucariae* |
| 8 | | *R. glutiniis* |
| 9 | | *R. mucilaginosa* |
| 10 | *Sporobolomyces* | *S. roseus* |
| 11 | *Trichosporon* | *T. species* (e.g., NCYC 3211) |

Yeast strains innately capable of producing a polypeptide which catalyses the hydrolysis of MEO (e.g., the hydrolysis cis-epoxysuccinate to optically active tartaric acid) can be selected from the following yeast species:

| | **Genus** | **Species** |
|---|---|---|
| 1 | *Cryptococcus* | *C. Curvatus* |
| 2 | | *C. laurentii* |
| 3 | | C. *podzolicus* |
| 4 | *Pichia* | *P. haplophila* |
| 5 | *Rhodosporidium* | *R. paludigenum* |
| 6 | | *R. toruloides* |
| 7 | *Rhodotorula* | *R. araucariae* |
| 8 | | *R. glutinis* |
| 9 | | *R. sp. minute* / *mucilaginosa* |
| 10 | | *R. species* (e.g., NCYC 3192) |
| 11 | *Trichosporon* | *T. montevideense* |
| 12 | | *T. species* (e.g., NCYC 3212) |

The yeast strain can be, for example, one selected from Tables 2-4 (see below).

Cultivation in bioreactors of wild-type or recombinant yeast strains expressing YEMH polypeptides (with the purpose of preparing yeasts stocks or for the enantioselective preparative methods of the invention) can be carried out under conditions that provide useful biomass and/or enzyme titer yields. Cultivation can be by batch, fed-batch or continuous culture methods. Useful cultivation conditions are dependent on the yeast strain used. General procedures for establishing useful growth conditions of yeasts, fungi and bacteria in bioreactors are known to those skilled in the art. The MEO can be added directly to the culture. The concentration of the MEO in the reaction mixtureshould preferentially be at least equal to the solubility of the MEO in the aqueous phase of the reaction mixture. The starting amount of MEO added to the reaction mixture is not critical. The MEO can be metered out continuously or in batch mode to the reaction mixture.

The amount of the yeast cells, crude yeast cell extract, or partially purified or isolated polypeptide having MEO enantio selective activity added to the reaction depends on the kinetic parameters of the specific reaction and the amount of epoxide that is to be hydrolysed. In the case of product inhibition, it can be advantageous to remove the formed vicinal diol from the reaction mixture or to maintain the concentration of the vicinal diol at levels that allow reasonable reaction rates. Techniques used to enhance enzyme and biomass yields include the identification of useful (or optimal) carbon sources, nitrogen sources, cultivation time, dilution rates (in the case of continuous culture) and feed rates, carbon starvation, addition of trace elements and growth factors to the culture medium, and addition of inducers for example substrates or substrate analogs of the epoxide hydrolases during cultivation. In the case of recombinant hosts, the conditions under which the promoters function workably for transcription of the gene encoding the polypeptide with epoxide hydrolase activity are taken into account. At the end of fermentation (culture), biomass and culture medium can be separated by methods known to one skilled in the art, such as filtration or centrifugation.

The processes are generally performed under mild conditions. For example, the reactions can be carried out at a pH from 5 to 10, preferably from 6.5 to 9, and most preferably from 7 to 8.5. The temperature for hydrolysis can be from 0 to 70 °C, preferably from 0 to 50 °C, most preferably from 4 to 40 °C. It is also known that lowering of the temperature of the reaction can enhance enantioselectivity of an enzyme.

The reaction mixture can also contain mixtures of water with at least one water-miscible solvent (e.g., organic water-miscible solvents). Preferably water-miscible solvents are added to the reaction mixture such that epoxide hydrolase activity remains measurable. Water-miscible solvents are preferably organic solvents and can be, for example, acetone, methanol, ethanol, propanol, isopropanol, acetonitrile, dimethylsulfoxide, *N*,*N*-dimethylfonnainide, *N*-methylpyrolidine, and the like.

The reaction mixture can also contain mixtures of water with at least one water-immiscible organic solvent. Examples of water-immiscible solvents that can be used include, for example, toluene, 1,1,2-trichlorotrifluoroethane, methyl *tert*-butyl ether, methyl isobutyl ketone, dibutyl-o-phtalate, aliphatic alcohols containing 6 to 9 carbon atoms (for example hexanol or octanol), aliphatic hydrocarbons containing 6 to 16 carbon atoms (for example cyclohexane, *n*-hexane, *n* -octane, *n* -decane, *n* -dodecane, *n-*tetradecane and *n* -hexadecane or mixtures of the aforementioned hydrocarbons), and the like. Thus, the reaction mixture can include water with at least one water-immiscible organic solvent selected from the group consisting of toluene, 1,1,2-trichlorotrifluoroethane, methyl *tert*-butyl ether, methyl isobutyl ketone, dibutyl-o-phtalate, aliphatic alcohols containing 6 to 9 carbon atoms, and aliphatic hydrocarbons containing 6 to 16 carbon.

The reaction mixture can also contain surfactants (for example Tween 80), cyclodextrins or phase-transfer catalysts and the like that can increase, selectively or otherwise, the solubility of the meso-epoxide in the reaction mixture.

The reaction mixture can also contain a buffer. Buffers are known in the art and include, for example, phosphate buffers, citrate buffers, Tris buffers, or HEPES buffers.

The production of the YEMH polypeptides, including functional fragments, can be, for example, as recited above in the section on Polypeptides and Polypeptide Fragments. Thus they can made by production in a natural host cell, production in a recombinant host cell, or synthetic production. Recombinant production can be carried out in host cells of microbial origin. Preferred yeast host cells are selected from, but are not limited to, the genera *Saccharomyces*, *Arxula*, *Kluyveromyces*, *Hansenula*, *Pichia*, *Yarrowia* and *Candida.* Preferred bacterial host cells include *Escherichia coli*, *Agrobacterium* species, *Bacillus* species and *Streptomyces* species. Preferred filamentous fungal host cells are selected from the group consisting of the genera *Aspergillus*, *Trichoderma*, and *Fusarium*. The production of the polypeptide can be, e.g., intra- or extra- cellular production and can be by, e.g., secretion into the culture medium.

In the fermentation reactions, the polypeptides (including functional fragments) can be immobilized on a solid support or free in solution. Procedures for immobilization of the yeast or preparation thereof include, but are not limited to, adsorption; covalent attachment; cross-linked enzyme aggregates; cross-linked enzyme crystals; entrapment in hydrogels; and entrapment into reverse micelles.

The progress of the reaction can be monitored by standard procedures known to one skilled in the art, which include, for example, gas chromatography or high-pressure liquid chromatography on columns containing chiral stationary phases. The vicinal diol formed can be removed from the reaction mixture at one or more stages of the reaction

The reaction can be stopped when one enantiomer of the vicinal diol is found to be in excess compared to the other enantiomer of the vicinal diol. Preferably, the reaction is stopped when one enantiomer of a vicinal diol of general formula (III), (IV), (VIII), (IX), or (X) is found to be in an enantiomeric excess of at least 90%. In a more preferred embodiment of the invention, the reaction is stopped when one enantiomer of a vicinal diol of general formula (III), (IV), (VIII), (IX), or (X) is found to be in an enantiomeric excess of at least 95%. The reaction can be stopped by the separation (for example centrifugation, membrane filtration and the like) of the yeast, or a preparation thereof, from the reaction mixture or by inactivation (for example by heat treatment or addition of salts and/or organic solvents) of the yeast or polypeptide, or preparation thereof. Thus, the reaction can be stopped for by, for example, the separation of the catalytic agent from the reactants and products in the mixture, or by ablation or inhibition of the catalytic activity, by techniques known to one skilled in the art.

The optically active vicinal diols produced by the reaction can be recovered from the reaction mixture, directly or after removal of the yeast, or preparation thereof. Preferably, the process can include continuously recovering the optically active vicinal diol produced by the reaction directly from the reaction mixture. Methods of removal of the optically active vicinal diol produced by the reaction include, for example, extraction with an organic solvent (such as hexane, toluene, diethyl ether, petroleum ether, dichloromethane, chloroform, ethyl acetate and the like), vacuum concentration, crystallisation, distillation, membrane separation, column chromatography and the like.

Thus, the present invention provides an efficient process with economical advantages compared to other chemical and biological methods for the production, in high enantiomeric purity, of optically active vicinal diols of the general formula (III), (IV), (VIII), (IX) or (X) from *meso*-epoxides in the presence of a yeast strain having enantioselective epoxide hydrolase activity or a polypeptide having such activity.

### Yeast epoxide hydrolase antibodies

The invention features antibodies that bind to yeast epoxide hydrolase polypeptides or fragments (e.g., antigenic or functional fragments) of such polypeptides. The polypeptides are preferably yeast epoxide polypeptides with enantioselective activity, and in particular those with *meso*-epoxide enantioselective activity (i.e.,YEMH), e.g., those with SEQ ID NOs: 1-9. The antibodies preferably bind specifically to yeast epoxide hydrolase polypeptides, i.e., not to epoxide hydrolase polypeptides of species other than yeast species. More preferably, they can bind specifically to yeast epoxide polypeptides with enantioselective activity, and in particular to YEMH polypeptides, e.g., those with SEQ ID NOs: 1-9. They can moreover bind specifically to one or more of polypeptides with SEQ ID NOs: 1, 2, 3, 4, 5, 6, 7, 8, or 9.

Antibodies can be polyclonal or monoclonal antibodies; methods for producing both types of antibody are known in the art. The antibodies can be of any class (e.g., IgM, IgG, IgA, IgD, or IgE). They are preferably IgG antibodies. Moreover, polyclonal antibodies and monoclonal antibodies can be generated in, or generated from B cells from, animals any number of vertebrate (e.g., mammalian) species, e.g., humans, non-human primates (e.g., monkeys, baboons, or chimpanzees), horses, goats, camels, sheep, pigs, bovine animals (e.g., cows, bulls, or oxen), dogs, cats, rabbits, gerbils, hamsters, guinea pigs, rats, mice, birds (such as chickens or turkeys), or fish.

Recombinant antibodies specific for YEMH polypeptides, such as chimeric monoclonal antibodies composed of portions derived from different species and humanized monoclonal antibodies comprising both human and non-human portions, are also encompassed by the invention. Such chimeric and humanized monoclonal antibodies can be produced by recombinant DNA techniques known in the art, for example, using methods described in Robinson et al., International Patent Publication PCT/US86/02269; Akira et al., European Patent Application 184,187; Taniguchi, European Patent Application 171,496; Morrison et al., European Patent Application 173,494; Neuberger et al., PCT Application WO 86/01533; Cabilly et al., U.S. Patent No. 4,816,567; Cabilly et al., European Patent Application 125,023; Better et al. (1988) Science 240, 1041-43; Liu et al. (1987) J. Immunol. 139, 3521-26; Sun et al. (1987) PNAS 84,214-18; Nishimura et al. (1987) Canc. Res. 47, 999-1005; Wood et al. (1985) Nature 314, 446-49; Shaw et al. (1988) J. Natl. Cancer Inst. 80, 1553-59; Morrison, (1985) Science 229, 1202-07; Oi et al. (1986) BioTechniques 4, 214; Winter, U.S. Patent No. 5,225,539; Jones et al. (1986) Nature 321, 552-25; Veroeyan et al. (1988) Science 239, 1534; and Beidler et al. (1988) J. Immunol. 141, 4053-60.

Also useful for the invention are antibody fragments and derivatives that contain at least the functional portion of the antigen-binding domain of an antibody that binds to a YEMH polypeptide. Antibody fragments that contain the binding domain of the molecule can be generated by known techniques. Such fragments include, but are not limited to: F(ab')₂ fragments that can be produced by pepsin digestion of antibody molecules; Fab fragments that can be generated by reducing the disulfide bridges of F(ab')₂ fragments; and Fab fragments that can be generated by treating antibody molecules with papain and a reducing agent. See, e.g., National Institutes of Health, 1 Current Protocols In Immunology, Coligan et al., ed. 2.8, 2.10 (Wiley Interscience, 1991). Antibody fragments also include Fv fragments, i.e., antibody products in which there are few or no constant region amino acid residues. A single chain Fv fragment (scFv) is a single polypeptide chain that includes both the heavy and light chain variable regions of the antibody from which the scFv is derived. Such fragments can be produced, for example, as described in U.S. Patent No. 4,642,334, which is incorporated herein by reference in its entirety. The antibody can be a "humanized" version of a monoclonal antibody originally generated in a different species.

The above-described antibodies can be used for a variety of purposes including, but not limited to, YEMH polypeptide purification, detection, and quantitative measurement.

The following examples serve to illustrate, not limit, the invention.

### EXAMPLES

### Example I. Materials and Methods

### Determination of concentrations and enantiomeric excesses

In the examples, quantitative determinations of the compounds and determination of enantiomeric excesses were carried out by gas chromatography or HPLC. Gas chromatography (GLC) was performed on a Hewlett-Packard 6890 gas chromatograph equipped with FID detector and using H₂ as carrier gas. HPLC was performed on a Hewlett-Packard HP 1100 high pressure liquid chromatograph equipped with a UV detector. Determination of the enantiomeric excesses of 2,3-butanediol, cyclohexane diol and tetrahydro-3,4-furandiol was performed by GLC using fused silica cyclodextrin capillary columns (Supelco) (30 m length, 25 mm ID and 25 µm film thickness). Determination of the enantiomeric excesses of tartaric acid formed from cis epoxysuccinic acid was performed by HPLC using an Astec CLC-D column (150 x 4.6 mm). Determination of the enantiomeric excess of 1,2-diphenyl-1,2-ethanediol was performed by HPLC using a Chiralcel OJ-R column (150 x 4.6 mm). Reaction conditions and retention times were as follows:
**2,3-butanediol:** β-DEX 110 at 90°C isotherm, head pressure 14 psi, split flow 80 ml/min. Retention times (min): Rₜ (S,S)-diol = 4.50, Rₜ (R,R)-diol = 4.65, Rₜ (meso)-diol = 5.26.
**cyclohexane diol:** β-DEX 225 at 110°C isotherm, head pressure 14 psi, split flow 80 ml/min. Retention times (min): Rₜ(S,S)-diol = 8.7, Rₜ(R,R)-diol = 9.6.
**cyclopentane diol:** β-DEX 225 at 100°C isotherm, head pressure 14 psi, split flow 80 ml/min. Retention times (min): Rₜ(S,S)-diol = 8.7, Rₜ(R,R)-diol = 9.6.
**tetrahydro-3,4-furandiol:** α-DEX 120 at 120°C isotherm, head pressure 14 psi, split flow 80 ml/min. Retention times (min):: Rₜ(R,R)-diol = 13.7, Rₜ(S,S)-diol = 14.5.
**tartaric acid:** Astec CLC-D column. Mobile phase: 5 mM CuSO₄, pH 3.1. Flow rate: 1 ml/min. Detection: UV, 254 nm. Rt (D)-tartaric acid = 5.66, Rt (L)-tartaric acid = 6.66.
**1,2-diphenyl-1,2-ethanediol:** Chiralcel OJ-R, mobile phase: Hexane / 2-propanol 90:10, flow rate 0.5 ml/min. Retention times (min): Rt (S,S) = 27.3, Rt (R,R) = 30.2.

Concentrations of the diol enantiomers were derived from calibration curves obtained from extractions of the diol from buffer in the absence of active cells.

### Preparation of frozen yeast cells for screening

Yeasts were grown at 30°C in 1 L shake-flask cultures containing 200 ml yeast extract/malt extract (YM) medium (3% yeast extract, 2% malt extract, 1% peptone w/v) supplemented with 1% glucose (w/v). At late stationary phase (48 - 72 h), the cells were harvested by centrifugation (10 000 g, 10 min, 4°C), washed with phosphate buffer (50 mM, pH7.5), pelleted by centrifugation, and frozen in phosphate buffer containing glycerol (20%) at -20°C as 20% (w/v) cell suspensions. The cells were stored for several months without significant loss of activity.

### Yeast isolate (strain) screening

Epoxide (10 µl of a 1M stock solution in EtOH or DMSO) was added to a final concentration of 20 or 50 mM to 500 µl cell suspension (20 or 50 % w/v) in phosphate buffer (50 mM, pH 7.5). The reaction mixtures were incubated at 30°C for 1 to 8 hours. The reaction mixtures were extracted with EtOAc (300 µl) and centrifuged. Vicinal diol formation was evaluated by TLC (silica gel Merck 60 F₂₅₄). Compounds were visualized by spraying with vanillin/conc. H₂SO₄ (5g/l). Reaction mixtures that showed substantial vicinal diol formation were evaluated for asymmetric hydrolysis of the epoxide by chiral GLC or HPLC analysis. Some reactions were repeated over longer or shorter times in order to analyse the reactions at suitable conversions.

### General procedure for the hydrolysis of meso-epoxides

Frozen cells were thawed, washed with phosphate buffer (50 mM, pH 7.5) and resuspended in buffer. Cell suspensions (10 ml, 20% or 50% w/v) were placed in 20 ml glass bottles with screw caps fitted with septa. The substrate (100 or 250 µl of a 2M (v/v) stock solution in EtOH or DMSO) was added to final concentrations of 20 mM or 50 mM. The mixtures were agitated on a shaking water bath at 30°C. The course of the bioconversions of epoxides was followed by withdrawing samples (500 µl) at appropriate time intervals. For chiral GLC analysis, samples were extracted with 300 µl EtOAc and centrifuged (3000 x g, 2 min). The organic layer was dried over anhydrous MgSO₄ prior to analysis by chiral GLC. For HPLC analysis of tartaric acid, the reactions were stopped by removal of the catalyst by centrifugation (10 000 x *g*, 10 min) and the supernatant analysed directly. For HPLC analysis of diphenylethanediol (hydrobenzoin), the samples were extracted with hexane and centrifuged (3000 x *g*, 2 min). The organic layer was dried over anhydrous MgSO₄, prior to analysis by chiral HPLC.

### Determination of the absolute configuration of diols

Absolute configurations of cyclopentane diol, cyclohexane diol, 2,3-butanediol and tartaric acid were determined by co-injection on GC of the commercially available (1R,2R)- and (1S,2S)- trans diols (Fluka). Absolute configurations of tetrahydrofurandiol and 1,2-diphenyl-1,2-ethanediol (hydrobenzoin) were deduced from the elution orders on the same chiral columns previously reported (Zhao et al., 2004; Li et. al., 2004).

### Yeast strains

Yeast strains designated "Jen" and numerical screen numbers were obtained from the Yeast Culture Collection of the University of the Free State. Yeast strains with screen numbers containing "AB" or "Car" or "Alf" or "Poh" were isolated from soil from specialised ecological niches selected based on the inventors' belief that selectivity for specific classes of epoxides in microorganisms would be determined by environmental factors such as terpene-rich environments or highly contaminated soil. "AB" and "Alf" strains were isolated from Cape Mountain fynbos, an ecological environment unique to South Africa, "Car" strains were isolated from soil under pine trees, and "Poh" strains from soil contaminated by high concentrations of cyanide. It seemed likely that microorganisms existing in these contaminated soil would have alternative respiratory mechanisms.

All new isolates that produced optically active vicinal diols during hydrolysis of MEO were identified by known biochemical characterisation methods, and most of the isolates were also subjected to molecular identification by sequence analyses of the D1/D2 region of the large subunit rDNA. These new isolates were subsequently deposited at the Yeast Culture Collection of University of the Orange Free State (UOFS) and assigned UOFS numbers. Some of the isolates were deposited at the National Collection of Yeast Cultures (NCYC), Norwich, United Kingdom.

### Example II. Sequencing, cloning and overexpression of wild type yeast epoxide hydrolases in Yarrowia lipolytica as production host under the control of different promoters

### 1. Vectors, Strains and Primers (Table 1)

The following features are common to all the *E. coli*/*Y. lipolytica* auto-cloning integrative vectors used:
- LIP2 terminator
- *Zeta* regions
- Kanamycin resistance for *E. coli* selection
- mono-copy auto cloning vectors (pINA 1311, pINA 1313, pINA 3313) with a fully functional selection marker gene carry the fully functional *ura3d1* allele from the *URA3* selection marker gene
- multi-copy auto cloning vectors (pINA 1291, pINA 1293, pINA 3293) with a defective selection marker gene (copy number amplification) carry the defective *ura3d4* allele from the *URA3* selection marker gene

**Table 1. Vectors, strains and primers**

| | | **Description** | | **Cloning sites** | |
|---|---|---|---|---|---|
| **Vectors** | **Promoter** | **Selection marker** | **Targeting sequence** | **Upstream/down stream** | **Reference/ Origin** |
| pINA1291 (pYLHmA) | hp4d | ura3d4 | none | *Pml*I (blunt) / *Bam*HI, *Kpn*I, *Avr*II | Nicaud *et al* (2002) |
| pYL3313 = | TEF | ura3d1 | none | *Xmn*I (in pro) / | This study |
| pKOV93 (pYLTsA) | | | | *Bam*HI, *Kpn*I, *Avr*II | |

| **Host Strain** | **Description** | | | | **Reference/ Origin** |
|---|---|---|---|---|---|
| *Yarrowia lipolytica* Po1h | *MATA*, *ura3-302*, *uxpr2-322*, *axp1-2* (deleted for both extracellular proteases and growth on sucrose) | | | | CLIB882 |

| **Primers** | **Sequence** | | | **Specifications** | |
|---|---|---|---|---|---|
| YL-fwd | 5'-GGA GTT CTT CGC CCA C-3' (SEQ ID NO:19) | | | amplification of expression | |
| YL-rev | 5'-GAT CCC CAC CGG AAT TG-3' (SEQ ID NO:20) | | | casette between *Not*I sites | |
| pINA-1 | 5'- CAT ACA ACC ACA CAC ATC CA-3' (SEQ ID NO:21) | | | PYLHmA fwd primer | |
| pINA-2 | 5'-TAA ATA GCT TAG ATA CCA CAG-3' (SEQ ID NO:22) | | | pYLTsA/pYLHmA rev primer | |
| pINA-3 | 5'-CTC TCT CTC CTT GTC AAC T-3' (SEQ ID NO:23) | | | pYLTsA fwd primer | |

### 2. Transformants (multi-copy and single-copy)

| **Transformants** | **Gene origin** |
|---|---|
| **TEF promoter** | **Vector: pYL3313 (1313) = (pYLTsA)** |
| YL23TsA | *Rhodotorula mucilaginosa* NCYC 3190 |
| YL25TsA | *Rhodotorula araucariae* NCYC 3183 |
| YL46TsA | *Rhodosporidium toruloides* UOFS Y-0471 |
| YL692TsA | *Rhodosporidium paludigenum* NCYC 3179 |
| YL1TsA | *Rhodosporidium toruloides* NCYC 3181 |
| YLCar54 TsA | *Cryptococcus curvatus* NCYC 3158 |
| YLJen 46-2TsA | *Yarrowia lipolytica* NCYC 3229 |
| YL777TsA | *Filobasidium neoformans var. neoformans* (GenBank accession no. XM 568708) (#777). |

| **Hp4d promoter** | **Vector: pINA1291=( pYLHmA)** |
|---|---|
| YL25HmA | *Rhodotorula araucariae* NCYC 3183 |
| YL46HmA | *Rhodosporidium toruloides* UOFS Y-0471 |
| YL692HmA | *Rhodosporidium valudigenum* NCYC 3179 |

### 3. Vector preparation

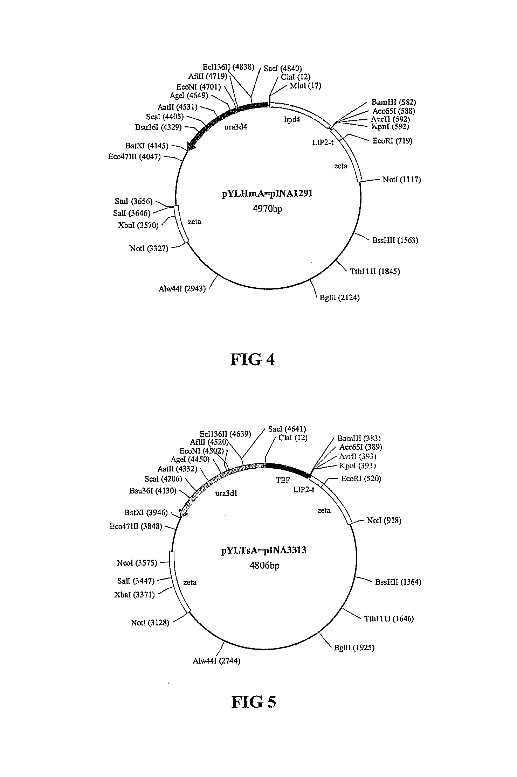

pINA1291 (Fig. 4) was obtained from Dr Madzak of Labo de Génétique, INRA, CNRS. This vector was renamed pYLHmA (***Y**arrowia **L**ipolytica* expression vector, with **H**p4d promoter, **m**ulti-copy integration selection, **A**bsent secretion signal).

pINA3313 (pKOV93) (Fig. 5) was prepared by the inventors. This vector was renamed pYLTsA (*Y**a**rrowia **L**ipolytica* expression vector, with **T**EF promoter, single-copy integration selection, **A**bsent secretion signal).

Examples and figures that make reference to recombinant strains utilize recombinant strain identification codes in such a way as to iterate host (e.g. **YL**), gene origin reference (e.g. #**25**), promoter (e.g. **H**p4d), copy insertion (e.g. **m**ulti), and absence of secretion signal (e.g. **A**bsent); e.g. **YL-25HmA.** (Note the variation of code text does not change the strain identification ; i.e. YL-25HmA = YL25HmA = YL 25HmA).

To prepare the vectors for ligation with an epoxide hydrolase coding sequence (or other insert to be expressed in *Y. lipolytica*), DNA was digested with *Bam*HI and *Avr*II.

### 4. Insert preparation

Total RNA was isolated from selected yeast strain cells and messenger RNA (mRNA) was purified from it. The mRNA was used as a template to synthesise complementary DNA (cDNA) using reverse transcriptase. The cDNA was then used as a template for Polymerase Chain Reaction (PCR) using appropriate primers. PCR primers were selected by repeated experimentation using multiple test primers for each yeast strain, the sequences of which were based on previously described epoxide hydrolase sequences from a variety of species. The nucleotide sequences of the forward and reverse primers used to generate cDNA coding sequences from mRNA from seven different yeast strains with appropriate restriction enzyme recognition sites at their termini are shown below. Restriction enzyme recognition sequences are underlined and the relevant restriction enzymes are shown in parentheses.

| **Strain** | **Forward primer** | **Reverse primer** |
|---|---|---|
| *R. toruloides* NCYC 3181 #1 | GTGGATCCATGGCG ACACACA (BamHI) (SEQ ID NO:24) | GACCTAGGCTACTTC TCCCACA (BlnI) (SEQ ID NO:25) |
| *R. toruloides* UOFS Y-0471 #46 | | |
| *C*. *curvatus* NCYC 3158 Car 054 | | |
| *R. araucariae* NCYC 3183 #25 | GATTAATGATCAAT GAGCGAGCA (BclI) (SEQ ID NO:26) | GACCTAGGTCACGAC GACAG (BlnI) (SEQ ID NO:27) |
| *R. paludigenum* NCYC 3179 #692 | GTGGATCCATGGCT GCCCA (BamHI) (SEQ ID NO:28) | GAGCTAGCTCAGGCC TGG (NheI) (SEQ ID NO:29) |
| *R. mucilaginosa* NCYC 3190 # 23 | GTATATCTATGCCC GCCCGCT (BglII) (SEQ ID NO:30) | GACCTAGGCTACGAT TTTTGCT (BlnI) (SEQ ID NO:31) |
| *Y. lipolytica* NCYC 3229 Jen 46-2 | GCAGATCTATGTCA TCACTCG (BglII) (SEQ ID NO:32) | GACCTAGGCTACAAC TTCGACG (BlnI) (SEQ ID NO:33) |
| *Debaromyces hansenii* NCYC 3167 (#113) | GTGGATCCATGATG CAAGG(BamHI) (SEQ ID NO:34) | GACCTAGGCTAAGGA TATT (BlnI) (SEQ ID NO:35) |
| Filobasidium neoformans (# 777) | GAGGATCCATGTCG TATTCAGA (BamHI) (SEQ ID NO:36) | GAGCTAGCTCAGTAA TTACCTTTG (NheI) (SEQ ID NO:37) |

Each PCR reaction contained 200 µM dNTPs, 250 nM of each primer, 2 mM of MgCl₂, cDNA and 2.5 U of Taq polymerase in a 50 µl reaction volume. The PCR profile used was: 95°C for 5 minutes, followed by 30 cycles of: 95°C - 1 min, 50°C - 1 min, 72°C - 2 min, then a final extension of 72°C for 10 minutes. The PCR products were purified and digested with the restriction enzymes whose recognition sites are engineered at the end of the primers. The cDNA fragment was cloned into a vector and sequenced for confirmation.

Coding seqences to be inserted in either pYLHmA or pYLTsA were prepared with *Bam*HI and *Avr*II at their termini. The above PCR primers were designed with these restriction sites, unless the sites were also present in the gene to be inserted. If this occurred, appropriate compatible restriction enzymes were selected. PCR template DNA was either the insert cloned into a different vector, or cDNA synthesized from the original host organism. PCR reactions consisted of 200 µM dNTP's, 250 nM each primer, 1X Taq polymerase buffer, and 2.5 units Taq polymerase per 100 µl reaction. The amplification programme used was: 95°C for 5 minutes, 30 cycles of 95°C for 1 minute, 50°C for 1 minute, and 72°C for 2 minutes, followed by 72°C for 10 minutes.

PCR products were purified and digested with the relevant restriction enzymes. The digested DNA was subsequently repurified and was ready for ligation into the prepared vector.

### 5. Preparation of pYLHmA or pYLTsA constructs

Vector and insert were ligated at pmol end ratios of 3:1 - 10:1 (insert:vector), using commercial T4 DNA Ligase. Ligations were electroporated into any laboratory strain of *Escherichia coli,* using the Bio-Rad GenePulser, or equivalent electroporator. Transformants were selected on LM media (10 g/L yeast extract, 10 g/L tryptone, 5 g/L NaCl), supplemented with kanamycin (50 µg/ml). Transformants were selected based on restriction enzyme digests of purified plasmid DNA.

### 6. Yarrowia lipolytica transformation

### 6.1.1. Preparation of DNA - method 1

Digestion of the pINA-series of plasmids with *Not*I resulted in the release of a bacterial DNA-free expression cassette, containing the ura3d4 (pYLHmA) or the ura3d1 (pYLTsA) marker gene and the promoter-gene-terminator.

Scaled-up quantities of each plasmid were isolated. *Not*I was used to restrict the plasmid DNA, and the digested DNA was run on an agarose gel. *Not*I digests resulted in generation of the bacterial fragment of the plasmid as a band at 2210 bp, and the expression cassette as a band of 2760 bp + size of insert (pYLHmA) or 2596 bp + size of insert (pYLTsA). The expression cassette fragments were excised from the gel and purified from the agarose. The purified fragment was used for transformation of *Y. lipolytica* Po1h.

### 6.1.2. Preparation of DNA - method 2

Primers YL-Fwd and YL-Rev (Table 1) were used to amplify the expression cassette. PCR reactions consisted of 200 µM dNTP's, 250 pmol each primer, 1X Taq buffer and 2.5 units Taq per 100 µl reaction. The amplification programme used was 95°C for 5 minutes, 30 cycles of 95°C for 1 minute, 50°C for 1 minute, and 72°C for 3 ½ minutes, followed by a single incubation at 72°C for 10 minutes. The PCR product was purified from the PCR reaction mix and used for transformation of *Y. lipolytica* Polh.

### 6.1.3. Preparation of carrier DNA

DNA from salmon testes was made up as a 10 mg/ml stock in TE (10 mM Tris-HCl, pH 8.0, 1 mM EDTA) and sonicated to result in fragments that ranged from approximately 15 kb to 100 bp, with most fragments in a range of 6 to 10 kb. The DNA was denatured by boiling. Aliquots were stored at -20°C.

### 6.1.4. Transformation of Yarrowia lipolytica with pYLHmA or pYLTsA

An adaptation of the method of Xuan *et al* (1988) was used for the transformation of *Y. lipolytica* Polh. The yeast was inoculated into 50 ml YPD (10 g/L yeast extract, 20 g/L peptone, 20 g/L glucose). The culture was incubated at 30°C, 220 rpm until cell densities of 8 x 10⁷ - 2 x 108 cells/ml were reached. The entire culture was harvested, the pellet resuspended in 10 ml TE and reharvested. 1 ml TE + 0.1 M LiOAc was used to resuspend the pellet and the culture was incubated at 28°C in a ProBlot Jr (Labnet) hybridisation oven, set at 4 rpm (or similar incubator) for 1 hour. Transformation mixes were set up with 0.5 - 2 µg of transforming DNA + 5 µg of carrier DNA with 100 µl of treated cells.

Each mix was set up in a 1.5 ml microfuge tube, and incubated in a 28°C heating block for 30 minutes. 7 volumes of PEG reagent (40% PEG 4,000, 0.1 M LiOAc, 10 mM Tris, 1 mM EDTA, pH 7.5, filter-sterilised) were added to each, mixed carefully and incubated at 28°C for a further 1 hour. The tubes were transferred to a 37°C heating block for 15 minutes, and then the cells were pelleted by centrifugation for 1 minute at 13,000 rpm. The cell pellets were carefully resuspended in 100 µl dH₂O. The transformations were plated on *Y. lipolytica* selective plates (17 g/l Difco yeast nitrogen base without amino acids and without (NH₄)₂SO₄, 20 g/L glucose, 4 g/L NH₄Cl, 2 g/L casamino acids, 300 mg/L leucine) and incubated at 28°C.

Colonies which appeared on the selective plates after 3 - 7 days were transferred onto fresh plates and regrown.

### 6.1.5. Confirmation of integration of pYLHmA or pYLTsA

Colonies that grew on the newly-streaked selective plates were inoculated into 5 ml of YPD medium and grown at 30°C, 200 rpm for 24-48 hours. A small-scale genomic DNA isolation was performed.

PCR was performed using this genomic DNA as template, with either pINA-1 and pINA-2 as primers (transformants with pYLHmA), or pINA-3 and pINA-2 (pYLTsA) (Table 1). Each PCR reaction contained 200 µM dNTPs, 250 nM of each primer, 2 mM of MgCl₂, genomic DNA and 2.5 U/50µl of Taq polymerase. The PCR profile was as described above in this example. These primer sets gave products the size of the inserted genes.

### Example III. Selection of wild type yeasts that are able to produce optically active vicinal diols from MEO

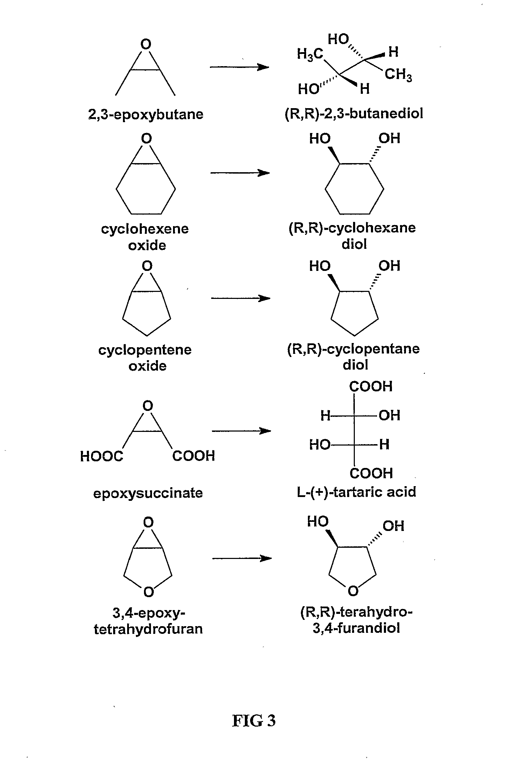

Yeasts were cultivated, harvested and frozen as 20% or 50 % (w/v) cell suspensions as described above. The MEO was added to a final concentration of 20 mM and the screening was performed as described above. Strains with the highest activities as judged by TLC from diol formation were subjected to chiral GC or HPLC analysis as described above. The enantiomeric excesses of the formed diol after incubation for 2-20 hours of three MEO (cis-2,3-epoxybutane; cyclohexene oxide; and cis-epoxysuccinic acid) with the different yeast strains are shown as Samples 1-145 in Tables 2-4. The generation of the optically active vicinal diols from these three MEO is illustrated in Fig. 3. Enantiomeric excesses (ee) were determined from the formula: E.e. = [[R,R]-[S,S]}/{[R,R]+[S,S]} x 100. [S,S] is the concentration at the appropriate time point of the (S,S) diol enantiomer and [R,R] is the concentration at the appropriate time point of the (R,R)-diol enantiomer.

**Table 2. (Samples 1-47) Enantiomeric excesses (ee) of formed diol obtained after incubation of different yeast strains with cis-2,3-epoxybutane (50 mM, 50% w/v cells, 16 hours).**

| **Sample No.** | **Internal strain No.** | **Strain/species** | **Culture collection No.** | **Abs Conf.** | **ee (%)** |
|---|---|---|---|---|---|
| 1 | K01 | *Brettanomyces anomalus* | NCYC 3149 | R,R | 71.0 |
| 2 | 52 | *Brettanomyces bruxellensis* | NCYC 3150 | R,R | 19.3 |
| 3 | N13 | *Brettanomyces sp.* | NCYC 3151 | R,R | 74.0 |
| 4 | 704 | *Candida fabianii* | UOFS Y-0963 | R,R | 84.1 |
| 5 | X19 | *Candida kruisii* | NCYC 3153 | R,R | 77.7 |
| 6 | Jen 13 | *Cryptococcus gastricus* | UOFS Y-0477 | R,R | 31.9 |
| 7 | AB 54 | *Cryptococcus podzolicus* | UOFS Y-1900 | R,R | 27.4 |
| 8 | 110 | *Debaryomces hansenii* | UOFS Y-0614 | R,R | 59.5 |
| 9 | 108 | *Debaryomces hansenii* | UOFS Y-0612 | R,R | 86.0 |
| 10 | 93 | *Debaryomyces hansenii* | NCYC 3169 | R,R | 55.6 |
| 11 | 105 | *Debaryomyces hansenii* | UOFS Y-0608 | R,R | 93.9 |
| 12 | 111 | *Debaryomyces hansenii* | UOFS Y-0615 | R,R | 95.8 |
| 13 | TVN 119 | *Debaryomyces napalensis* | NCYC 3220 | R,R | >98% |
| 14 | 88 | *Dekkera anomala* | NCYC 3170 | R,R | 17.4 |
| 15 | 227 | *Geotricum sp.* | UOFS Y-0111 | R,R | 33.4 |
| 16 | TVN 228 | *Issatchenkia orientalis* | NCYC 3221 | R,R | 60.1 |
| 17 | TVN 229 | *Issatchenkia orientalis* | NCYC 3222 | R,R | 63.0 |
| 18 | TVN 231 | *Issatchenkia orientalis* | NCYC 3223 | R,R | 62.5 |
| 19 | 45a | *Lipomyces sp.* | UOFS Y-2159 | R,R | 77.5 |
| 20 | 41 | *Myxozyma melibiosi* | NCYC 3172 | R,R | 18.9 |
| 21 | 520* | *Pichia finlandica* | NCYC 3173 | R,R | >98% |
| 22 | 673 | *Pichia haplophila* | NCYC 3177 | R,R | 65.7 |
| 23 | 169 | *Rhodosporidium lusitaniae* | NCYC 3178 | R,R | 31.7 |
| 24 | 40 | *Rhodosporidium sphaerocarpum* | NCYC 3180 | R,R | 27.4 |
| 25 | Alf 01 | *Rhodosporidium toruloides* | NCYC 3181 | R,R | 60.0 |
| 26 | 46 | *Rhodosporidium toruloides* | UOFS Y-0471 | R,R | 45.0 |
| 27 | 2 | *Rhodosporidium toruloides* | UOFS Y-0518 | R,R | 88.7 |
| 28 | Car 067 | *Rhodosporidium toruloides* | UOFS Y-2236 | R,R | 49.6 |
| 29 | Car 076 | *Rhodosporidium toruloides* | UOFS Y-2238 | R,R | 85.3 |
| 30 | Car 131 | *Rhodosporidium toruloides* | UOFS Y-2252 | R,R | 83.9 |
| 31 | Car 142 | *Rhodosporidium toruloides* | UOFS Y-2255 | R,R | 43.3 |
| 32 | Car 209 | *Rhodosporidium toruloides* | UOFS Y-2260 | R,R | 84.0 |
| 33 | 25 | *Rhodotorula araucariae* | NCYC 3183 | R,R | 81.1 |
| 34 | EP 230 | *Rhodotorula aurantiaca* | NCYC 3185 | R,R | 41.8 |
| 35 | 50 | *Rhodotorula glutinis* | NCYC 3186 | R,R | 86.1 |
| 36 | 680 | *Rhodotorula glutinis* | NCYC 3203 | R,R | 77.9 |
| 37 | 713 | *Rhodotorula glutinis* | UOFS Y-0489 | R,R | 45.2 |
| 38 | Alf 6 | *Rhodotorula glutinis* | UOFS Y-0513 | R,R | 95.5 |
| 39 | 185 | *Rhodotorula glutinis* | UOFS Y-0559 | R,R | 49.5 |
| 40 | 686 | *Rhodotorula minuta var. minuta* | NCYC 3188 | R,R | 67.2 |
| 41 | Rh 27 | *Rhodotorula sp.* | NCYC 3224 | R,R | 89.0 |
| 42 | 697 | *Rhodotorula sp. minuta* / *mucilaginosa* | UOFS Y-0958 | R,R | 46.7 |
| 43 | 698 | *Rhodotorula sp. minuta* / *mucilaginosa* | UOFS Y-0959 | R,R | 50.2 |
| 44 | BV04 | *Trichosporon moniliiforme* | NCYC 3214 | R,R | 33.6 |
| 45 | 61 | *Trichosporon pullulans* | NCYC 3209 | R,R | 91.4 |
| 46 | 231 | *Trichosporon sp.* | NCYC 3210 | R,R | 97.6 |
| 47 | 39 | *Wingea robertsiae* | NCYC 3228 | R,R | 22.1 |

**Table 3. (Samples 48-97) Enantiomeric excesses (ee) of formed diol obtained after incubation of different yeast strains with cyclohexene oxide (20 mM, 20% w/v cells, 6 hours).**

| **Sample No.** | **Internal strain No.** | **Strain/species** | **Culture Collection No.** | **Abs. Conf.** | **ee (%)** |
|---|---|---|---|---|---|
| 48 | 43 | *Bullera dendrophila* | NCYC 3208 | R,R | 71 |
| 49 | Jen 12 | *Cryptococcus laurentii* | NCYC 3160 | R,R | 67 |
| 50 | AB 58 | *Cryptococcus podzolicus* | NCYC 3164 | R,R | 71 |
| 51 | AB-50 | *Cryptococcus podzolicus* | UOFS Y-1883 | R,R | 65 |
| 52 | AB-30 | *Cryptococcus podzolicus* | UOFS Y-1904 | R,R | 77 |
| 53 | 385 | *Cryptococcus terreus* | NCYC 3166 | R,R | 54 |
| 54 | 692 | *Rhodosporidium paludigenum* | NCYC 3179 | R,R | 64 |
| 55 | Alf 01 | *Rhodosporidium toruloides* | NCYC 3181 | R,R | 66 |
| 56 | 46 | *Rhodosporidium toruloides* | UOFS Y-0471 | R,R | 83 |
| 57 | Alf 2 | *Rhodosporidium toruloides* | UOFS Y-0518 | R,R | 83 |
| 58 | Car-003 | *Rhodosporidium toruloides* | UOFS Y-2222 | R,R | 53 |
| 59 | Car-006 | *Rhodosporidium toruloides* | UOFS Y-2223 | R,R | 67 |
| 60 | Car-020 | *Rhodosporidium toruloides* | UOFS Y-2226 | R,R | 66 |
| 61 | Car-038 | *Rhodosporidium toruloides* | UOFS Y-2228 | R,R | 88 |
| 62 | Car-052 | *Rhodosporidium toruloides* | UOFS Y-2230 | R,R | 87 |
| 63 | Car-059 | *Rhodosporidium toruloides* | UOFS Y-2231 | R,R | 78 |
| 64 | Car-067 | *Rhodosporidium toruloides* | UOFS Y-2236 | R,R | 75 |
| 65 | Car-070 | *Rhodosporidium toruloides* | UOFS Y-2237 | R,R | 91 |
| 66 | Car-076 | *Rhodosporidium toruloides* | UOFS Y-2238 | R,R | 66 |
| 67 | Car-077 | *Rhodosporidium toruloides* | UOFS Y-2239 | R,R | 70 |
| 68 | Car-078 | *Rhodosporidium toruloides* | UOFS Y-2240 | R,R | 93 |
| 69 | Car-092 | *Rhodosporidium toruloides* | UOFS Y-2241 | R,R | 72 |
| 70 | Car-093 | *Rhodosporidium toruloides* | UOFS Y-2242 | R,R | 74 |
| 71 | Car-094 | *Rhodosporidium toruloides* | UOFS Y-2243 | R,R | 78 |
| 72 | Car-100 | *Rhodosporidium toruloides* | UOFS Y-2245 | R,R | 83 |
| 73 | Car-103 | *Rhodosporidium toruloides* | UOFS Y-2246 | R,R | 86 |
| 74 | Car-108 | *Rhodosporidium toruloides* | UOFS Y-2247 | R,R | 62 |
| 75 | Car-118 | *Rhodosporidium toruloides* | UOFS Y-2248 | R,R | 70 |
| 76 | Car-120 | *Rhodosporidium toruloides* | UOFS Y-2249 | R,R | 86 |
| 77 | Car-121 | *Rhodosporidium toruloides* | UOFS Y-2250 | R,R | 86 |
| 78 | Car-126 | *Rhodosporidium toruloides* | UOFS Y-2251 | R,R | 80 |
| 79 | Car-131 | *Rhodosporidium toruloides* | UOFS Y-2252 | R,R | 52 |
| 80 | Car-134 | *Rhodosporidium toruloides* | UOFS Y-2253 | R,R | 52 |
| 81 | Car-142 | *Rhodosporidium toruloides* | UOFS Y-2255 | R,R | 74 |
| 82 | Car-204 | *Rhodosporidium toruloides* | UOFS Y-2257 | R,R | 92 |
| 83 | Car-205A | *Rhodosporidium toruloides* | UOFS Y-2258 | R,R | 89 |
| 84 | Car-209 | *Rhodosporidium toruloides* | UOFS Y-2260 | R,R | 78 |
| 85 | Car-210 | *Rhodosporidium toruloides* | UOFS Y-2261 | R,R | 70 |
| 86 | 25 | *Rhodotorula araucariae* | NCYC 3183 | R,R | 83 |
| 87 | 50 | *Rhodotorula glutinis* | NCYC 3186 | R,R | 55 |
| 88 | 713 | *Rhodotorula glutinis* | UOFS Y-0489 | R,R | 82 |
| 89 | Alf 6 | *Rhodotorula glutinis* | UOFS Y-0513 | R,R | 78 |
| 90 | 681 | *Rhodotorula glutinis* | UOFS Y-0653 | R,R | 93 |
| 91 | Car-022 | *Rhodotorula glutinis* | UOFS Y-2227 | R,R | 80 |
| 92 | Car-060 | *Rhodotorula glutinis* | UOFS Y-2232 | R,R | 70 |
| 93 | Car-061 | *Rhodotorula glutinis* | UOFS Y-2233 | R,R | 73 |
| 94 | Car-062 | *Rhodotorula glutinis* | UOFS Y-2234 | R,R | 76 |
| 95 | Car-075 | *Rhodotorula glutinis* | UOFS Y-2265 | R,R | 73 |
| 96 | 285 | *Sporobolomyces roseus* | NCYC 3197 | R,R | 72 |
| 97 | 225 | *Trichosporon sp.* | NCYC 3211 | R,R | 77 |

**Table 4. (Examples 98-145) Enantiomeric excesses (ee) of formed diol obtained after incubation of different yeast strains with cis-epoxysuccinic acid (20 mM, 50% w/v cells, 6 hours).**

| **Sample No.** | **Internal strain No.** | **Strain/species** | **Culture Collection No.** | **Abs. Conf** | **ee (%)** |
|---|---|---|---|---|---|
| 98 | Car 054 | *Cryptococcus curvatus* | NCYC 3158 | R,R | 98 |
| 99 | Jen 12 | *Cyptococcus laurentii* | NCYC 3160 | R,R | 98 |
| 100 | AB 24 | *Cryptococcus laurentii* | NCYC 3161 | R,R | 98 |
| 101 | AB 25 | *Cryptococcus laurentii* | UOFS Y-1884 | R,R | 98 |
| 102 | AB 58 | *Cryptococcus podzolicus* | NCYC 3164 | R,R | 98 |
| 103 | AB 49 | *Cryptococcus podzolicus* | UOFS Y-1882 | R,R | 98 |
| 104 | AB 50 | *Cryptococcus podzolicus* | UOFS Y-1883 | R,R | 98 |
| 105 | AB 52 | *Cryptococcus podzolicus* | UOFS Y-1895 | R,R | 98 |
| 106 | AB 47 | *Cryptococcus podzolicus* | UOFS Y-1908 | R,R | 98 |
| 107 | 676 | *Pichia haplophila* | NCYC 3176 | R,R | 44 |
| 108 | 692 | *Rhodosporidium paludigenum* | NCYC 3179 | R,R | 98 |
| 109 | Car 118 | *Rhodosporidium toruloides* | NCYC 3182 | R,R | 98 |
| 110 | POH 28 | *Rhodosporidium toruloides* | NCYC 3215 | R,R | 98 |
| 111 | 46 | *Rhodosporidium toruloides* | UOFS Y-0471 | R,R | 22 |
| 112 | Car 003 | *Rhodosporidium toruloides* | UOFS Y-2222 | R,R | 98 |
| 113 | Car 020 | *Rhodosporidium toruloides* | UOFS Y-2226 | R,R | 98 |
| 114 | Car 038 | *Rhodosporidium toruloides* | UOFS Y-2228 | R,R | 98 |
| 115 | Car 052 | *Rhodosporidium toruloides* | UOFS Y-2230 | R,R | 98 |
| 116 | Car 059 | *Rhodosporidium toruloides* | UOFS Y-2231 | R,R | 98 |
| 117 | Car 076 | *Rhodosporidium toruloides* | UOFS Y-2238 | R,R | 98 |
| 118 | Car 078 | *Rhodosporidium toruloides* | UOFS Y-2240 | R,R | 98 |
| 119 | Car 092 | *Rhodosporidium toruloides* | UOFS Y-2241 | R,R | 98 |
| 120 | Car 093 | *Rhodosporidium toruloides* | UOFS Y-2242 | R,R | 98 |
| 121 | Car 094 | *Rhodosporidium toruloides* | UOFS Y-2243 | R,R | 98 |
| 122 | Car 100 | *Rhodosporidium toruloides* | UOFS Y-2245 | R,R | 98 |
| 123 | Car 103 | *Rhodosporidium toruloides* | UOFS Y-2246 | R,R | 98 |
| 124 | Car 108 | *Rhodosporidium toruloides* | UOFS Y-2247 | R,R | 98 |
| 125 | Car 120 | *Rhodosporidium toruloides* | UOFS Y-2249 | R,R | 98 |
| 126 | Car 131 | *Rhodosporidium toruloides* | UOFS Y-2252 | R,R | 98 |
| 127 | Car 134 | *Rhodosporidium toruloides* | UOFS Y-2253 | R,R | 98 |
| 128 | Car 142 | *Rhodosporidium toruloides* | UOFS Y-2255 | R,R | 98 |
| 129 | Car 200 | *Rhodosporidium toruloides* | UOFS Y-2256 | R,R | 98 |
| 130 | Car 204 | *Rhodosporidium toruloides* | UOFS Y-2257 | R,R | 98 |
| 131 | Car 205 A | *Rhodosporidium toruloides* | UOFS Y-2258 | R,R | 98 |
| 132 | Car 209 | *Rhodosporidium toruloides* | UOFS Y-2260 | R,R | 98 |
| 133 | Car 210 | *Rhodosporidium toruloides* | UOFS Y-2261 | R,R | 54 |
| 134 | 25 | *Rhodotorula araucariae* | NCYC 3183 | R,R | 98 |
| 135 | 50 | *Rhodotorula glutinis* | NCYC 3186 | R,R | 76 |
| 136 | 713 | *Rhodotorula glutinis* | UOFS Y-0489 | R,R | 98 |
| 137 | Alf 06 | *Rhodotorula glutinis* | UOFS Y-0513 | R,R | 98 |
| 138 | 681 | *Rhodotorula glutinis* | UOFS Y-0653 | R,R | 98 |
| 139 | Car 022 | *Rhodotorula glutinis* | UOFS Y-2227 | R,R | 98 |
| 140 | Car 066 | *Rhodotorula glutinis* | UOFS Y-2235 | R,R | 98 |
| 141 | Car 075 | *Rhodotorula glutinis* | UOFS Y-2265 | R,R | 98 |
| 142 | 172 | *Rhodotorula sp.* | NCYC 3192 | R,R | 98 |
| 143 | 158 | *Rhodotorula sp. minuta* / *mucilaginosa* | NCYC 3194 | R,R | 98 |
| 144 | Car 205B | *Trichosporon montevideense* | NCYC 3225 | R,R | 23 |
| 145 | 155 | *Trichosporon sp.* | NCYC 3212 | R,R | 98 |

All the yeast strains referred to in this and the following examples are kept and maintained at the University of the Orange Free State (UOFS), Department of Microbial, Biochemical and Food Biotechnology, Faculty of Natural and Agricultural Sciences, P.O. Box 339, Bloemfontein 9300, South Africa (Tel +27 51 401 2396, Fax + 27 51 444 3219) and are readily identified by the yeast species and culture collection number as indicated. Representative examples of strains belonging to the different species have been deposited under the Budapest Treaty at National Collection of Yeast Cultures (NCYC), Institute of Food Research Norwich Research Park Colney Norwich NR.4 7UA, U.K. (Tel: +44-(0)1603-255274 Fax: +44-(0)1603-458414 Email: ncyc@bbsrc.ac.uk) and are readily identified by the yeast species and culture collection accession number as indicated. The samples deposited with the NCYC are taken from the same deposit maintained by the South African Council for Scientific and Industrial Research (CSIR) since prior to the filing date of this application. The deposits will be maintained without restriction in the NCYC depository for a period of 30 years, or 5 years after the most recent request, or for the effective life of the patent, whichever is longer, and will be replaced if the deposit becomes non-viable during that period. Samples of the yeast strains not deposited at NCYC will be made available upon request on the same basis and conditions of the Budapest Treaty.

### Example IV. Desymmetrisation of cis-2,3-epoxybutane, cyclohexene oxide, cyclopentene oxide, cis-epoxy succinic acid, and 3,4-epoxytetrahydrofuran.

Fig. 3 gives examples of desymmetrisation reactions performed using various MEO as substrates for hydrolase-catalysed reactions giving rise to various corresponding vicinal diol products.

### Example V. Desymmetrisation of cis-2,3-epoxybutane to produce (R,R)-2,3-butanediol

(a) Various wild-type yeast strains selected from Tables 2-4 were tested (in this example as Samples 146 - 148) for their ability to produce optically active 2,3-butanediol from cis-2,3-epoxybutane. For each example, a line graph is supplied that shows the change in concentrations of the vicinal diol enantiomers with time (left y-axis) and the Enantiomeric excess (ee) of the formed diol at different times (right y-axis) (Figs. 6 - 8). As expected for desymmetrisation reactions, the ee remained essentially constant throughout the reaction.
(b) Figs. 9 -12 show the desymmetrisation of cis-2,3-epoxybutane by various recombinant yeast strains (tested in this Example as Samples 149-152) expressing, under control of the constitutive TEF promoter (Madzak *et al.*, 2004), exogenous epoxide hydrolases from selected wild-type yeast strains. For each example, a line graph is supplied that shows the change in concentrations of the vicinal diol enantiomers with time (left y-axis) and the enantiomeric excess (ee) of the formed diol at different times (right y-axis). As expected for desymmetrisation reactions, the ee remained essentially constant throughout the reaction.

### Example VI. Desymmetrisation of cyclohexene oxide to produce (R,R)-cyclohexane diol

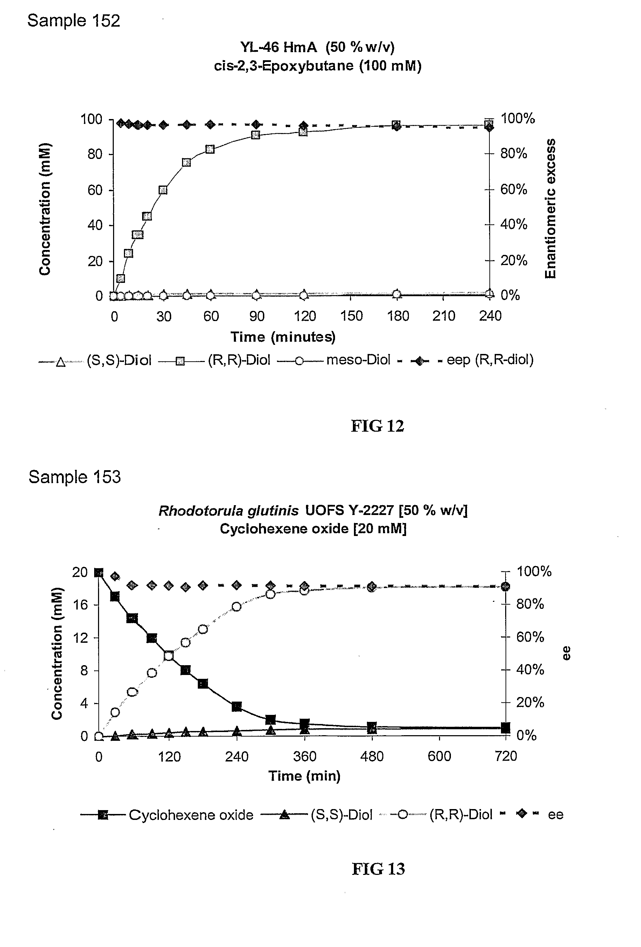

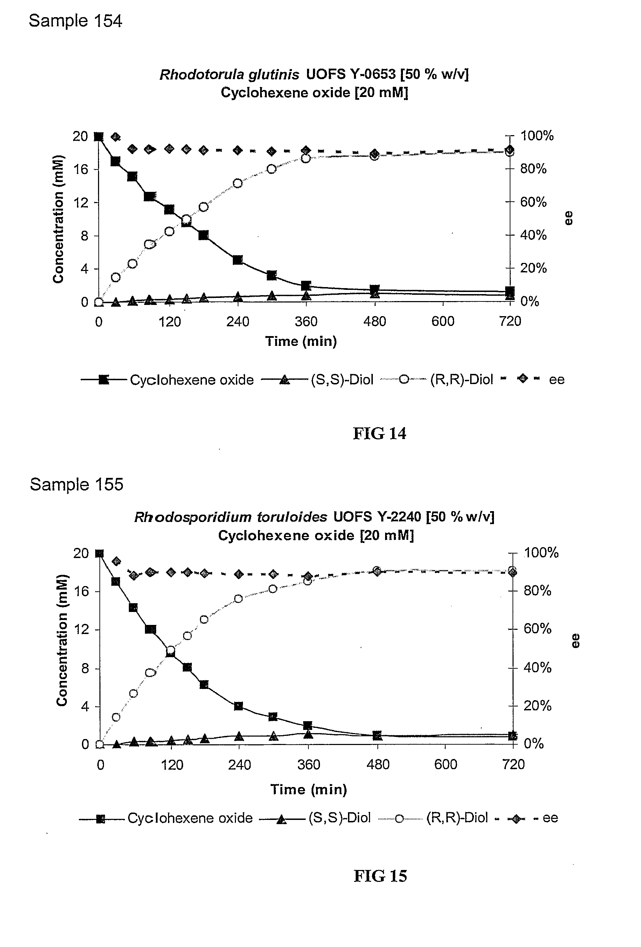

(a) Various wild-type yeast strains selected from Tables 2-4 were tested (in this example as Samples 153 - 155) for their ability to produce optically active cyclohexane diol from cyclohexene oxide. For each example, a line graph is supplied that shows the change in concentrations of the vicinal diol enantiomers with time (left y-axis) and the enantiomeric excess (ee) of the formed diol at different times (right y-axis) (Figs. 13 - 15). As expected for desymmetrisation reactions, the ee remained essentially constant throughout the reaction.
(b) Figs. 16 -20 show the desymmetrisation of cyclohexene oxide by various recombinant yeast strains (tested in this Example as Samples 156-160) expressing, under control of the constitutive TEF promoter (Madzak et al., 2004), exogenous epoxide hydrolases from selected wild-type yeast strains. For each example, a line graph is supplied that shows the change in concentrations of the vicinal diol enantiomers with time (left y-axis) and the enantiomeric excess (ee) of the formed diol at different times (right y-axis). As expected for desymmetrisation reactions, the ee remained essentially constant throughout the reaction.

### Example VII. Production of optically active cyclohexane diol from cyclohexene oxide using whole or lysed yeast host cells transformed with the epoxide hydrolase genes from selected wild type yeasts

Whole cells (Fig. 21 and 24) and lysed cell suspensions (Figs. 22 and 23) of yeast host cells transformed with the epoxide hydrolase gene from *Rhodosporidium paludigenum* (NCYC 3179) (Figs 21 and 22) or *Rhodotorula araucariae* (NCYC 3183) (Fig. 23) or *Rhodosporidium toruloides* (UOFS Y-0471) (Fig. 24) strains under control of the quasi-constitutive hp4d promoter (Madzak *et al.*, 2004) were tested (as Samples 161-164) for their ability to produce optically active cyclohexane diol from cyclohexene oxide. Lysed cell suspensions were prepared by treatment of the cells with Y-PER®, a yeast protein extraction reagent from Sigma-Aldrich (St. Louis, MO, U.S.A.) according to the instructions of the manufacturer. No significant differences in activity or enantioselectivity between and whole and lysed yeast cells were observed.

### Example VIII. Desymmetrisation of cyclopentene oxide to produce (R,R)-cyclopentane diol

Figs. 25-31 show the desymmetrisation of cyclopentene oxide by various recombinant yeast strains (tested in this Example as Samples 165-171) expressing, under control of the single-copy constitutive TEF promoter or the quasi-constitutive multi-copy hp4d promoter (Madzak *et al.*, 2004), exogenous epoxide hydrolases from selected wild-type yeast strains. For each example, a line graph is supplied that shows the change in concentrations of the vicinal diol enantiomers with time (left y-axis) and the enantiomeric excess (ee) of the formed diol at different times (right y-axis). As expected for desymmetrisation reactions, the ee remained essentially constant throughout the reaction.

### Example IX. Desymmetrisation of cis-epoxysuccinate to produce (R,R)-tartaric acid

Table 5 shows the desymmetrisation of cis-epoxysuccinate by various recombinant yeast strains (tested in this Example as Samples 172-182) expressing, under control of the single-copy constitutive TEF promoter or the quasi-constitutive multi-copy hp4d promoter (Madzak *et al.*, 2004), exogenous epoxide hydrolases from selected wild-type yeast strains.

Reaction conditions: Biomass (25 % w/v) of *Yarrowia lipolytica* transformants expressing the epoxide hydrolases of different yeast strains was suspended in phosphate buffer (pH 7.5, 50 mM) and incubated with cis-epoxysuccinic acid disodium salt for 4 hours at 25°C. The catalysts wa.s removed by centrifugation and the supernatant was analysed by chiral HPLC to determine the ee of the L-tartaric acid formed.

Figs. 32 show the desymmetrisation of cis epoxysuccinate by a recombinant yeast strain (tested in this Example as Sample 183) expressing, under control of the single-copy constitutive TEF promoter (Madzak *et al.*, 2004), exogenous epoxide hydrolases from thewild-type yeast strain *Cryptococcus curvatus* NCYC 3158. For this example, a line graph is supplied that shows the change in concentrations of the vicinal diol enantiomers with time (left y-axis) and the enantiomeric excess (ee) of the formed diol at different times (right y-axis). As expected for desymmetrisation reactions, the ee remained essentially constant throughout the reaction.

**Table 5. Enantiomeric excesses (ee) of L-tartaric acid after 4 hours of reaction.**

| **Sample No.** | **Yeast strain** | **Promoter** | **ee (%)** |
|---|---|---|---|
| 172 | *Rhodotorula mucilaginosa* NCYC 3190 | *TEF* | 52.7 |
| 173 | *Rhodotorula araucariae* NCYC 3183 | *TEF* | 19.6 |
| 174 | *Rhodotorula araucariae* NCYC 3183 | *hp4d* | 13.0 |
| 175 | *Rhodosporidium toruloides* UOFS Y-0471 | *TEF* | 10.5 |
| 176 | *Rhodosporidium paludigenum* NCYC 3179 | *TEF* | 63.4 |
| 177 | *Rhodosporidium paludigenum* NCYC 3179 | *hp4d* | 21.3 |
| 178 | *Filobasidium neoformans var neoformans* | *TEF* | 47.3 |
| 179 | *Rhodosporidium toruloides* NCYC 3181 | *TEF* | 23.3 |
| 180 | *Cryptococcus curvatus* NCYC 3158 | *TEF* | 82.9 |
| 181 | *Yarrowia lipolytica* NCYC 3229 | *TEF* | 20.7 |
| 182 | *Yarrowia lipolytica* NCYC 3229 | *TEF* | 79.9 |

### Example X. Desymmetrisation of 3,4-epoxytetrahydrofuran to produce (R,R)-tetrahydro-3,4-furandiol

Figs. 33 show the desymmetrisation of 3,4-epoxytetrahydrofuan by a recombinant yeast strain (tested in this Example as Sample 184) expressing, under control of the single-copy constitutive TEF promoter (Madzak *et al.*, 2004), exogenous epoxide hydrolases from thewild-type yeast strain *Rhodotorula mucilaginosa* NCYC 3190. For this example, a line graph is supplied that shows the change in concentrations of the vicinal diol enantiomers with time (left y-axis) and the enantiomeric excess (ee) of the formed diol at different times (right y-axis). As expected for desymmetrisation reactions, the ee remained essentially constant throughout the reaction.

### References

Madzak, C., Gaillardin, C., Beckerich, J-M. (2004). Heterologous protein expression and secretion in the non-conventional yeast Yarrowia lipolytica: a review. Journal of Biotechnology 109: 63-81.
Nicaud J-M, Madzak C, Van den Broek P, Gysler C, Duboc P, Niederberger P, Gaillardin C. (2002) Protein expression and secretion in the yeast Yarrowia lipolytica. FEMS Yeast Research 2, 371-279.
Zhao, L., Han, B., Huang, Z., Miller, M., Huang, H., Malashock, D.S., Zhu, Z., Milan, A., Robertson, D.E., Weiner, D.P. & Burk, M.J. (2004). Epoxide hydrolase-catalyzed enantioselective synthesis of chiral 1,2-diols via desymmetrization of meso-epoxides. J. Am. Chem. Soc., 126: 11156-11157.

## Claims

1. A process for obtaining an optically active vicinal diol, which process includes the steps of:
providing a *meso*-epoxide;
creating a reaction mixture by mixing with the meso-epoxide a *Yarrowia lipolytica* cell comprising an exogenous nucleic acid encoding, and capable of expressing, a heterologous polypeptide or a functional fragment thereof, having enantioselective epoxide hydrolase activity;
incubating the reaction mixture; and
recovering from the reaction mixture an enantiopure, or a substantially enantiopure, vicinal diol.

2. The process of claim 1, wherein the polypeptide is a full-length yeast epoxide hydrolase.

3. The process of claim 1, wherein the polypeptide is a functional fragment of yeast epoxide hydrolase.

4. The process of any one of claims 1 to 3, wherein the process is carried out at a pH from 5 to 10.

5. The process of any one of claims 1 to 4, wherein the process is carried out at a temperature of 0ºC to 70ºC.

6. The process of any one of claims 1 to 5, wherein the concentration of the *meso-*epoxide in the reaction matrix is at least equal to the solubility of the *meso*-epoxide in water.

7. The process of any one of claims 1 to 6, wherein the *meso*-epoxide is a compound of the general formula (I), (II), (V), (VI) or (VII) and the vicinal diol produced by the process is a compound of the general formula (III), (IV), (VIII), (IX) or (X), 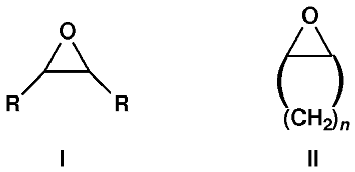 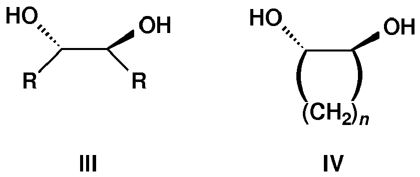 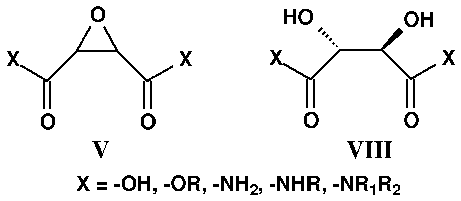 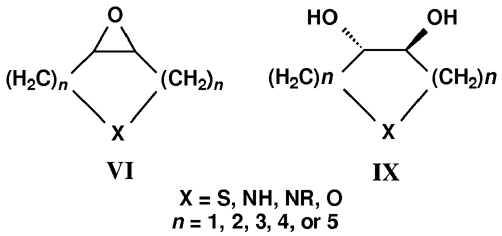 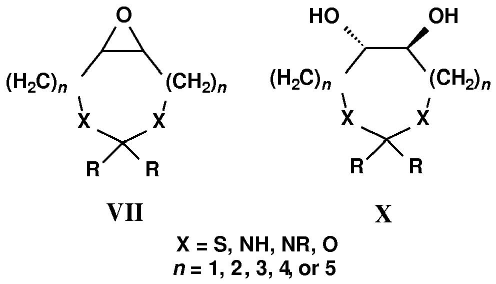 wherein:
R is selected from the group consisting of a variably substituted straight-chain or branched alkyl group, a variably substituted straight-chain or branched alkenyl group, a variably substituted straight-chain or branched alkynyl group, a variably substituted cycloalkyl group as well as cycloalkenyl groups, a variably substituted aryl group, a variably substituted aryl alkyl group, a variably substituted heterocyclic group, a variably substituted straight-chain or branched alkoxy group, a variably substituted straight-chain or branched alkenyloxy group, a variably substituted aryloxy group, a variably substituted aryl alkyloxy group, a variably substituted alkylthio group, a variably substituted alkoxycarbonyl group, a variably substituted straight chain or branched alkylamino or alkenyl amino group, a variably substituted arylamino or arylalkylamino group, a variably substituted carbamoyl group, a variably substituted acyl group, and a functional group.

8. The process of any of claims 1 to 7, which process includes adding to the reaction mixture water and at least one water-immiscible solvent.

9. The process of any of claims 1 to 7, which process includes adding to the reaction mixture water and at least one water-miscible organic solvent.

10. The process of any one of claims 1 to 9, which process includes adding to the reaction mixture at least one reagent selected from the group consisting of one or more surfactants, one or more cyclodextrins, and one or more phase-transfer catalysts.

11. The process of any one of claims 1 to 10, which process includes stopping the reaction when one enantiomer of the vicinal diol is in excess compared to the other enantiomer of the vicinal diol.

12. The process of any one of claims 1 to 11, which process includes recovering continuously during the reaction the optically active vicinal diol produced by the reaction directly from the reaction mixture.

## Patentansprüche

1. Verfahren zur Gewinnung eines optisch aktiven vicinalen Diols, wobei das Verfahren folgende Schritte enthält:
- Bereitstellen eines *meso*-Epoxids,
- Herstellen eines Reaktionsgemisches durch Mischen des *meso*-Epoxids mit einer *Yarrowia lipolytica*-Zelle, welche eine exogene Nukleinsäure enthält, die ein heterologes Polypeptid oder ein funktionelles Fragment davon kodiert und in der Lage ist, dieses zu enthalten, wobei dieses eine enantioselektive Epoxidhydrolaseaktivität aufweist,
- Inkubieren des Reaktionsgemisches, und
- Rückgewinnung eines enantiomerenreinen oder eines im Wesentlichen enantiomerenreinen vicinalen Diols aus dem Reaktionsgemisch.

2. Verfahren nach Anspruch 1, wobei das Polypeptid eine volllängige Hefe-Epoxidhydrolase ist.

3. Verfahren nach Anspruch 1, wobei das Polypeptid ein funktionelles Fragment einer Hefe-Epoxidhydrolase ist.

4. Verfahren nach einem der Ansprüche 1 bis 3, wobei das Verfahren bei einem pH-Wert von 5 bis 10 durchgeführt wird.

5. Verfahren nach einem der Ansprüche 1 bis 4, wobei das Verfahren bei einer Temperatur von 0 °C bis 70 °C durchgeführt wird.

6. Verfahren nach einem der Ansprüche 1 bis 5, wobei die Konzentration des *meso*-Epoxids in der Reaktionsmatrix mindestens gleich der Löslichkeit des *meso-*Epoxids in Wasser ist.

7. Verfahren nach einem der Ansprüche 1 bis 6, wobei das *meso*-Epoxid eine Verbindung der allgemeinen Formel (I), (II), (V), (VI) oder (VII) und das nach dem Verfahren produzierte vicinale Diol eine Verbindung der allgemeinen Formel (111), (IV), (VIII), (IX) oder (X) ist: 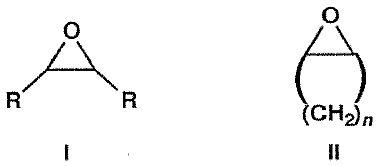 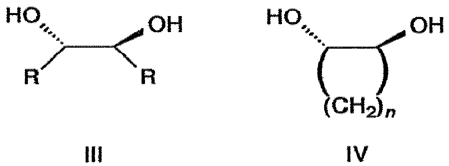 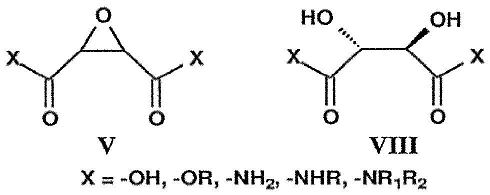 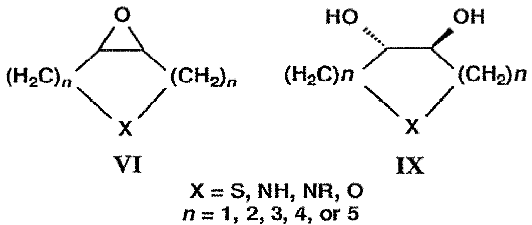 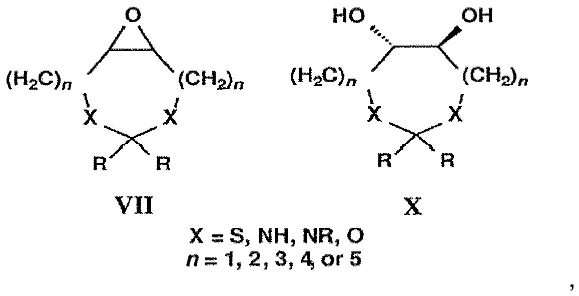 wobei R ausgewählt ist aus einer Gruppe bestehend aus einer variabel substituierten geradkettigen oder verzweigten Alkylgruppe, einer variabel substituierten geradkettigen oder verzweigten Alkenylgruppe, einer variabel substituierte geradkettigen oder verzweigten Alkinylgruppe, einer variabel substituierten Cycloalkylgruppe sowie Cycloalkenylgruppen, einer variabel substituierten Arylgruppe, einer variabel substituierten Arylalkylgruppe, einer variabel substituierten heterocyclischen Gruppe, einer variabel substituierten geradkettigen oder verzweigten Alkoxygruppe, einer variabel substituierten geradkettigen oder verzweigten Alkenyloxygruppe, einer variabel substituierten Aryloxygruppe, einer variabel substituierten Arylalkyloxygruppe, einer variabel substituierten Alkylthiogruppe, einer variabel substituierten Alkoxycarbonylgruppe, einer variabel substituierten geradkettigen oder verzweigten Alkylaminogruppe oder Alkenylaminogruppe, einer variabel substituierten Arylamino- oder Arylalkylaminogruppe, einer variabel substituierten Carbamoylgruppe, einer variabel substituierten Acylgruppe, und einer funktionellen Gruppe.

8. Verfahren nach einem der Ansprüche 1 bis 7, wobei das Verfahren die Zugabe von Wasser und von wenigstens einem nicht wassermischbaren Lösungsmittel zu dem Reaktionsgemisch beinhaltet.

9. Verfahren nach einem der Ansprüche 1 bis 7, wobei das Verfahren die Zugabe von Wasser und von wenigstens einem wassermischbaren organischen Lösungsmittel zu dem Reaktionsgemisch beinhaltet.

10. Verfahren nach einem der Ansprüche 1 bis 9, wobei das Verfahren die Zugabe von wenigstens einem Reagenz zu dem Reaktionsgemisch beinhaltet, welches aus einer Gruppe bestehend aus einem oder mehreren Tensiden, einem oder mehreren Cyclodextrinen und einem oder mehreren Phasentransferkatalysatoren ausgewählt wird.

11. Verfahren nach einem der Ansprüche 1 bis 10, wobei das Verfahren ein Stoppen der Reaktion umfasst, wenn ein Enantiomer des vicinalen Diols verglichen mit dem anderen Enantiomer des vicinalen Diols im Überschuss vorliegt.

12. Verfahren nach einem der Ansprüche 1 bis 11, wobei das Verfahren ein kontinuierliches Abziehen des durch die Reaktion produzierten optisch aktiven vicinalen Diols während der Reaktion direkt aus der Reaktionsmischung beinhaltet.

## Revendications

1. Procédé permettant d'obtenir un diol vicinal optiquement actif, lequel procédé comprend les étapes suivantes :
la fourniture d'un époxyde méso ;
la création d'un mélange réactionnel en mélangeant avec l'époxyde méso une cellule de *Yarrowia lipolytica* comprenant un acide nucléique exogène codant, et capable d'exprimer, un polypeptide hétérologue ou l'un de ses fragments fonctionnels, doté d'une activité époxyde hydrolase énantiosélective ;
l'incubation du mélange réactionnel ; et
la récupération à partir du mélange réactionnel d'un diol vicinal énantiopur ou substantiellement énantiopur.

2. Procédé selon la revendication 1, dans lequel le polypeptide est une époxyde hydrolase de levure pleine longueur.

3. Procédé selon la revendication 1, dans lequel le polypeptide est un fragment fonctionnel d'époxyde hydrolase de levure.

4. Procédé selon l'une quelconque des revendications 1 à 3, le procédé étant réalisé à un pH de 5 à 10.

5. Procédé selon l'une quelconque des revendications 1 à 4, le procédé étant réalisé à une température de 0 °C à 70 °C.

6. Procédé selon l'une quelconque des revendications 1 à 5, dans lequel la concentration de l'époxyde méso dans la matrice réactionnelle est au moins égale à la solubilité de l'époxyde méso dans l'eau.

7. Procédé selon l'une quelconque des revendications 1 à 6, dans lequel l'époxyde méso est un composé de formule générale (I), (II), (V), (VI) ou (VII) et le diol vicinal produit par le procédé est un composé de formule générale (III), (IV), (VIII), (IX) ou (X), 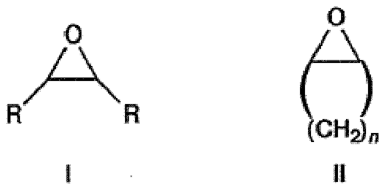 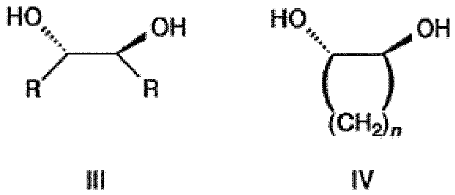 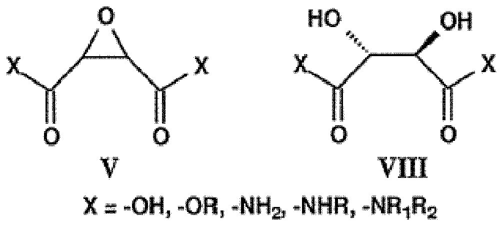 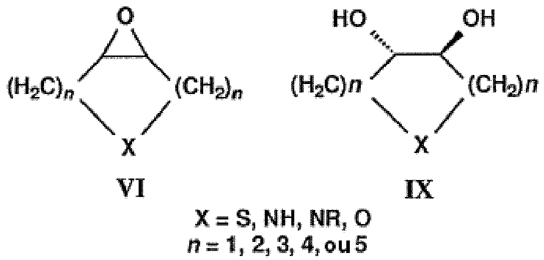 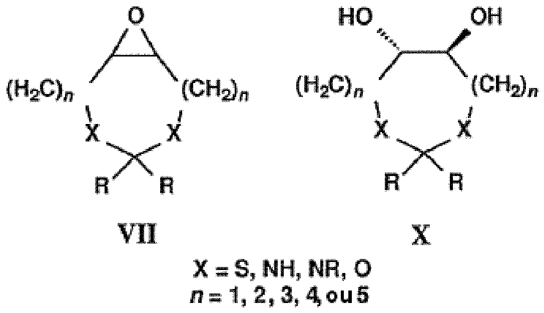 où :
R est choisi dans le groupe constitué d'un groupe alkyle à chaîne linéaire ou ramifiée substitué de façon variable, d'un groupe alcényle à chaîne linéaire ou ramifiée substitué de façon variable, d'un groupe alcynyle à chaîne linéaire ou ramifiée substitué de façon variable, d'un groupe cycloalkyle substitué de façon variable ainsi que de groupes cycloalcényle, d'un groupe aryle substitué de façon variable, d'un groupe arylalkyle substitué de façon variable, d'un groupe hétérocyclique substitué de façon variable, d'un groupe alcoxy à chaîne linéaire ou ramifiée substitué de façon variable, d'un groupe alcényloxy à chaîne linéaire ou ramifiée substitué de façon variable, d'un groupe aryloxy substitué de façon variable, d'un groupe alkyloxy substitué de façon variable, d'un groupe alkylthio substitué de façon variable, d'un groupe alcoxycarbonyle substitué de façon variable, d'un groupe alkylamino ou alcénylamino à chaîne linéaire ou ramifiée substitué de façon variable, d'un groupe arylamino ou arylalkylamino substitué de façon variable, d'un groupe carbamoyle substitué de façon variable, d'un groupe acyle substitué de façon variable, et d'un groupe fonctionnel.

8. Procédé selon l'une quelconque des revendications 1 à 7, lequel procédé comprend l'addition au mélange réactionnel d'eau et d'au moins un solvant non miscible à l'eau.

9. Procédé selon l'une quelconque des revendications 1 à 7, lequel procédé comprend l'addition au mélange réactionnel d'eau et d'au moins un solvant organique miscible à l'eau.

10. Procédé selon l'une quelconque des revendications 1 à 9, lequel procédé comprend l'addition au mélange réactionnel d'au moins un réactif choisi dans le groupe constitué d'un ou de plusieurs tensioactifs, d'une ou de plusieurs cyclodextrines, et d'un ou de plusieurs catalyseurs de transfert de phase.

11. Procédé selon l'une quelconque des revendications 1 à 10, lequel procédé comprend l'arrêt de la réaction lorsqu'un énantiomère du diol vicinal est en excès comparativement à l'autre énantiomère du diol vicinal.

12. Procédé selon l'une quelconque des revendications 1 à 11, lequel procédé comprend la récupération en continu durant la réaction du diol vicinal optiquement actif produit par la réaction directement à partir du mélange réactionnel.
